# EUROPEAN PATENT APPLICATION

(11) **EP 4 471 057 A1**
(43) Date of publication of application: **04.12.2024**
(21) Application number: 23747054.7
(22) Date of filing: 26.01.2023
(51) Int. Cl.: C07K 16/18, A61K 39/395, A61K 45/00, A61P 1/00, A61P 35/00, A61P 43/00, C12N 15/13

(54) **ANTI-HUMAN CXCL1 ANTIBODY**

(30) Priority: 27.01.2022 JP 2022011107
(71) Applicant: CHIOME BIOSCIENCE INC., Shibuya-ku Tokyo 151-0071 (JP); University Public Corporation Osaka, Osaka-shi, Osaka 545-0051 (JP)
(72) Inventor: YASHIRO, Masakazu, Osaka-shi, Osaka 558-8585 (JP); YAMAMOTO, Yurie, Osaka-shi, Osaka 558-8585 (JP); YOSHIOKA, Akiko, Tokyo 151-0071 (JP); NAKAMURA, Koji, Tokyo 151-0071 (JP); YANAI, Hiroyuki, Tokyo 151-0071 (JP); INOUE, Toshikazu, Tokyo 151-0071 (JP)
(74) Representative: J A Kemp LLP
(86) International application number: PCT/JP2023/002533
(87) International publication number: WO 2023/145844

(57) **Abstract**

The present invention provides a substance which exerts an antitumor effect by causing alterations in the tumor microenvironment (TME), including the inhibition or suppression of TME formation. The present invention is directed to an antibody against human CXCL1 or an antibody fragment thereof, which has particular CDR sequences.

## Description

### Technical Field

The present invention relates to an antibody against human CXCL1 or an antibody fragment thereof, and uses thereof.

### Background Art

Cancer is a disease that occurs in a wide range of organs and affects many people in the world. Standard therapy for cancer includes surgical therapy, radiotherapy and chemotherapy (including an anticancer agent composed of an antibody). Particularly in the case of solid cancer, surgical excision is often most effective as radical treatment, while treatment with an anticancer agent is frequently used as adjuvant therapy before and after excision.

Many anticancer agents effective against various types of cancer have been marketed and used for therapeutic purposes. Recent anticancer agents include those which activate immunocytes by removal of immunosuppressive action and thereby exert an antitumor effect, and those which suppress intratumoral angiogenesis and thereby exert an antitumor effect. Thus, recent attention has been focused on the development of antitumor agents whose mechanism of action is different from conventional antitumor activity that directly inhibits the growth or survival of cancer cells.

One of them is an antitumor agent which alters the tumor microenvironment (TME) and thereby exerts an antitumor effect. TME refers to an environment beneficial to tumor, which is formed when cancer cells and stromal cells/components are mixed and interacted with each other in tumor tissue, and recent studies have been elucidating that the formation of this TME plays a significant role in the growth and drug resistance mechanism of tumor (see, e.g., Non-patent Document 1). For this reason, the alternation of TME would lead to the suppression of cancer cell growth and further the suppression of tumor exacerbation; and hence there is an ongoing development of antitumor agents with a focus on the formation of TME.

Stromal cells constituting TME include bone marrow-derived mesenchymal stem cells (BM-MSCs). BM-MSCs are known to play a critical role in the formation of TME through the production of humoral factors or through the production of extracellular matrices (e.g., collagen) after differentiation into cancer-associated fibroblasts (see, e.g., Non-patent Document 2). Extracellular matrices serve as scaffolds for cancer cell growth, and also act as barriers against existing chemotherapeutics which are designed to directly target cancer cells. For this reason, when BM-MSCs involved in TME formation are eliminated from TME, their elimination would probably contribute to the suppression of tumor growth and the efficacy of chemotherapeutics (see, e.g., Non-patent Document 3).

### Prior Art Documents

### Non-patent Documents

Non-patent Document 1: Lillian Sun et al., JCI Insight, 2019 Apr 4;4(7)
Non-patent Document 2: Hiroaki Kasashima et al., Am. J. Pathol., Vol. 186, No. 11, November 2016
Non-patent Document 3: Augustin Le Naour et al., J. Mol. Biol., Vol. 12(3), pp. 202-2015, 2020
Non-patent Document 4: Krishna Mohan Sepuru et al., J. Biol Chem. 2016 Feb 19; 291(8):4247-55

### Summary of the Invention

### Problem to be Solved by the Invention

Under these circumstances, there has been a demand for the development of a substance or the like which exerts an antitumor effect by causing alterations in the tumor microenvironment (TME), including the inhibition or suppression of TME formation.

### Means to Solve the Problem

The present invention has been made in consideration of the above situation and aims to provide an anti-human CXCL1 antibody and an antibody fragment thereof, as well as uses thereof (e.g., a pharmaceutical composition), etc., as shown below.
[1] An antibody against human CXCL1, wherein
   (a) the amino acid sequences of heavy chain variable region (VH) complementarity determining region (CDR) 1, CDR2 and CDR3 consist of:
      the amino acid sequences shown in SEQ ID NOs: 2, 3 and 4, respectively; or
      the amino acid sequences shown in SEQ ID NOs: 2, 34 and 4, respectively, and
      the amino acid sequences of light chain variable region (VL) CDR1, CDR2 and CDR3 consist of:
         the amino acid sequences shown in SEQ ID NOs: 6, 7 and 8, respectively,
   (b) the amino acid sequences of VH CDR1, CDR2 and CDR3 consist of:
      the amino acid sequences shown in SEQ ID NOs: 10, 11 and 12, respectively, and
      the amino acid sequences of VL CDR1, CDR2 and CDR3 consist of:
         the amino acid sequences shown in SEQ ID NOs: 14, 15 and 16, respectively;
         the amino acid sequences shown in SEQ ID NOs: 14, 36 and 16, respectively;
         the amino acid sequences shown in SEQ ID NOs: 14, 38 and 16, respectively; or
         the amino acid sequences shown in SEQ ID NOs: 14, 40 and 16, respectively,
   (c) the amino acid sequences of VH CDR1, CDR2 and CDR3 consist of:
      the amino acid sequences shown in SEQ ID NOs: 18, 19 and 20, respectively;
      the amino acid sequences shown in SEQ ID NOs: 18, 42 and 20, respectively;
      the amino acid sequences shown in SEQ ID NOs: 18, 44 and 20, respectively;
      the amino acid sequences shown in SEQ ID NOs: 18, 46 and 20, respectively;
      the amino acid sequences shown in SEQ ID NOs: 18, 48 and 20, respectively;
      the amino acid sequences shown in SEQ ID NOs: 18, 50 and 20, respectively;
      the amino acid sequences shown in SEQ ID NOs: 18, 52 and 20, respectively;
      the amino acid sequences shown in SEQ ID NOs: 18, 54 and 20, respectively;
      the amino acid sequences shown in SEQ ID NOs: 18, 56 and 20, respectively;
      the amino acid sequences shown in SEQ ID NOs: 18, 58 and 20, respectively;
      the amino acid sequences shown in SEQ ID NOs: 18, 60 and 20, respectively; or
      the amino acid sequences shown in SEQ ID NOs: 18, 62 and 20, respectively, and
      the amino acid sequences of VL CDR1, CDR2 and CDR3 consist of:
         the amino acid sequences shown in SEQ ID NOs: 22, 23 and 24, respectively, or
   (d) the amino acid sequences of VH CDR1, CDR2 and CDR3 consist of:
      the amino acid sequences shown in SEQ ID NOs: 26, 27 and 28, respectively;
      the amino acid sequences shown in SEQ ID NOs: 26, 64 and 28, respectively; or
      the amino acid sequences shown in SEQ ID NOs: 26, 66 and 28, respectively, and
      the amino acid sequences of VL CDR1, CDR2 and CDR3 consist of:
         the amino acid sequences shown in SEQ ID NOs: 30, 31 and 32, respectively.
[2] An antibody, wherein
   (a) the amino acid sequences of VH CDR1, CDR2 and CDR3 consist of the amino acid sequences shown in SEQ ID NOs: 2, 34 and 4, respectively, and
      the amino acid sequences of VL CDR1, CDR2 and CDR3 consist of the amino acid sequences shown in SEQ ID NOs: 6, 7 and 8, respectively,
   (b) the amino acid sequences of VH CDR1, CDR2 and CDR3 consist of the amino acid sequences shown in SEQ ID NOs: 10, 11 and 12, respectively, and
      the amino acid sequences of VL CDR1, CDR2 and CDR3 consist of the amino acid sequences shown in SEQ ID NOs: 14, 36 and 16, respectively,
   (c) the amino acid sequences of VH CDR1, CDR2 and CDR3 consist of the amino acid sequences shown in SEQ ID NOs: 18, 54 and 20, respectively, and
      the amino acid sequences of VL CDR1, CDR2 and CDR3 consist of the amino acid sequences shown in SEQ ID NOs: 22, 23 and 24, respectively, or
   (d) the amino acid sequences of VH CDR1, CDR2 and CDR3 consist of the amino acid sequences shown in SEQ ID NOs: 26, 64 and 28, respectively, and
      the amino acid sequences of VL CDR1, CDR2 and CDR3 consist of the amino acid sequences shown in SEQ ID NOs: 30, 31 and 32, respectively.
[3] An antibody against human CXCL1, wherein
   (a) the amino acid sequence of the heavy chain variable region (VH) consists of the amino acid sequence shown in SEQ ID NO: 1 or 33, and the amino acid sequence of the light chain variable region (VL) consists of the amino acid sequence shown in SEQ ID NO: 5,
   (b) the amino acid sequence of VH consists of the amino acid sequence shown in SEQ ID NO: 9, and the amino acid sequence of VL consists of the amino acid sequence shown in SEQ ID NO: 13, 35, 37 or 39, or
   (c) the amino acid sequence of VH consists of the amino acid sequence shown in SEQ ID NO: 17, 41, 43, 45, 47, 49, 51, 53, 55, 57, 59 or 61, and the amino acid sequence of VL consists of the amino acid sequence shown in SEQ ID NO: 21.
[4] An antibody against human CXCL1, wherein
   (a) the amino acid sequence of the heavy chain variable region (VH) consists of the amino acid sequence shown in SEQ ID NO: 68 or 69, and the amino acid sequence of the light chain variable region (VL) consists of the amino acid sequence shown in SEQ ID NO: 70 or 71,
   (b) the amino acid sequence of VH consists of the amino acid sequence shown in SEQ ID NO: 72 or 73, and the amino acid sequence of VL consists of the amino acid sequence shown in SEQ ID NO: 74 or 75,
   (c) the amino acid sequence of VH consists of the amino acid sequence shown in SEQ ID NO: 76 or 77, and the amino acid sequence of VL consists of the amino acid sequence shown in SEQ ID NO: 78 or 79, or
   (d) the amino acid sequence of VH consists of the amino acid sequence shown in SEQ ID NO: 25, 63 or 65, and the amino acid sequence of VL consists of the amino acid sequence shown in SEQ ID NO: 29 or 67.
[5] An antibody against human CXCL1, wherein
   (a) the amino acid sequence of VH consists of the amino acid sequence shown in SEQ ID NO: 68, and the amino acid sequence of VL consists of the amino acid sequence shown in SEQ ID NO: 70,
   (b) the amino acid sequence of VH consists of the amino acid sequence shown in SEQ ID NO: 72, and the amino acid sequence of VL consists of the amino acid sequence shown in SEQ ID NO: 74,
   (c) the amino acid sequence of VH consists of the amino acid sequence shown in SEQ ID NO: 76, and the amino acid sequence of VL consists of the amino acid sequence shown in SEQ ID NO: 78, or
   (d) the amino acid sequence of VH consists of the amino acid sequence shown in SEQ ID NO: 63, and the amino acid sequence of VL consists of the amino acid sequence shown in SEQ ID NO: 67.
[6] The antibody according to any one of [1], [2], [4] and [5] above, wherein the antibody is a human antibody.
[7] The antibody according to any one of [1] to [6] above, wherein the antibody has antitumor activity.
[8] The antibody according to any one of [1] to [7] above, which is used for the treatment or prevention of a tumor.
[9] The antibody according to [7] or [8] above, wherein the tumor is associated with stromal hyperplasia.
[10] The antibody according to any one of [7] to [9] above, wherein the tumor is at least one selected from the group consisting of human gastric cancer, human pancreatic cancer, human breast cancer, human lung cancer, human skin cancer, human ovarian cancer, human colorectal cancer, human bladder cancer, human liver cancer, human esophageal cancer, prostate cancer and human biliary tract cancer.
[11] The antibody according to [10] above, wherein the human gastric cancer is scirrhous gastric cancer.
[12] The antibody according to any one of [1] to [6] above, which has inhibitory or suppressive activity against the migration of CXCR2-expressing cells.
[13] An antibody fragment derived from the antibody according to any one of [1] to [12] above.
[14] A pharmaceutical composition comprising the antibody according to any one of [1] to [12] above and/or the antibody fragment according to [13] above.
[15] The pharmaceutical composition according to [14] above, which is used for the treatment or prevention of a tumor.
[16] The pharmaceutical composition according to [15] above, wherein the tumor is associated with stromal hyperplasia.
[17] The pharmaceutical composition according to [15] or [16] above, wherein the tumor is at least one selected from the group consisting of human gastric cancer, human pancreatic cancer, human breast cancer, human lung cancer, human skin cancer, human ovarian cancer, human colorectal cancer, human bladder cancer, human liver cancer, human esophageal cancer, prostate cancer and human biliary tract cancer.
[18] The pharmaceutical composition according to [17] above, wherein the human gastric cancer is scirrhous gastric cancer.
[19] The pharmaceutical composition according to any one of [14] to [18] above, which further comprises or is used in combination with at least one or more selected from the group consisting of a compound having antitumor activity, a compound having cell killing activity, and an immune checkpoint inhibitor.
[20] The pharmaceutical composition according to [14] above, which is used for the inhibition or suppression of the migration of CXCR2-expressing cells.
[21] A method for treating or preventing a tumor, which comprises administering the antibody according to any one of [1] to [12] above and/or the antibody fragment according to [13] above, or the pharmaceutical composition according to any one of [14] to [19] above to a subject.
[22] The method according to [21] above, wherein the tumor is associated with stromal hyperplasia.
[23] The method according to [21] or [22] above, wherein the tumor is at least one selected from the group consisting of human gastric cancer, human pancreatic cancer, human breast cancer, human lung cancer, human skin cancer, human ovarian cancer, human colorectal cancer, human bladder cancer, human liver cancer, human esophageal cancer, prostate cancer and human biliary tract cancer.
[24] The method according to [23] above, wherein the human gastric cancer is scirrhous gastric cancer.
[25] A method for inhibiting or suppressing the migration of CXCR2-expressing cells, which comprises administering the antibody according to any one of [1] to [12] above and/or the antibody fragment according to [13] above, or the pharmaceutical composition according to [20] above to a subject.
[26] A kit for treating, preventing or diagnosing a tumor, which comprises the antibody according to any one of [1] to [12] above and/or the antibody fragment according to [13] above.
[27] The kit according to [26] above, wherein the tumor is associated with stromal hyperplasia.
[28] The kit according to [26] or [27] above, wherein the tumor is at least one selected from the group consisting of human gastric cancer, human pancreatic cancer, human breast cancer, human lung cancer, human skin cancer, human ovarian cancer, human colorectal cancer, human bladder cancer, human liver cancer, human esophageal cancer, prostate cancer and human biliary tract cancer.
[29] The kit according to [28] above, wherein the human gastric cancer is scirrhous gastric cancer.
[30] An inhibitor or suppressor of the binding of human CXCL and/or mouse CXCL to glucosaminoglycan (GAG), which comprises the antibody according to any one of [1] to [12] above and/or the antibody fragment according to [13] above.

Examples of human CXCL intended here include, but are not limited to, human CXCL1, human CXCL2, human CXCL3 and human CXCL5, while examples of mouse CXCL intended here include, but are not limited to, mouse CXCL1.

### Effects of the Invention

The present invention enables the provision of an anti-human CXCL1 antibody and a fragment thereof, which are capable of inducing alterations in the tumor microenvironment (TME), including the inhibition or suppression of TME formation. The anti-human CXCL1 antibody and fragment thereof according to the present invention are useful, for example, in terms of having antitumor activity and being available for use in the treatment and/or prevention of a tumor, etc.

Moreover, the present invention also enables the provision of a pharmaceutical composition and a kit, etc., each comprising the anti-human CXCL1 antibody or a fragment thereof.

### Brief Description of the Drawings

Figure 1A shows the results of antibodies in an antitumor activity test.
Figure 1B shows the results of antibodies in a migration inhibitory activity test on CXCR2-expressing cells.
Figure 2A shows the results of antibodies in an antitumor activity test.
Figure 2B shows the results of antibodies in a migration inhibitory activity test on CXCR2-expressing cells.
Figure 3A shows the results of antibodies in an antitumor activity test.
Figure 3B shows the results of antibodies in a migration inhibitory activity test on CXCR2-expressing cells.
Figure 4 shows the results of efficacy evaluation for antibodies in a model with OCUM-2MLN orthotopic transplantation.
Figure 5A shows the results of a CXCR2 inhibitor (Navarixin) in a migration inhibitory activity test on CXCR2-expressing cells.
Figure 5B shows the results of a CXCR2 inhibitor (Navarixin) in an antitumor activity test.
Figure 6 shows the results of human and humanized antibodies in an antigen-binding activity test.
Figure 7A shows the results of human and humanized antibodies in an antigen-neutralizing activity test.
Figure 7B shows the results of human and humanized antibodies in an antigen-neutralizing activity test.
Figure 8A shows the results of human and humanized antibodies (Hu4A-2F7, Hu5A-5E11 and ADLib#028-8-12) in a migration inhibitory activity test on CXCR2-expressing cells in a prevention model with OCUM-12 orthotopic transplantation.
Figure 8B shows the results of human and humanized antibodies (Hu4A-2F7, Hu5A-5E11 and ADLib#028-8-12) in an antitumor activity test in a prevention model with OCUM-12 orthotopic transplantation.
Figure 9A shows the results of a humanized antibody (Hu1A-7H10) in a migration inhibitory activity test on CXCR2-expressing cells in a prevention model with OCUM-12 orthotopic transplantation.
Figure 9B shows the results of a humanized antibody (Hu1A-7H10) in an antitumor activity test in a prevention model with OCUM-12 orthotopic transplantation.
Figure 10A shows the results of a humanized antibody, Hu5A-5E11, in a dose-response test for inhibitory activity against the migration of CXCR2-expressing cells in a prevention model with OCUM-12 orthotopic transplantation.
Figure 10B shows the results of a humanized antibody, Hu5A-5E11, in a dose-response test for antitumor activity in a prevention model with OCUM-12 orthotopic transplantation.
Figure 11A shows the results of a humanized antibody, Hu5A-5E11, in a dose-response test for inhibitory activity against the migration of CXCR2-expressing cells in a treatment model with OCUM-12 orthotopic transplantation.
Figure 11B shows the results of a humanized antibody, Hu5A-5E11, in a dose-response test for antitumor activity in a treatment model with OCUM-12 orthotopic transplantation.
Figure 12A shows the results of ELISA-based evaluation for the inhibitory activity of Hu5A-5E11 against the binding between CXCLs and heparin.
Figure 12B shows the results of ELISA-based evaluation for the inhibitory activity of Hu5A-5E11 against the binding between CXCLs and heparan sulfate.

### Description of Embodiments

The present invention will be described in more detail below. The scope of the present invention is not limited by the following descriptions, and any embodiments other than those illustrated below may also be carried out with appropriate modifications without departing from the spirit of the invention.

It should be noted that this specification incorporates the specification of Japanese Patent Application No. 2022-011107 (filed on January 27, 2022) in its entirety, based on which the present application claims priority. Moreover, all publications cited herein, including prior art documents, patent gazettes and other patent documents, are incorporated herein by reference.

### 1. Summary of the present invention

CXCL1 (C-X-C motif chemokine ligand 1) is a member of the CXC family and is also known as a keratinocyte-derived chemokine (KC) or a growth-related oncogene (GRO). CXCL1 is expressed by macrophages, neutrophils and epithelial cells, but is also known to be enhanced in several types of human cancer, and specifically binds to a CXC chemokine receptor, CXCR2 (C-X-C Motif Chemokine Receptor 2).

The present invention relates to an antibody against human CXCL1 (HuCXCL1) (anti-HuCXCL1 antibody) or an antibody fragment thereof, which is capable of binding to HuCXCL1 and also binding to other members of the human CXC family. Moreover, as described above, the present invention is characterized in that the amino acid sequences of complementarity determining region (CDR) 1, CDR2 and CDR3 in the heavy chain variable region (VH) and light chain variable region (VL) of this antibody or the amino acid sequences of the entire VH and VL of this antibody consist of particular sequences.

The anti-HuCXCL1 antibody of the present invention or an antibody fragment thereof is capable of inducing alterations in the tumor microenvironment (TME) to inhibit or suppress the formation of TME, and thereby has antitumor activity. In more detail, stromal cells constituting TME include bone marrow-derived mesenchymal stem cells (BM-MSCs), and these BM-MSCs play a critical role in TME construction. Moreover, in the migration of BM-MSCs toward tumor sites, signal transduction between CXCL1 released from cancer cells or tumor sites and CXCR2 expressed in BM-MSCs plays an important role. When the anti-HuCXCL1 antibody of the present invention or an antibody fragment thereof specifically recognizes CXCL1 released from cancer cells or tumor sites, signal transduction between CXCL1 and CXCR2 is inhibited, whereby the migration of BM-MSCs toward tumor sites is inhibited or suppressed (i.e., the formation of TME is inhibited or suppressed). As a result, the anti-HuCXCL1 antibody of the present invention or an antibody fragment thereof has antitumor effect.

As shown in the Example section described later, as a result of repeating various experiments and studies, the anti-HuCXCL1 antibody of the present invention was obtained as having the above antitumor effect by screening from among as many as about 10,000 types of anti-HuCXCL1 antibody clones. The anti-HuCXCL1 antibody of the present invention or an antibody fragment thereof is useful and highly practical in the treatment and/or prevention of tumors (particularly tumors with stromal hyperplasia), etc.

### 2. Antibody against HuCXCL1 (anti-HuCXCL1 antibody)

### (1) Antigen preparation

Information about the amino acid sequence (SEQ ID NO: 81) of HuCXCL1 has been made public, e.g., on the website of Uniprot (https://www.uniprot.org/) under "Primary (citable) accession number: P09341" or on the website of NCBI (GenBank) (http://www.ncbi.nlm.nih.gov/) under "Accession number: AAP35526." It should be noted that information about the nucleotide sequence (SEQ ID NO: 80) encoding the amino acid sequence of HuCXCL1 has been made public on the website of NCBI (GenBank) under "Accession number: BT006880."

As an antigen, it is possible to use a polypeptide or peptide (hereinafter also simply referred to as a peptide) comprising at least a part (the whole or a part) of the amino acid sequence of HuCXCL1.

The peptide for use as an antigen may be prepared either by chemical synthesis or by synthesis through genetic engineering procedures using *E. coli* or the like, and techniques well known to those skilled in the art may be used for this purpose.

For chemical synthesis, the peptide may be synthesized by well-known peptide synthesis techniques. Moreover, the synthesis may be accomplished by applying either solid phase synthesis or liquid phase synthesis. A commercially available peptide synthesizer (e.g., PSSM-8, Shimadzu Corporation, Japan) may also be used for this purpose.

For peptide synthesis through genetic engineering procedures, DNA encoding the peptide is first designed and synthesized. The design and synthesis may be accomplished, for example, by PCR techniques using a vector or the like containing the full-length HuCXCL1 gene as a template and using primers which have been designed to allow synthesis of a desired DNA region. Moreover, based on the amino acid sequence of the peptide, gene synthesis may also be conducted to obtain DNA comprising a Kozak translation initiation sequence inserted at the 5'-terminal end and a translation termination codon inserted on the 3'-terminal side (e.g., using the services of GENEWIZ). Then, the above DNA may be ligated to an appropriate vector to obtain a recombinant vector for protein expression, and this recombinant vector may be introduced into a host, such that a desired gene can be expressed therein, thereby obtaining a transformant (Molecular cloning 4th Ed. Cold Spring Harbor Laboratory Press (2012)).

As a vector, a phage or plasmid which is autonomously replicable in host microorganisms is used. Further, it is also possible to use an animal virus or insect virus vector. To prepare a recombinant vector, purified DNA may be cleaved with an appropriate restriction enzyme and ligated to a vector by being inserted into, e.g., an appropriate restriction enzyme site in the vector DNA. There is no particular limitation on the host for use in transformation as long as it is capable of expressing a desired gene. Examples include bacteria (e.g., *E. coli, Bacillus subtilis*), yeast, animal cells (e.g., COS cells, CHO cells), insect cells or insects. It is also possible to use a mammal (e.g., goat) as a host. Procedures for introduction of a recombinant vector into a host are known. Moreover, the above transformant may be cultured, and the peptide for use as an antigen may be collected from the cultured product. The term "cultured product" is intended to mean either (a) a culture supernatant, or (b) cultured cells or cultured microorganisms, or a homogenate thereof.

After culture, when the desired peptide is produced within microorganisms or cells, the microorganisms or cells may be homogenized to thereby extract the peptide. Alternatively, when the desired peptide is produced outside microorganisms or cells, the cultured solution may be used directly or treated by centrifugation or other techniques to remove the microorganisms or cells. Then, the desired peptide may be isolated and purified by biochemical techniques commonly used for isolation and purification of peptides, as exemplified by ammonium sulfate precipitation, gel filtration, ion exchange chromatography, affinity chromatography and so on, which may be used either alone or in combination as appropriate.

The peptide for use as an antigen may also be obtained by *in vitro* translation using a cell-free synthesis system. In this case, it is possible to use two methods, i.e., a method in which RNA is used as a template and a method in which DNA is used as a template (transcription/translation). As a cell-free synthesis system, a commercially available system may be used, as exemplified by Expressway^{™} system (Invitrogen), PURESYSTEM^{®} (Post Genome Institute Co., Ltd., Japan), TNT system^{®} (Promega), etc.

The peptide obtained as described above may also be linked to an appropriate carrier protein such as bovine serum albumin (BSA), keyhole limpet hemocyanin (KLH), human thyroglobulin, avian gamma globulin, etc.

Moreover, the antigen may be a peptide consisting of an amino acid sequence with deletion, substitution or addition of one or more amino acids in the amino acid sequence of HuCXCL1 (SEQ ID NO: 81) or its partial sequence as described above. For example, it is also possible to use a peptide consisting of an amino acid sequence with deletion of one or more (preferably one or several (e.g., 1 to 10, more preferably 1 to 5)) amino acids, with substitution of other amino acids for one or more (preferably one or several (e.g., 1 to 10, more preferably 1 to 5)) amino acids, or with addition of one or more (preferably one or several (e.g., 1 to 10, more preferably 1 to 5)) other amino acids in the amino acid sequence of HuCXCL1 or its partial sequence.

The gene to be introduced into cells or the like may be a gene encoding a HuCXCL1 protein or a partial fragment thereof or a mutated protein or fragment thereof. For example, it is possible to use a gene having the nucleotide sequence shown in SEQ ID NO: 80 or a partial sequence thereof for this purpose.

Alternatively, as a gene to be introduced into cells or the like, it is also possible to use a nucleotide sequence encoding a protein having the same activity as HuCXCL1, or a partial sequence thereof, which is hybridizable under stringent conditions with a sequence complementary to the nucleotide sequence shown in SEQ ID NO: 80.

The term "stringent conditions" refers to washing conditions after hybridization, and is intended to mean conditions where the salt (sodium) concentration in buffer is 10 to 500 mM and the temperature is 42°C to 72°C, preferably where the above salt concentration is 50 to 300 mM and the temperature is 55°C to 68°C.

For introduction of mutations into a gene, known techniques (e.g., Kunkel method or Gapped duplex method) may be used for this purpose. For example, it is possible to use a kit for mutation introduction based on site-directed mutagenesis, as exemplified by GeneTailor^{™} Site-Directed Mutagenesis System (Invitrogen), TaKaRa Site-Directed Mutagenesis System (e.g., Mutan-K, Mutan-Super Express Km; Takara Bio Inc., Japan).

### (2) Preparation of polyclonal antibodies

The antigen prepared above is administered to a mammal for the purpose of immunization. Such a mammal is not limited in any way, and examples include rats, mice and rabbits, with mice being particularly preferred.

The amount of the antigen to be administered per animal may be determined, as appropriate, depending on the presence or absence of an adjuvant. Examples of an adjuvant include Freund's complete adjuvant (FCA), Freund's incomplete adjuvant (FIA), aluminum hydroxide adjuvant and so on. Immunization may be primarily accomplished by injection via the intravenous, footpad, subcutaneous or intraperitoneal route, etc. Moreover, the interval between immunizations is not limited in any way, and immunization may be repeated once to 10 times, preferably twice to three times, at intervals of several days to several weeks, preferably at intervals of 1 week. Further, at 3 to 7 days after the day of the final immunization, the animals are measured for their antibody titers by enzyme immunoassay (ELISA or EIA) or radioactive immunoassay (RIA), etc., and blood may be collected at the day when each animal shows the desired antibody titer, thereby obtaining antisera. In cases where antibodies are required to be purified in the above antibody collection procedures, known techniques such as salting out with ammonium sulfate, ion exchange chromatography, gel filtration chromatography, affinity chromatography and so on may be selected as appropriate or used in combination for purification purposes. Then, polyclonal antibodies in the antisera are measured for their reactivity by ELISA assay, etc.

### (3) Preparation of monoclonal antibody

### (3-1) Collection of antibody-producing cells

The anti-HuCXCL1 antibody of the present invention is not limited in any way, but is preferably a monoclonal antibody.

The antigen prepared above is administered to a mammal (e.g., rat, mouse, rabbit) for the purpose of immunization. The amount of the antigen to be administered per animal may be determined, as appropriate, depending on the presence or absence of an adjuvant. Examples of an adjuvant are the same as described above. Immunization procedures are also the same as described above. Further, at 1 to 60 days, preferably 1 to 14 days, after the day of the final immunization, antibody-producing cells are collected. Antibody-producing cells may be exemplified by spleen cells, lymph node cells and peripheral blood cells, etc., with lymph node cells or spleen cells being particularly preferred.

### (3-2) Cell fusion

To obtain hybridomas (antibody-producing cell lines), cell fusion is conducted between antibody-producing cells and myeloma cells. As myeloma cells to be fused with antibody-producing cells, it is possible to use generally available established cell lines of mouse or other animal origin. Cell lines preferred for use are those having drug selectivity and having the property of not surviving in HAT selective medium (i.e., a medium containing hypoxanthine, aminopterin and thymidine) in an unfused state, but surviving only when fused with antibody-producing cells.

Examples of myeloma cells include mouse myeloma cell lines, as exemplified by P3-X63-Ag8.653, P3-X63-Ag8(X63), P3-X63-Ag8.U1(P3U1), P3/NS I/1-Ag4-1(NS1) and Sp2/0-Ag14(Sp2/0), etc. Myeloma cells may be selected as appropriate in consideration of their compatibility with antibody-producing cells.

Then, myeloma cells and antibody-producing cells are provided for cell fusion. For cell fusion, in a serum-free medium for animal cell culture (e.g., DMEM or RPMI-1640 medium), 1 × 10⁶ to 1 × 10⁷/mL of antibody-producing cells are mixed with 2 × 10⁵ to 2 × 10⁶/mL of myeloma cells. The ratio of antibody-producing cells to myeloma cells (antibody-producing cells:myeloma cells) is not limited in any way, but is usually set to preferably 1:1 to 10:1, more preferably 3:1. Then, fusion reaction is conducted in the presence of a cell fusion promoter. As a cell fusion promoter, it is possible to use polyethylene glycol having an average molecular weight of 1,000 to 6,000 daltons (D), etc. Alternatively, a commercially available cell fusion apparatus using electrical stimulation (e.g., electroporation) may also be used to cause cell fusion between antibody-producing cells and myeloma cells.

### (3-3) Screening and cloning of hybridomas

After cell fusion, the cells are screened to select desired hybridomas. For screening, a cell suspension may be diluted as appropriate with, e.g., RPMI-1640 medium containing fetal bovine serum and then seeded on microtiter plates, and a selective medium may be added to each well, followed by culture while replacing the selective medium as appropriate. As a result, cells growing at around 14 days after the initiation of culture in the selective medium may be obtained as hybridomas.

Subsequently, the growing hybridomas are screened as to whether an antibody reactive to HuCXCL1 is present in their culture supernatants. Screening of these hybridomas is not limited in any way and may be conducted in accordance with commonly used procedures. For example, aliquots of the culture supernatants contained in the wells where hybridomas have grown may be sampled and screened by ELISA, EIA and RIA, etc.

The fused cells may be cloned by limiting dilution or other techniques. An antibody strongly reactive to HuCXCL1 is determined by flow cytometry or other techniques, and a hybridoma producing this antibody is selected and established as a clone.

### (3-4) Collection of monoclonal antibody

For culture of the establish hybridoma and collection of a monoclonal antibody from the resulting cultured product, commonly used procedures, e.g., cell culture-based procedures or ascites formation procedures may be used for this purpose. The term "culture" is intended to mean that a hybridoma is allowed to grow in a culture dish or a culture bottle, or that a hybridoma is allowed to proliferate in the abdominal cavity of an animal as described below.

In cell culture-based procedures, the hybridoma may be cultured in an animal cell culture medium (e.g., 10% fetal bovine serum-containing RPMI-1640 medium, MEM medium or serum-free medium) under standard culture conditions (e.g., 37°C, 5% CO₂ concentration) for 7 to 14 days to obtain an antibody from its culture supernatant.

In the case of ascites formation procedures, the hybridoma may be intraperitoneally administered at about 1 × 10⁷ cells to an animal of the same species as the mammal from which myeloma cells are derived, whereby the hybridoma is allowed to proliferate in abundance. Then, its ascites is preferably collected after 2 to 3 weeks.

In cases where the antibody is required to be purified in the above antibody collection procedures, known techniques such as salting out with ammonium sulfate, ion exchange chromatography, gel filtration, affinity chromatography and so on may be selected as appropriate or used in combination for purification purposes.

### (3-5) Selection of clone having antitumor activity

The anti-HuCXCL1 antibody of the present invention is preferably an antibody having antitumor activity, by way of example.

As used herein, the term "antitumor activity" is intended to mean tumor cell (cancer cell) killing activity or tumor growth inhibitory activity. Antitumor activity is preferably exemplified by cancer cell growth inhibitory activity and tumor angiogenesis inhibitory activity. As to the type of human tumor (tumor cells) against which the antibody of the present invention can exert antitumor activity, examples include various known human tumors which have been confirmed to express HuCXCL1, preferably those associated with stromal hyperplasia, without being limited thereto. In more detail, such human tumors preferably include one or two or more of various human tumors such as human gastric cancer, human pancreatic cancer, human breast cancer, human lung cancer, human skin cancer, human ovarian cancer, human colorectal cancer, human bladder cancer, human liver cancer, human esophageal cancer, prostate cancer and human biliary tract cancer, with human gastric cancer being more preferred and scirrhous gastric cancer being particularly preferred. Further, the above tumors may be of recurrent or metastatic type, and the antibody of the present invention may also exert excellent antitumor activity against these tumors.

The presence of *in vivo* antitumor activity may be confirmed, for example, by using a cancer-bearing mouse (cancer-bearing animal therapeutic model) transplanted subcutaneously with desired tumor cells, and administering this mouse with the antibody obtained as described above. In this case, the antibody may be administered either immediately after transplantation of tumor cells (prevention model) or after confirming that the tumor has grown to a certain volume after transplantation (treatment model). The antibody may be administered in any manner, for example, may be administered once every 3 days, 1 week, 10 days or 2 weeks or singly (i.e., only once) at a dose of 5 to 20 mg/kg body weight via the intraperitoneal route, etc. In the case of the prevention model, the presence or absence of antitumor activity and the level thereof may be evaluated on the basis of tumorigenesis frequency and tumor volume. In the case of the treatment model, the presence or absence of antitumor activity and the level thereof may be evaluated on the basis of tumor volume.

Examples of the anti-HuCXCL1 antibody in the present invention include those in which
(a) the amino acid sequences of heavy chain variable region (VH) complementarity determining region (CDR) 1, CDR2 and CDR3 consist of:
   the amino acid sequences shown in SEQ ID NOs: 2, 3 and 4, respectively; or
   the amino acid sequences shown in SEQ ID NOs: 2, 34 and 4, respectively
   (particularly preferably the amino acid sequences shown in SEQ ID NOs: 2, 34 and 4, respectively),
      and
   the amino acid sequences of light chain variable region (VL) CDR1, CDR2 and CDR3 consist of:
      the amino acid sequences shown in SEQ ID NOs: 6, 7 and 8, respectively,
(b) the amino acid sequences of VH CDR1, CDR2 and CDR3 consist of:
   the amino acid sequences shown in SEQ ID NOs: 10, 11 and 12, respectively, and
   the amino acid sequences of VL CDR1, CDR2 and CDR3 consist of:
      the amino acid sequences shown in SEQ ID NOs: 14, 15 and 16, respectively;
      the amino acid sequences shown in SEQ ID NOs: 14, 36 and 16, respectively;
      the amino acid sequences shown in SEQ ID NOs: 14, 38 and 16, respectively; or
      the amino acid sequences shown in SEQ ID NOs: 14, 40 and 16, respectively
      (particularly preferably the amino acid sequences shown in SEQ ID NOs: 14, 36 and 16, respectively),
(c) the amino acid sequences of VH CDR1, CDR2 and CDR3 consist of:
   the amino acid sequences shown in SEQ ID NOs: 18, 19 and 20, respectively;
   the amino acid sequences shown in SEQ ID NOs: 18, 42 and 20, respectively;
   the amino acid sequences shown in SEQ ID NOs: 18, 44 and 20, respectively;
   the amino acid sequences shown in SEQ ID NOs: 18, 46 and 20, respectively;
   the amino acid sequences shown in SEQ ID NOs: 18, 48 and 20, respectively;
   the amino acid sequences shown in SEQ ID NOs: 18, 50 and 20, respectively;
   the amino acid sequences shown in SEQ ID NOs: 18, 52 and 20, respectively;
   the amino acid sequences shown in SEQ ID NOs: 18, 54 and 20, respectively;
   the amino acid sequences shown in SEQ ID NOs: 18, 56 and 20, respectively;
   the amino acid sequences shown in SEQ ID NOs: 18, 58 and 20, respectively;
   the amino acid sequences shown in SEQ ID NOs: 18, 60 and 20, respectively; or
   the amino acid sequences shown in SEQ ID NOs: 18, 62 and 20, respectively
   (particularly preferably the amino acid sequences shown in SEQ ID NOs: 18, 54 and 20, respectively),
      and
   the amino acid sequences of VL CDR1, CDR2 and CDR3 consist of:
      the amino acid sequences shown in SEQ ID NOs: 22, 23 and 24, respectively, and
(d) the amino acid sequences of VH CDR1, CDR2 and CDR3 consist of:
   the amino acid sequences shown in SEQ ID NOs: 26, 27 and 28, respectively;
   the amino acid sequences shown in SEQ ID NOs: 26, 64 and 28, respectively; or
   the amino acid sequences shown in SEQ ID NOs: 26, 66 and 28, respectively
   (particularly preferably the amino acid sequences shown in SEQ ID NOs: 26, 64 and 28, respectively)
      and
   the amino acid sequences of VL CDR1, CDR2 and CDR3 consist of:
      the amino acid sequences shown in SEQ ID NOs: 30, 31 and 32, respectively.

Moreover, the anti-HuCXCL1 antibody in the present invention also preferably includes antibodies (anti-HuCXCL1 monoclonal antibodies) produced from the above deposited various hybridomas.

### (3-6) Epitope for anti-HuCXCL1 antibody

An epitope (antigenic determinant) for the anti-HuCXCL1 antibody in the present invention may be a region of at least a part of the antigen HuCXCL1. The anti-HuCXCL1 antibody recognizing such a region (i.e., binding to such a region or a portion containing the same) is useful, for example, because it can more effectively exert the properties of the anti-HuCXCL1 antibody as described later.

Moreover, antibodies capable of binding to an epitope region to which the anti-HuCXCL1 antibody in the present invention binds (recognizes) also fall within the present invention.

### (3-7) Properties of anti-HuCXCL1 antibody

The anti-HuCXCL1 antibody of the present invention is an antibody having antitumor activity, as described above, and is preferably an antibody showing tumor growth inhibitory activity in a cancer-bearing animal model, by way of example. This tumor growth inhibitory activity is preferably shown at a lower dose, for example, preferably at a dose of 20 mg/kg body weight or less (preferably 10 mg/kg body weight or less, more preferably 5 mg/kg body weight or less, even more preferably 1 mg/kg body weight or less) in a cancer-bearing animal model.

For example, the tumor growth inhibitory activity (%) can be calculated according to the following equation. Tumor growth inhibitory activity (%) = 100 - [(tumor volume or tumor weight in the antibody-receiving group)/(tumor volume or tumor weight in the control group)] × 100

Moreover, the anti-HuCXCL1 antibody of the present invention is preferably an antibody having inhibitory or suppressive activity against the migration of CXCR2-expressing cells (more specifically BM-MSCs, etc.), as described above.

For example, the inhibitory or suppressive activity (%) against the migration of CXCR2-expressing cells can be calculated according to the following equation. Migration inhibitory or suppressive activity (%) = 100 - [(the number of migrated cells per unit area in the antibody-receiving group)/(the number of migrated cells per unit area in the control group)] × 100

Further, the anti-HuCXCL1 antibody of the present invention preferably has a dissociation constant (Kd value) of 1.0 × 10⁻¹⁰ M or less, more preferably 1.0 × 10⁻¹¹ M or less, and even more preferably 1.0 × 10⁻¹² M or less, without being limited thereto. The antibody's binding ability (affinity) may be measured as a dissociation constant (Kd value), dissociation rate constant (Kdiss [1/Sec]) or association rate constant (Kass [1/M.Sec]), for example, by Scatchard analysis or with a surface plasmon resonance sensor called Biacore. Examples of a Biacore apparatus include Biacore 3000, Biacore 2000, Biacore X, Biacore J, Biacore Q (all from Biacore) and so on. The antibody is preferred in terms of having higher binding ability (affinity) at a lower dissociation constant (Kd value). The Kd value is determined by two parameters, i.e., Kdiss and Kass, and can be expressed by the equation: Kd [M] = Kdiss/Kass, by way of example.

### (4) Genetically recombinant antibodies and antibody fragments

### (4-1) Genetically recombinant antibodies

A preferred embodiment of the anti-HuCXCL1 antibody of the present invention may be a genetically recombinant antibody. Examples of a genetically recombinant antibody include, but are not limited to, a chimeric antibody, a humanized antibody, and a human antibody (a complete human antibody), etc.

A chimeric antibody (i.e., a humanized chimeric antibody) is an antibody in which antibody variable regions of mouse origin are linked (conjugated) to constant regions of human origin (see, e.g., Proc. Natl. Acad. Sci. U.S.A. 81, 6851-6855 (1984)). For preparation of a chimeric antibody, gene recombination technology may be used for easy construction such that the thus linked antibody is obtained.

For preparation of a humanized antibody, complementarity determining regions (CDRs) from mouse antibody variable regions are grafted into human variable regions to prepare reconstituted variable regions whose framework regions (FRs) are of human origin and whose CDRs are of mouse origin (so-called CDR grafting). Then, these humanized reconstituted human variable regions are linked to human constant regions. Procedures for preparation of such a humanized antibody have been well known in the art (see Nature, 321, 522-525 (1986); J. Mol. Biol., 196, 901-917 (1987); Queen C et al., Proc. Natl. Acad. Sci. USA, 86: 10029-10033 (1989); JP H04-502408 A (Japanese Patent No. 2828340; Queen et al.), etc.).

Preferred examples of the anti-HuCXCL1 antibody of the present invention as a chimeric antibody include those in which
(a) the amino acid sequence of the heavy chain variable region (VH) consists of the amino acid sequence shown in SEQ ID NO: 1 or 33, and the amino acid sequence of the light chain variable region (VL) consists of the amino acid sequence shown in SEQ ID NO: 5,
(b) the amino acid sequence of VH consists of the amino acid sequence shown in SEQ ID NO: 9, and the amino acid sequence of VL consists of the amino acid sequence shown in SEQ ID NO: 13, 35, 37 or 39, and
(c) the amino acid sequence of VH consists of the amino acid sequence shown in SEQ ID NO: 17, 41, 43, 45, 47, 49, 51, 53, 55, 57, 59 or 61, and the amino acid sequence of VL consists of the amino acid sequence shown in SEQ ID NO: 21.

Further, preferred examples of the anti-HuCXCL1 antibody of the present invention as a humanized antibody include those in which
(a) the amino acid sequence of the heavy chain variable region (VH) consists of the amino acid sequence shown in SEQ ID NO: 68 or 69 (particularly preferably the amino acid sequence shown in SEQ ID NO: 68), and the amino acid sequence of the light chain variable region (VL) consists of the amino acid sequence shown in SEQ ID NO: 70 or 71 (particularly preferably the amino acid sequence shown in SEQ ID NO: 70),
(b) the amino acid sequence of VH consists of the amino acid sequence shown in SEQ ID NO: 72 or 73 (particularly preferably the amino acid sequence shown in SEQ ID NO: 72), and the amino acid sequence of VL consists of the amino acid sequence shown in SEQ ID NO: 74 or 75 (particularly preferably the amino acid sequence shown in SEQ ID NO: 74), and
(c) the amino acid sequence of VH consists of the amino acid sequence shown in SEQ ID NO: 76 or 77 (particularly preferably the amino acid sequence shown in SEQ ID NO: 76), and the amino acid sequence of VL consists of the amino acid sequence shown in SEQ ID NO: 78 or 79 (particularly preferably the amino acid sequence shown in SEQ ID NO: 78).

A human antibody (complete human antibody) is usually an antibody in which hyper variable regions serving as antigen-binding sites in the V regions, the other portions in the V regions and the constant regions have the same structures as those of an antibody of human origin. However, hyper variable regions may be of other animal origin. Techniques to prepare a human antibody have also been known, and a method through genetic engineering procedures has been established for the preparation of gene sequences common to humans. Such a human antibody may be obtained, for example, by using human antibody-producing mice carrying human chromosome fragments containing genes for human antibody H and L chains (see, e.g., Tomizuka, K. et al., Nature Genetics, (1977) 16, 133-143; Kuroiwa, Y. et al., Nuc. Acids Res., (1998) 26, 3447-3448; Yoshida, H. et al., Animal Cell Technology: Basic and Applied Aspects, (1999) 10, 69-73 (Kitagawa, Y., Matuda, T. and Iijima, S. eds.), Kluwer Academic Publishers; Tomizuka, K. et al., Proc. Natl. Acad. Sci. USA, (2000) 97, 722-727) or by screening human antibody libraries to obtain a phage display-derived human antibody (see, e.g., Wormstone, I. M. et al, Investigative Ophthalmology & Visual Science., (2002) 43 (7), 2301-8; Carmen, S. et al., Briefings in Functional Genomics and Proteomics, (2002) 1 (2), 189-203; Siriwardena, D. et al., Opthalmology, (2002) 109 (3), 427-431).

Alternatively, as a human antibody, it is also possible to use an antibody produced (and further purified) from a clone specifically binding to a desired antigen (i.e., HuCXCL1 in the present invention) by using the human ADLib technique (the library shown in WO 2015/167011).

Preferred examples of the anti-HuCXCL1 antibody of the present invention as a human antibody include those in which
the amino acid sequence of VH consists of the amino acid sequence shown in SEQ ID NO: 25, 63 or 65 (particularly preferably the amino acid sequence shown in SEQ ID NO: 63), and the amino acid sequence of VL consists of the amino acid sequence shown in SEQ ID NO: 29 or 67 (particularly preferably the amino acid sequence shown in SEQ ID NO: 67).

The above chimeric, humanized and human antibodies are configured such that the N-glycoside-linked complex sugar chain in the antibody Fc region preferably has no fucose linked to N-acetylglucosamine at the reducing terminal of the sugar chain, as specifically exemplified by antibodies composed of genetically recombinant antibody molecules whose Fc region has a sugar chain in which the 1-position of the fucose is not α-liked to the 6-position of N-acetylglucosamine at the reducing terminal of the N-glycoside-linked complex sugar chain. Such an antibody allows an improvement in ADCC activity. It should be noted that this point (i.e., the characteristics of the N-glycoside-linked complex sugar chain in the antibody Fc region) is also preferred for the above polyclonal and monoclonal antibodies.

### (4-2) Antibody fragments

A fragment (partial fragment) of the anti-HuCXCL1 antibody of the present invention also falls within the antibody of the present invention. The antibody fragment of the present invention has binding activity to HuCXCL1 (i.e., is capable of binding to HuCXCL1), as in the case of the anti-HuCXCL1 antibody of the present invention, and preferably has inhibitory or suppressive activity against the migration of CXCR2-expressing cells, as described above, and thereby has antitumor activity.

A fragment of the antibody is intended to mean a partial region of the anti-HuCXCL1 polyclonal antibody or the anti-HuCXCL1 monoclonal antibody (i.e., an antibody fragment derived from the anti-HuCXCL1 antibody of the present invention), and examples include Fab, Fab', F(ab')₂, Fv (variable fragment of antibody), single chain antibody (e.g., H chain, L chain, H chain V region, and L chain V region), scFv, diabody (scFv dimer), dsFv (disulfide stabilized V region), as well as peptides at least partially containing complementarity determining regions (CDRs), etc.

Fab is an antibody fragment having a molecular weight of about 50,000 and having antigen binding activity, which is composed of the N-terminal half of H chain and the full length of L chain linked via a disulfide bond, among fragments obtained by treating an antibody molecule with a protease, papain. Alternatively, Fab may also be prepared as follows: DNA encoding antibody Fab is inserted into a prokaryotic or eukaryotic expression vector, and this vector is introduced into a prokaryotic or eukaryotic organism for expression.

F(ab')₂ is an antibody fragment having a molecular weight of about 100,000 and having antigen binding activity, which is slightly larger than that composed of Fab fragments linked via disulfide bonds in the hinge region, among fragments obtained by treating an antibody molecule with a protease, pepsin. Alternatively, F(ab')₂ may also be prepared by linking Fab fragments described later via thioether bonds or disulfide bonds.

Fab' is an antibody fragment having a molecular weight of about 50,000 and having antigen binding activity, which is obtained by cleaving the disulfide bonds in the hinge region of the above F(ab')₂. Alternatively, Fab' may also be prepared as follows: DNA encoding an antibody Fab' fragment is inserted into a prokaryotic or eukaryotic expression vector, and this vector is introduced into a prokaryotic or eukaryotic organism for expression.
scFv is an antibody fragment having antigen binding activity, which is composed of a single heavy chain variable region (VH) and a single light chain variable region (VL) linked via an appropriate peptide linker (P), i.e., a VH-P-VL or VL-P-VH polypeptide. For preparation of scFv, cDNAs encoding antibody VH and VL may be obtained to construct DNA encoding scFv, and this DNA may be inserted into a prokaryotic or eukaryotic expression vector, followed by introducing this expression vector into a prokaryotic or eukaryotic organism for expression.

Diabody is an antibody fragment composed of dimerized scFv fragments and having divalent antigen binding activity. The divalent antigen binding activity may be directed to the same antigen or to different antigens. For preparation of diabody, cDNAs encoding antibody VH and VL may be obtained to construct DNA encoding scFv such that the amino acid sequence of P has a length of 8 residues or less, and this DNA may be inserted into a prokaryotic or eukaryotic expression vector, followed by introducing this expression vector into a prokaryotic or eukaryotic organism for expression.

dsFv is an antibody fragment composed of VH and VL polypeptides, in each of which a single amino acid residue is replaced with a cysteine residue and which are linked via a disulfide bond between these cysteine residues. An amino acid residue to be replaced with a cysteine residue can be selected based on three-dimensional structure prediction of antibody according to the method reported by Reiter et al. (Protein Engineering, 7, 697-704, 1994). For preparation of dsFv, cDNAs encoding antibody VH and VL may be obtained to construct DNA encoding dsFv, and this DNA may be inserted into a prokaryotic or eukaryotic expression vector, followed by introducing this expression vector into a prokaryotic or eukaryotic organism for expression.

A CDR-containing peptide is configured to comprise at least one or more regions of VH CDRs (CDRs 1 to 3) and VL CDRs (CDRs 1 to 3), more preferably configured to comprise all VH CDRs or all VL CDRs, and particularly preferably configured to comprise all VH and VL CDRs (i.e., 6 regions in total). The amino acid sequences of CDRs are preferably exemplified by the various amino acid sequences of VH and VL CDRs 1 to 3 mentioned above. In the case of a peptide containing a plurality of CDRs, these CDRs may be linked directly or through an appropriate peptide linker. For preparation of a CDR-containing peptide, DNA encoding CDR in antibody VH or VL may be constructed, and the DNA may be inserted into a prokaryotic or eukaryotic expression vector, followed by introducing this expression vector into a prokaryotic or eukaryotic organism for expression. Alternatively, a CDR-containing peptide may also be prepared by chemical synthesis such as Fmoc (fluorenylmethyloxycarbonyl) and tBoc (t-butyloxycarbonyl) methods.

The antibody fragments of the present invention may be antibody fragments comprising a part or the whole of the antibody Fc region whose N-glycoside-linked complex sugar chain has no fucose linked to N-acetylglucosamine at the reducing terminal of the sugar chain, or alternatively, may be fusion proteins of the antibody fragments mentioned above with a part or the whole of the antibody Fc region whose N-glycoside-linked complex sugar chain has no fucose linked to N-acetylglucosamine at the reducing terminal of the sugar chain. Such antibody fragments allow a dramatic improvement in ADCC activity and are therefore preferred.

In the following descriptions in this specification, the above antibody fragments also fall within the anti-HuCXCL1 antibody of the present invention.

### 3. Polynucleotide, recombinant vector and transformant

The present invention also enables the provision of a polynucleotide (gene, DNA) encoding the above anti-HuCXCL1 antibody of the present invention or an antibody fragment thereof. In more detail, such a polynucleotide is preferably a polynucleotide comprising a nucleotide sequence encoding any of the amino acid sequences shown as examples for the above anti-HuCXCL1 antibody of the present invention or an antibody fragment thereof. Moreover, the polynucleotide of the present invention may consist only of a polynucleotide encoding the anti-HuCXCL1 antibody of the present invention or an antibody fragment thereof, or may comprise, in addition to this polynucleotide, known nucleotide sequences (e.g., a transcription promoter, an SD sequence, a Kozak sequence, a terminator) required for gene expression, without being limited thereto.

Such a polynucleotide encoding the anti-HuCXCL1 antibody of the present invention or an antibody fragment thereof has no particular limitation on its codons corresponding to individual amino acids after translation, and may comprise nucleotide DNA showing codons commonly used (preferably codons frequently used) in humans or other mammals after transcription or may comprise nucleotide DNA showing codons commonly used (preferably codons frequently used) in microorganisms (e.g., *E. coli* or yeast) or plants, etc., after transcription.

The present invention also enables the provision of a recombinant vector comprising the above polynucleotide of the present invention and a transformant comprising the recombinant vector.

A polynucleotide (gene, DNA) to be integrated into an expression vector used as a recombinant vector may optionally be pre-ligated upstream with a transcription promoter, an SD sequence (when prokaryotic cells are used as a host) and a Kozak sequence (when eukaryotic cells are used as a host) and pre-ligated downstream with a terminator, and may also be pre-ligated with an enhancer, a splicing signal, a poly-A addition signal, a selective marker, etc. It should be noted that individual elements required for gene expression including a transcription promoter as mentioned above may be contained initially in the polynucleotide, or alternatively, those contained originally in an expression vector, if any, may be used. There is no particular limitation on the mode of use of each element.

For integration of such a polynucleotide into an expression vector, various known techniques based on gene recombination technology may be used, including those using restriction enzymes, those using topoisomerases, etc. Moreover, an expression vector is not limited in any way as long as it may carry a polynucleotide (gene, DNA) encoding the anti-HuCXCL1 antibody of the present invention or an antibody fragment thereof, as exemplified by plasmid DNA, bacteriophage DNA, retrotransposon DNA, a retroviral vector, artificial chromosomal DNA, etc., and a vector suitable for host cells to be used may be selected as appropriate for use.

Then, the above recombinant vector thus constructed may be introduced into a host to obtain a transformant, and this transformant may be cultured to allow the expression of the anti-HuCXCL1 antibody of the present invention or an antibody fragment thereof. It should be noted that the term "transformant" in the context of the present invention is intended to mean a host into which a foreign gene has been introduced, and examples include a host into which a foreign gene has been introduced by introduction of plasmid DNA or the like (transformation) and a host into which a foreign gene has been introduced by infection with various viruses and phages (transduction).

Such a host is not limited in any way and may be selected as appropriate as long as it allows the expression of the anti-HuCXCL1 antibody of the present invention or an antibody fragment thereof after introduction of the above recombinant vector, and examples include known hosts such as various animal cells (e.g., human and mouse cells), various plant cells, bacteria, yeast, plant cells, etc.

When animal cells are used as a host, examples include human fibroblasts, human fetal kidney cells, HEK293 cells, 293F cells, CHO cells, monkey cells COS-7, Vero, mouse L cells, rat GH3, human FL cells, etc. Alternatively, insect cells such as Sf9 cells and Sf21 cells may also be used.

When bacteria are used as a host, examples include *E. coli, Bacillus subtilis,* etc.

When yeast is used as a host, examples include *Saccharomyces cerevisiae, Schizosaccharomyces pombe,* etc.

When plant cells are used as a host, examples include tobacco BY-2 cells, etc.

Procedures for obtaining a transformant are not limited in any way and may be selected as appropriate in consideration of the combination between host and expression vector types. Preferred examples include electroporation, lipofection, heat shock transfection, PEG transfection, calcium phosphate transfection, DEAE-dextran transfection, and infection with various viruses (e.g., DNA virus, RNA virus).

In the resulting transformant, the codon types of the polynucleotide contained in the recombinant vector are not limited in any way and may be either identical with or different from the codon types in the host actually used.

### 4. Pharmaceutical composition and others

The anti-HuCXCL1 antibody of the present invention is useful as an active ingredient contained in a pharmaceutical composition.

Because of having antitumor activity, the anti-HuCXCL1 antibody of the present invention is preferably used for the treatment and/or prevention of a tumor; and hence such a pharmaceutical composition is useful as a pharmaceutical composition for the treatment and/or prevention of a tumor, and further for the diagnosis of a tumor. Namely, the anti-HuCXCL1 antibody of the present invention is useful as an active ingredient contained in a therapeutic agent for a tumor and a diagnostic agent for a tumor. It should be noted that in the present invention, the above treatment of a tumor is intended to also include the inhibition and suppression of tumor growth. In more detail, for example, a therapeutic agent for a tumor may also take the form of an inhibitor of tumor growth or a suppressor of tumor growth.

Moreover, the pharmaceutical composition of the present invention may be a composition for inhibiting or suppressing the migration of CXCR2-expressing cells (more specifically BM-MSCs, etc.).

The pharmaceutical composition of the present invention is preferably provided in the form of a pharmaceutical composition comprising the anti-HuCXCL1 antibody of the present invention as an active ingredient and further comprising a pharmaceutically acceptable carrier. Moreover, the pharmaceutical composition of the present invention may be combined with any one or two or more of known drugs, for example, a compound having antitumor activity (e.g., cisplatin), a compound having cell killing activity, and an immune checkpoint inhibitor (e.g., a PD-1 inhibitor, a PD-L1 inhibitor, a PD-L2 inhibitor). The embodiment of combined use is not limited in any way, and may be, for example, an embodiment where the pharmaceutical composition of the present invention further comprises such a compound, or an embodiment where the pharmaceutical composition of the present invention is used in combination with such a compound. Such combined use gives a higher antitumor effect. It should be noted that the above immune checkpoint inhibitor is also referred to as a so-called PD-1 pathway antagonist, and includes those capable of inhibiting or suppressing immunosuppressive signals mediated by PD-1 expressed on T cells and its ligand PD-L1 or PD-L2. Specific examples of a PD-1 pathway antagonist include an anti-PD-1 antibody, an anti-PD-L1 antibody, an anti-PD-L2 antibody, a PD-1 extracellular domain, a PD-L1 extracellular domain, a PD-L2 extracellular domain, PD-1-Ig (i.e., a fusion protein of a PD-1 extracellular domain with the FC region of Ig), PD-L1-Ig, PD-L2-Ig and so on. Among them, an anti-PD-1 antibody, an anti-PD-L1 antibody and an anti-PD-L2 antibody are preferred, and an anti-PD-1 antibody and an anti-PD-L1 antibody are more preferred. The above antibodies may each be in the form of, for example, an F(ab')₂, Fab', Fab or Fv fragment. The above antibodies each include either known or commercially available antibodies. For example, the anti-PD-1 antibody may be exemplified by pembrolizumab, nivolumab, cemiplimab, dostarlimab, and zimberelimab, etc., while the anti-PD-L1 antibody may be exemplified by atezolizumab, durvalumab, avelumab, etc. The above antibodies also include their biosimilars.

Diseases (human tumors) to be applied by the pharmaceutical composition of the present invention include various known human tumors which have been confirmed to express HuCXCL1. In more detail, such human tumors preferably include one or two or more of various human tumors such as human gastric cancer, human pancreatic cancer, human breast cancer, human lung cancer, human skin cancer, human ovarian cancer, human colorectal cancer, human bladder cancer, human liver cancer, human esophageal cancer, prostate cancer and human biliary tract cancer, with human gastric cancer being more preferred and scirrhous gastric cancer being particularly preferred. These tumors may occur either alone or in combination. Moreover, tumors to be applied may be recurrent cancers or metastatic cancers, and the pharmaceutical composition of the present invention (and thus the anti-HuCXCL1 antibody of the present invention) can also be effectively used as a therapeutic agent, a prophylactic agent or a diagnostic agent for recurrent cancers or metastatic cancers.

The term "pharmaceutically acceptable carrier" is intended to include an excipient, a diluent, an extender, a disintegrant, a stabilizer, a preservative, a buffering agent, an emulsifier, an aromatic, a coloring agent, a sweetener, a thickener, a corrective, a solubilizer or other additives, etc. When using one or more of such carriers, a pharmaceutical composition can be prepared in the form of an injection, a solution, a capsule, a suspension, an emulsion or a syrups, etc. These pharmaceutical compositions may be administered orally or parenterally. Other forms for parenteral administration include an injection comprising one or more active substances and formulated in a standard manner. In the case of an injection, it may be prepared by being dissolved or suspended in a pharmaceutically acceptable carrier such as physiological saline or commercially available distilled water for injection, etc.

In particular, when antibody fragments (particularly small antibody fragments) derived from the anti-HuCXCL1 antibody of the present invention are administered *in vivo,* a colloidal dispersion system may be used, in addition to the forgoing embodiments. A colloidal dispersion system can be expected to exert the effect of enhancing the *in vivo* stability of a compound (antibody fragment) and the effect of efficiently delivering a compound to a particular organ, tissue or cell. Such a colloidal dispersion system is not limited in any way as long as it is commonly used, and examples include polyethylene glycol, a polymer complex, a polymer aggregate, a nanocapsule, a microsphere, a bead, and lipid-based dispersion systems including an oil-in-water emulsion, a micelle, a mixed micelle and a liposome. Preferred are several types of liposomes and artificial membrane vesicles having the effect of efficiently delivering a compound to a particular organ, tissue or cell (Mannino et al., Biotechniques, 1988, 6, 682; Blume and Cevc, Biochem. et Biophys. Acta, 1990, 1029, 91; Lappalainen et al., Antiviral Res., 1994, 23, 119; Chonn and Cullis, Current Op. Biotech., 1995, 6, 698).

The dose of the pharmaceutical composition of the present invention will vary depending on the age, sex, body weight and symptom of a patient, the therapeutic effect, the mode of administration, the time required for treatment, or the type of the anti-HuCXCL1 antibody of the present invention or an antibody-drug conjugate to be contained in the pharmaceutical composition, etc. In general, the pharmaceutical composition of the present invention may be administered at a single dose ranging from 600 µg to 6000 mg per adult, without being limited thereto.

For example, when administered as an injection, the pharmaceutical composition of the present invention may be administered to a human patient at a dose of 100 µg to 100 mg per kg body weight per dosing once to several times per day average, and preferably may be administered once every 3 days, 1 week, 10 days or 2 weeks or singly (i.e., the total number of administration is once). The mode of administration may be exemplified by intravenous injection, subcutaneous injection, intracutaneous injection, intramuscular injection or intraperitoneal injection, etc., but preferred is intravenous injection. Moreover, an injection may optionally be prepared as a non-aqueous dilution (e.g., polyethylene glycol, a vegetable oil sch as olive oil, an alcohol such as ethanol), a suspension or an emulsion. Such an injection may be sterilized by filtration sterilization through a filter, incorporation with a disinfectant, etc. These injections may be prepared in reconstitutable form. Namely, they may be converted into sterile solid compositions by freeze-drying or other techniques, and then dissolved in sterile distilled water for injection or other solvents before use.

It should be noted that the present invention provides the use of the above anti-HuCXCL1 antibody of the present invention for the manufacture of a medicament (drug) for treating, preventing and/or diagnosing a tumor or for inhibiting or suppressing the migration of CXCR2-expressing cells. Moreover, the present invention provides the above anti-HuCXCL1 antibody of the present invention for treating, preventing and/or diagnosing a tumor or for inhibiting or suppressing the migration of CXCR2-expressing cells.

The present invention further provides a method for treating, preventing and/or diagnosing a tumor or a method for inhibiting or suppressing the migration of CXCR2-expressing cells, which is characterized in that the above anti-HuCXCL1 antibody of the present invention is used (i.e., administered to a subject (patient)), and also provides the use of the above anti-HuCXCL1 antibody of the present invention for treating, preventing and/or diagnosing a tumor or for inhibiting or suppressing the migration of CXCR2-expressing cells.

Moreover, the anti-HuCXCL1 antibody of the present invention is capable of inhibiting or suppressing the migration of CXCR2-expressing cells, and the mechanism of this action is deemed to involve the function of being able to inhibit or suppress the binding of various CXCLs to glucosaminoglycan (GAG) (see Example 11 described later). For this reason, the present invention provides an inhibitor or suppressor of the binding of CXCLs to GAG, which comprises the anti-HuCXCL1 antibody of the present invention, a method for inhibiting or suppressing the binding of CXCLs to GAG, which is characterized in that the anti-HuCXCL1 antibody of the present invention is used (e.g., administered to a subject), the use of the above anti-HuCXCL1 antibody of the present invention for the manufacture of a medicament (drug) for inhibiting or suppressing the binding of CXCLs to GAG, and the use of the above anti-HuCXCL1 antibody of the present invention for inhibiting or suppressing the binding of CXCLs to GAG. The above CXCLs are not limited in any way, but examples include human CXCLs such as human CXCL1, human CXCL2, human CXCL3 and human CXCL5, and mouse CXCLs such as mouse CXCL1.

### 5. Kit for diagnosing or detecting a tumor and others

The anti-HuCXCL1 antibody of the present invention can also be provided in the form of a kit for treating and/or preventing a tumor or a kit for inhibiting or suppressing the migration of CXCR2-expressing cells, and also in the form of a kit for diagnosing or detecting a tumor. As to specific examples of a tumor to be diagnosed or detected, the explanations described above can also be applied.

The diagnosis and detection may be accomplished, for example, by reacting the anti-HuCXCL1 antibody of the present invention with a sample taken *in vivo* (hereinafter referred to as a biological sample) and then detecting signals from the reacted antibody. Since HuCXCL1 has been confirmed to be expressed in various tumor cells, HuCXCL1 can also be used as a marker for various tumors. The detected antibody signals are indicative of the antigen level (i.e., HuCXCL1 level or free HuCXCL1 level) in the biological sample. For tumor diagnosis and detection with the antibody of the present invention, a biological sample taken as an analyte from a subject (e.g., a tissue piece or blood to be examined) and the antibody of the present invention are first bound to each other through antigen-antibody reaction. Then, based on the results measured for the bound antibody level, the antigen level of interest in the biological sample is measured. This measurement may be conducted according to known immunological procedures. For example, immunoprecipitation, immunoagglutination, labeled immunoassay, immunonephelometry, Western blotting, flow cytometry and other techniques may be used for this purpose. In the case of labeled immunoassay, antibody signals may be expressed as the amount of labeling which is directly detected with a labeled antibody, or alternatively, may be expressed relative to an antibody of known concentration or known titer, which is used as a standard solution. Namely, a standard solution and an analyte may be measured with a meter, and antibody signals in a biological sample can be expressed relative to the value of the standard solution. Examples of labeled immunoassay include ELISA assay, El assay, RIA assay, fluorescence immunoassay (FIA), luminescence immunoassay, etc. Among them, ELISA assay is particularly preferred in terms of simplicity and high sensitivity.

The detection results obtained as above can be used as an indicator to evaluate or diagnose the status of tumor. For example, the detection results exceeding a given reference value are defined to be tumor-positive, while the detection results not exceeding the given reference value are defined to be tumor-negative, and a positive case is determined to probably have developed any tumor, whereby the status of tumor can be evaluated. The status of tumor intended here refers to the presence or absence of tumor affection or the degree of progression thereof, as exemplified by the presence or absence of tumor onset, the degree of tumor progression, the grade of tumor malignancy, the presence or absence of tumor metastasis, and the presence or absence of tumor recurrence, etc.

These candidates for the status of tumor may be selected either alone or in combination for the above evaluation. The presence or absence of tumor can be evaluated by using a given reference value as a threshold to determine whether or not a subject is affected by the tumor on the basis of the resulting detection results. The grade of tumor malignancy is indicative of the degree of cancer progression, and can be evaluated by stage classification on the basis of the detection results or evaluated by classification into early cancer or advanced cancer. For example, the tumor can be evaluated as early cancer or advanced cancer using the detection results. For evaluation of tumor metastasis, the detection results can be used to determine whether or not a neoplasm appears at a site distant from the primary focus. Tumor recurrence can be evaluated by determining whether or not the detection results exceed again the given reference value after intermission or remission.

The kit of the present invention comprises the anti-HuCXCL1 antibody of the present invention, and may also comprise a labeling substance or an immobilized reagent in which an antibody or a labeled product thereof is immobilized. The labeled product of an antibody is intended to mean the antibody labeled with an enzyme, a radioisotope, a fluorescent compound or a chemiluminescent compound, etc. The kit of the present invention may also comprise, in addition to the above constituent elements, other reagents required to conduct the detection of the present invention, as exemplified by an enzyme substrate (e.g., a chromogenic substrate), a solvent for the enzyme substrate, a stop solution for enzymatic reaction, or a solution for analyte dilution, etc., if the labeled product is an enzyme-labeled product. Moreover, the kit may also comprise various buffers, sterilized water, various cell culture vessels, various reaction vessels (e.g., Eppendorf tubes), a blocking agent (bovine serum albumin (BSA), skimmed milk, goat serum or other serum components), a detergent, a surfactant, various plates, an antiseptic (e.g., sodium azide), and an instruction manual for experimental operations (manufacturer's instructions), etc.

### Examples

The present invention will be further described in more detail by way of the following examples, although the present invention is not limited only to these examples.

### [Example 1]

### [1] Establishment of monoclonal antibodies against human CXCL1 (HuCXCL1)

### [1-1] Preparation of HuCXCL1 protein (antigen)

Based on the amino acid sequence of a HuCXCL1 protein (Uniprot registration number: P09341; SEQ ID NO: 81), gene synthesis was conducted to obtain DNA comprising a Kozak translation initiation sequence inserted at the 5'-terminal end and a translation termination codon inserted on the 3'-terminal side (using the service of GENEWIZ).

Using an "In-Fusion HD Cloning Kit" (Takara Bio Inc., Japan), the synthesized DNA was inserted into the cloning site of "pcDNA3.4" (Thermo Fisher Scientific) to obtain a HuCXCL1 expression vector.

Then, the HuCXCL1 expression vector was used as a transfection plasmid to effect transient expression using an "Expi293 expression system" (Thermo Fisher Scientific). After transient expression, the culture supernatant was collected and affinity-purified on a "HiTrap Protein G HP column" (Cytiva). Elution fractions from gel filtration purification were confirmed for their bands by SDS-PAGE, and a fraction whose band could be confirmed was collected and used as the purified HuCXCL1 protein.

### [1-2] Immunization of mice

For antibody preparation, the HuCXCL1 protein was immunized into mice based on the following procedure. Immunization was performed on three strains of mice, i.e., MRL/MpJJmsSlc-lpr/lpr, BALB/cAJcl and ICR (Sankyo Labo Service Co., Ltd., Japan, CLEA Japan, Inc., Japan).

The antigen was mixed with an adjuvant "TiterMax Gold" (TiterMax) and intravenously administered into footpads such that the antigen dose per mouse was 100 µg. At 7 days and 10 days after administration, the mice were boosted in the same manner as above such that the antigen dose per mouse was 10 µg.

### [1-3] Preparation of hybridomas

Lymph node cells isolated from the immunized mice were used to prepare monoclonal antibodies against HuCXCL1.

After completion of the immunization, popliteal lymph nodes were collected from the mice to prepare cell suspensions, each of which was then mixed with SP2/0-Ag14 myeloma cells, followed by electrical cell fusion with an electrical cell fusion apparatus "ECFG21" (Nepa Gene Co., Ltd., Japan).

The fused cells were suspended in "ClonaCell^{™}-HY Medium D" (STEM CELL) and seeded on plastic dishes. After seeding, colonies formed after 8 to 10 days were isolated into a 96-well plastic plate containing a hybridoma medium dispensed in advance, and their culture supernatants were used for *in vitro* screening of antibodies. It should be noted that for use as a hybridoma medium, RPMI 1640 (Thermo Fisher Scientific) was supplemented with 1/50 volumes of Nutridoma-CS (Merck & Co., Inc.) and 1/50 volumes of HAT Supplement (Thermo Fisher Scientific).

### [1-4] Human ADLib system

The antibody with the clone's name "028-8-12" described later is a human antibody produced from a clone specifically binding to CXCL1 by means of the human ADLib technique (the library shown in WO2015/167011).

### [Example 2]

### [2] Antibody screening in an in vitro test system

The hybridomas obtained in Example 1 and the purified antibody obtained using the above ADLib technique were provided for antibody screening. The screening was accomplished by the following three methods.

### [2-1] Binding activity

Antibodies produced from the hybridomas were evaluated for their HuCXCL1 binding activity by ELISA assay using plates on which the antigen (HuCXCL1 protein) used for immunization had been directly immobilized.

To 96-well plates, phosphate-buffered saline (PBS) containing 3 µg/mL of HuCXCL1 was added in a volume of 50 µL per well, and the plates were allowed to stand overnight at 4°C. After removal of the antigen solution, PBS containing 2% skimmed milk and 0.05% Tween-20 (hereinafter referred to as the blocking solution) was added to each well for blocking reaction, and the plates were allowed to stand at room temperature for 1 hour. After removal of the blocking solution, the culture supernatants of the hybridomas or DT40 were each added in a volume of 50 µL per well and reacted at room temperature for 1 hour. After removal of the culture supernatants and washing with PBS containing 0.05% Tween-20, an HRP-labeled secondary antibody diluted with the blocking solution was added and reacted at room temperature for 1 hour. After washing, TMB (Dako) was added as a chromogenic substrate solution to cause color development, and after 10 minutes, 1 N sulfuric acid was added to stop the reaction, followed by measurement of absorbance at 450 nm.

As a result of screening about 10,000 clones, 500 clones with particularly strong binding ability to HuCXCL1 were selected as positive clones.

### [2-2] Ca²⁺ Flux assay

Ca²⁺ Flux assay was used to evaluate inhibitory activity against HuCXCL1 stimulatory signal transduction through a G protein-coupled receptor (GPCR). As cells expressing human CXCR2 (HuCXCR2) (i.e., the receptor of HuCXCL1), AequoScree/CXCR2 (Perkin Elmer) was purchased and used. AequoScree/CXCR2 is a recombinant cell line stably co-expressing CXCR2 and a calcium ion-sensitive photoprotein, aequorin, and receptor stimulation-associated changes in intracellular calcium ions can be measured as the luminescence intensity of aequorin.

In 0.1% BSA-containing DMEM/F-12 with HEPES, w/o phenol red (Thermo Fisher Scientific) (hereinafter referred to as the assay buffer), AequoScren/CXCR2 cells were suspended and adjusted to 2.29 × 10⁶ cells per ml. To this cell suspension, 5 µM coelenterazine h (Sigma-Aldrich Corporation) was added as a luminescent substrate for aequorin. After inversion culture at room temperature for 5 hours, the cell suspension was diluted 3-fold with the assay buffer, and inversion culture was continued for an additional 1.5 hours.

After HuCXCL1 was added to 96-well plates in an amount of 100 ng per ml, the culture supernatants of the hybridomas or DT40 or purified antibodies from their culture supernatants were each added in a volume of 60 µL per well, and the plates were allowed to stand at room temperature for 30 minutes. To these plates, the AequoScree/CXCR2 cell suspension adjusted as described above was added in a volume of 50 µL per well, immediately followed by measurement of luminescence intensity.

As a result of screening about 500 clones selected as described above, about 80 clones found to particularly strongly inhibit HuCXCL1-induced receptor stimulation-associated changes in intracellular calcium ions, i.e., found to have strong neutralizing activity against HuCXCL1 were selected as positive clones.

Further, the above selected 80 clones were calculated for their 50% effective concentration (EC50) for HuCXCL1 neutralization. EC50 was evaluated in the following manner.

AequoScrenn/CXCR2 cells were adjusted in the same manner as described above. After HuCXCL1 was added to 96-well plates in an amount of 100 ng per ml, purified antibodies were each added in an amount of 100 µg, 50 µg, 15 µg, 5 µg, 1.5 µg, 0.5 µg, 0.15 µg or 0.05 µg per ml, and the plates were allowed to stand at room temperature for 30 minutes. To these plates, the AequoScree/CXCR2 cell suspension was added in a volume of 50 µL per well, immediately followed by measurement of luminescence intensity. EC50 was calculated from the resulting dose-response curve.

### [2-3] Migration inhibitory activity (Migration assay)

The 80 clones with neutralizing activity against HuCXCL1 were evaluated for their inhibitory activity against the migration of HuCXCR2-expressing cells toward HuCXCL1.

Cell chemotaxis was measured by Boyden chamber assay, and QCM Chemoraxis Cell Migration Assay (Sigma-Aldrich Corporation) was purchased and used as an assay kit. AequoScrenn/CXCR2 cells were used as HuCXCR2-expressing cells. An isotype control antibody and a goat anti-HuCXCL1 polyclonal antibody (hereinafter referred to as AF275) were purchased from R&D Systems and used.

The HuCXCR2-expressing cells were cultured overnight at 37°C in a serum-free culture medium, and then suspended in the assay buffer and adjusted to 2 × 10⁶ cells per ml. The upper chamber of a Boyden chamber was filled with the HuCXCR2-expressing cell suspension, while the lower chamber was filled with the assay buffer alone or a solution containing HuCXCL1 in an amount of 200 ng per ml with or without a purified antibody, followed by incubation overnight at 37°C.

After incubation, the upper chamber was washed with PBS, placed into a new lower chamber containing a Cell Detachment Solution (Sigma-Aldrich Corporation), and then allowed to stand at 37°C for 30 minutes to detach the migrated cells adhered to the bottom of the membrane in the upper chamber.

The migrated cell suspension was supplemented with 4 × Lysis Buffer (Sigma-Aldrich Corporation) in a volume of 75 µL per well and allowed to stand at room temperature for 20 minutes, followed by measurement of fluorescence at an excitation wavelength of 480 nm and a fluorescence wavelength of 520 nm. The above about 80 clones were screened and evaluated for their inhibitory activity against the migration of

CXCR2 cells.

Based on the evaluation of binding activity to HuCXCL1, EC50 and inhibitory activity against the migration of CXCR2-expressing cells toward HuCXCL1, 15 types of antibodies which were functional *in vitro* were selected. These results are shown in Table A below.

**[Table A]**

| No. | Clone name | Binding activity to HuCXCL1 | HuCXCL1 neutralizing activity EC50 (nM) | CXCR2-expressing cell migration inhibitory activity | Binding activity to CyCXCL1 | CyCXCL1 neutralizing activity |
|---|---|---|---|---|---|---|
| | | ELISA | Ca²⁺ Flux assay | Chemotaxis assay | ELISA | Ca²⁺ Flux assay |
| 1 | 5A-5E11 | ○ | 3.96 | ○ | ○ | ○ |
| 2 | 4A-2F7 | ○ | 4.8 | ○ | ○ | ○ |
| 3 | 028-8-12 | ○ | 5.73 | ○ | ○ | ○ |
| 7 | 1A-3C6 | ○ | 5.99 | ○ | × | × |
| 4 | 1A-7H10 | ○ | 6.24 | ○ | ○ | ○ |
| 8 | 4A-5B11 | ○ | 7.69 | ○ | × | × |
| 9 | 1A-2H3 | ○ | 7.83 | ○ | × | × |
| 10 | 3A-1 F9 | ○ | 7.85 | ○ | × | × |
| 11 | 1A-5E3 | ○ | 8.48 | ○ | × | × |
| 12 | 4A-7G7 | ○ | 8.51 | ○ | × | × |
| 5 | 1B-2E9 | ○ | 8.87 | ○ | ○ | × |
| 6 | 4A-6B8 | ○ | 8.9 | ○ | × | × |
| 13 | 4A-2E4 | ○ | 10.1 | ○ | × | × |
| 14 | 4A-17F3 | ○ | 28.9 | ○ | × | × |
| 15 | 6A-6H2 | ○ | 43.4 | ○ | × | × |

### [2-4] Cross-reactivity to other animal species

### [2-4-1] Binding activity to CyCXCL1

Binding activity to cynomolgus monkey CXCL1 (CyCXCL1) was evaluated.

The same procedure as shown in [1-1] above was repeated to prepare a CyCXCL1 protein (antigen), except that the amino acid sequence of a HuCXCL1 protein was replaced with the amino acid sequence of a CyCXCL1 protein (Uniprot registration number: Q0EAC3; SEQ ID NO: 82).

The evaluation of binding activity to CyCXCL1 was accomplished by ELISA assay. Absorbance was calculated in the same manner as shown in [2-1] above to evaluate antigen-binding activity, except that the protein immobilized on the plates was changed from a HuCXCL1 protein to a CyCXCL1 protein. An antibody whose absorbance to CyCXCL1 was 80% or more of its absorbance to HuCXCL1 was determined to have cross-reactivity and indicated with "o" while an antibody whose absorbance to CyCXCL1 was less than 80% of its absorbance to HuCXCL1 was indicated with "×" in Table A above.

### [2-4-2] Neutralizing activity against CyCXCL1

For evaluation of neutralizing activity against CyCXCL1, CyCXCL1-induced changes in intracellular calcium ions in AequoScrenn/CXCR2 cells were measured as the luminescence intensity of aequorin.

AequoScrenn/CXCR2 cells were adjusted in the same manner as shown in [2-2] above. After CyCXCL1 was added to 96-well plates in an amount of 100 ng per ml, purified antibodies were each added in an amount of 10 µg per ml, and the plates were allowed to stand at room temperature for 30 minutes. To these plates, the AequoScree/CXCR2 cell suspension was added in a volume of 50 µL per well, immediately followed by measurement of luminescence intensity.

An antibody found to completely suppress CyCXCL1-induced luminescence from aequorin was determined to have cross-reactivity and indicated with "o" while an antibody found to not completely suppress luminescence from aequorin was indicated with "×" in Table A above.

### [2-5] Conclusion

From among the above selected 15 types of antibodies, 4 types of clone antibodies (5A-5E11, 4A-2F7, 1A-7H10 and 028-8-12) were selected as candidate antibodies in consideration of their cross-reactivity with CyCXCL1.

It should be noted that 5A-5E11 and 4A-2F7 were found to also bind to human CXCL2, 3 and 5, while 1A-7H10 and 028-8-12 were found to also bind to human CXCL2 and 3.

### [Example 3]

### [3] Evaluation of antibodies with in vivo activity (1)

### [3-1] Preparation of a model with OCUM-12 orthotopic transplantation

A model with OCUM-12 orthotopic transplantation was prepared to evaluate the above 4 types of candidate antibodies for their *in vivo* CXCR2 migration inhibitory activity and antitumor activity in a xenograft model with OCUM-12 orthotopic transplantation.

For use as mice, female nude mice (BALB/cAJcl-nu/nu) at 5 to 6 weeks of age were purchased from CLEA Japan, Inc., Japan. For use as human cancer cells, a gastric cancer cell line, OCUM-12, was provided by University Public Corporation Osaka, Japan.

OCUM-12 cells were cultured in D-MED medium (FUJIFILM Wako Pure Chemical Corporation, Japan) supplemented with 10% fetal bovine serum (FBS; Sigma-Aldrich Corporation) and 1/100 volumes of a penicillin-streptomycin mixture (Nacalai Tesque, Inc., Japan). The cells to be transplanted were detached with 0.25% trypsin/0.02% EDTA (Thermo Fisher Scientific) and washed with PBS, and 4 × 10⁵ cells per mouse were transplanted into the gastric wall of each mouse.

### [3-2] Antitumor activity test

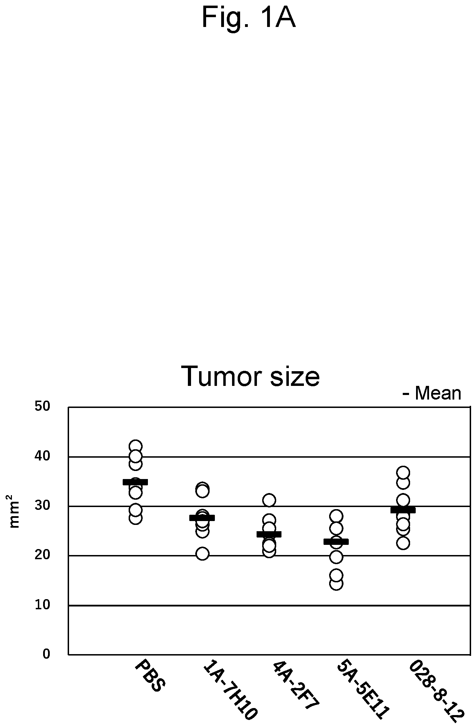

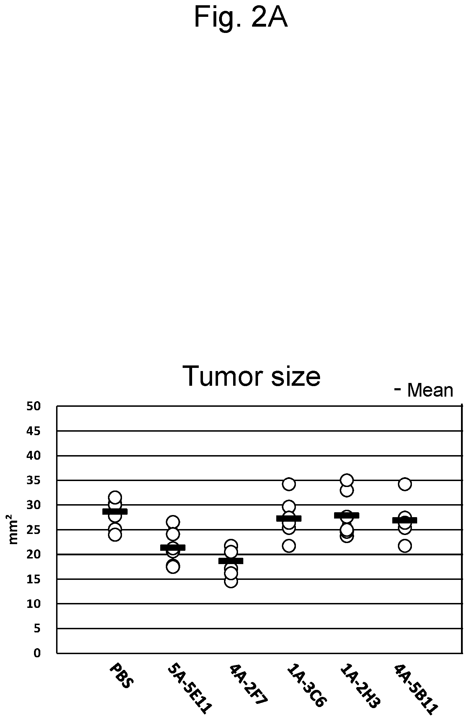

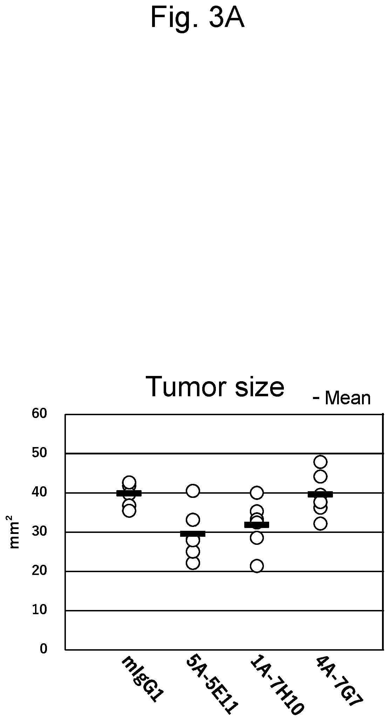

On the day following the above transplantation, the mice were randomized into groups (8 or 6 mice per group) and intraperitoneally administered with PBS as a control and with 4 types of candidate antibodies (1A-7H10, 4A-2F7, 5A-5E11 and 028-8-12) and 3 types of non-candidate antibodies (1A-3C6, 1A-2H3 and 4A-5B11) at a dose of 20 mg/kg. In addition, mouse IgG1 as a control was intraperitoneally administered at a dose of 20 mg/kg, and 3 types of antibodies (5A-5E11, 1A-4H10 and 4A-7G7) among the candidate antibodies were intraperitoneally administered at a dose of 20 mg/kg. The administration of these antibodies was then continued with a frequency of twice a week until the completion of the test. After 3 weeks had passed from the transplantation, the mice were sacrificed, and the short- and long-axis diameters of tumors formed in their gastric walls were each measured with a caliper. The tumor size was calculated according to the following: 1/2 short-axis diameter (mm) × 1/2 long-axis diameter (mm) × 3.14. The results obtained are shown in Figures 1A, 2A and 3A.

As indicated in these results, the groups receiving 1A-7H10, 4A-2F7, 5A-5E11 and 028-8-12 were found to significantly suppress tumor growth when compared to the group receiving PBS.

### [3-3] Migration inhibitory activity test on CXCR2-expressing cells

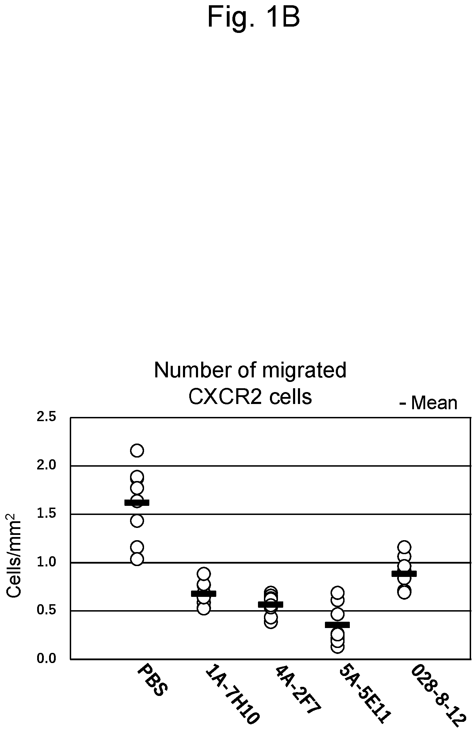

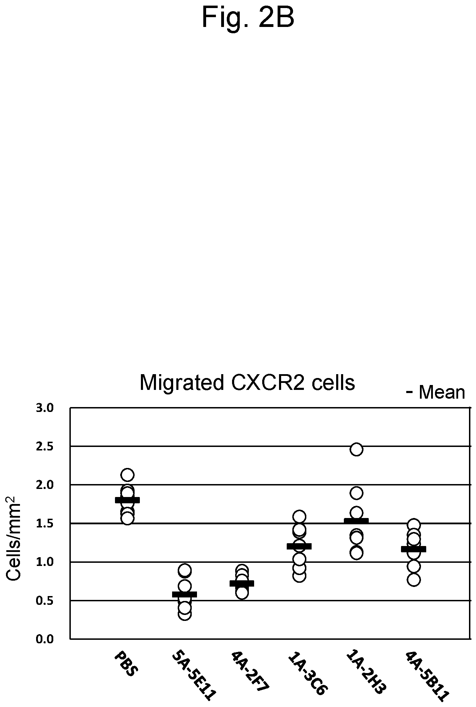

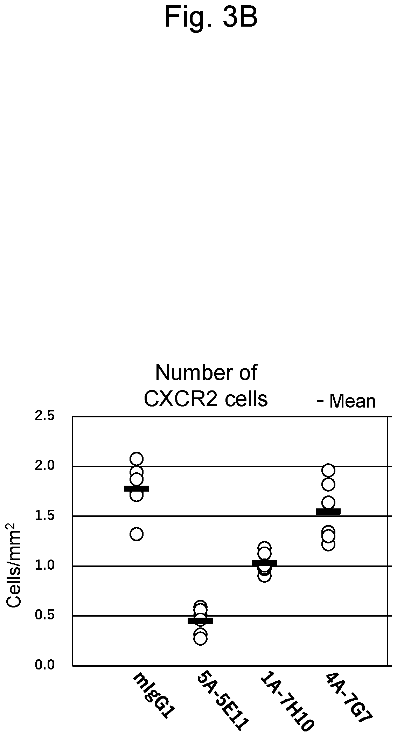

The stomachs after measurement of the tumor size in [3-1] above were excised and fixed overnight at room temperature with a 10% neutral buffered formalin solution (Wako Pure Chemical Industries, Ltd., Japan), followed by replacement with PBS. Then, paraffin embedding and section preparation were outsourced to Applied Medical Research Laboratory, Japan. The prepared gastric tumor sections were immunohistologically stained with an anti-CXCR2 antibody to evaluate *in vivo* CXCR2 cell migration inhibitory activity. The sections were deparaffinized and rehydrated, and then treated to inactivate endogenous peroxidase. After antigen activation treatment, the sections were blocked with PBS containing 2% skimmed milk and 0.1% Tween-20, and reacted overnight at 4°C with an anti-CXCR2 antibody (Biorbyt) as a primary antibody, which had been diluted to 0.3 µg/mL with the blocking solution. After washing with PBS containing 0.1% Tween-20, the sections were reacted at room temperature for 1 hour with an HRP-labeled goat anti-rabbit IgG antibody (Abcam) as a secondary antibody. After washing with a washing solution, a simple stain DAB solution (5052, Nichirei Corporation, Japan) was used to cause color development. After images of the stained tissues were captured with an Explore (Olympus Corporation, Japan), imaging software CellSens (Olympus Corporation, Japan) was used to calculate CXCR2-positive cells per unit area. The results obtained are shown in Figures 1B, 2B and 3B.

As indicated in these results, the groups receiving 1A-7H10, 4A-2F7, 5A-5E11, 1A-3C6, 4A-5B11 and 028-8-12 were found to significantly suppress the migration of CXCR2-expressing cells when compared to the group receiving PBS or mouse IgG1 (mIgG1) antibody.

### [3-4] Conclusion

In the *in vivo* tests, 4 types of candidate antibodies (1A-7H10, 4A-2F7, 5A-5E11 and 028-8-12) were found to have sufficient migration inhibitory activity and have an antitumor effect. On the other hand, among antibodies inhibiting the migration of CXCR2-expressing cells *in vitro,* some antibodies (1A-2H3 and 4A-7G7) were found to have no migration inhibitory activity *in vivo,* while some other antibodies (1A-3C6 and 4A-5B11) were found to have no antitumor effect *in vivo* even though they showed *in vivo* inhibitory activity against the migration of CXCR2-expressing cells (Figures 1 to 3). These findings indicate that migration inhibitory activity above a certain level would be required to ensure that an inhibitory effect against the migration of CXCR2-expressing cells leads to an antitumor effect.

### [Example 4]

### [4] Evaluation of antibodies with in vivo activity (2)

### [4-1] Efficacy evaluation of candidate antibodies in a model with OCUM-2MLN orthotopic transplantation

Among the candidate antibodies, 3 types of antibodies (4A-2F7, SA-5E11 and 028-8-12) were evaluated for their *in vivo* antitumor activity in a xenograft model with OCUM-2MLN orthotopic transplantation. Procedures for the evaluation are shown below.

For use as mice, female nude mice (BALB/cAJcl-nu/nu) at 5 to 6 weeks of age were purchased from Charles River Japan, Inc. As human cancer cells, a gastric cancer cell line, OCUM-2MLN, was used.

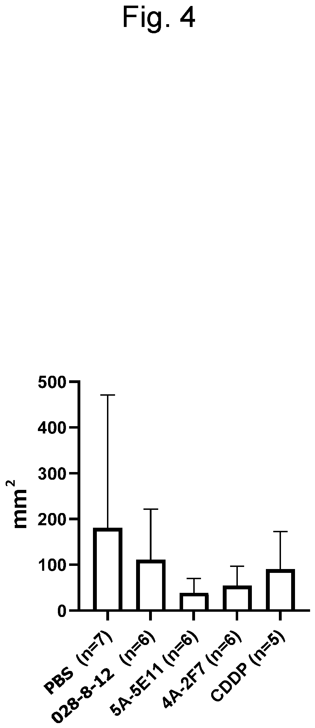

OCUM-2MLN cells were cultured in D-MEM medium (FUJIFILM Wako Pure Chemical Corporation, Japan) supplemented with 10% fetal bovine serum (FBS; NICHIREI BIOSCIENCE), 1/100 volumes of a penicillin-streptomycin mixture (FUJIFILM Wako Pure Chemical Corporation, Japan) and 1/100 volumes of a sodium pyruvate solution (SIGMA-ALDRICH). The cells to be transplanted were detached with a 0.05 w/v% trypsin/0.53 mmol/L EDTA 4Na solution (FUJIFILM Wako Pure Chemical Corporation, Japan) and washed with PBS, and 1 × 10⁶ cells per mouse were transplanted into the gastric wall of each mouse. On the day following the transplantation, the mice were randomized into groups (5 to 8 mice per group) and intraperitoneally administered with PBS (FUJIFILM Wako Pure Chemical Corporation, Japan) as a control and with test drugs (candidate antibodies) at a dose of 20 mg/kg. The administration of the test drugs was then continued with a frequency of twice a week until the completion of the test. After 3 weeks had passed from the transplantation, the mice were sacrificed, and the short- and long-axis diameters of tumors formed in their gastric walls were each measured with a caliper. The tumor size was calculated according to the following: short-axis diameter (mm) × long-axis diameter (mm). The results obtained are shown in Figure 4. As can be seen from Figure 4, particularly 4A-2F7 and 5A-5E11 were found to significantly suppress tumor growth when compared to the control.

[Reference Example] Migration inhibition of CXCR2-expressing cells and antitumor activity by a small molecular compound (Navarixin) serving as a CXCR2 inhibitor

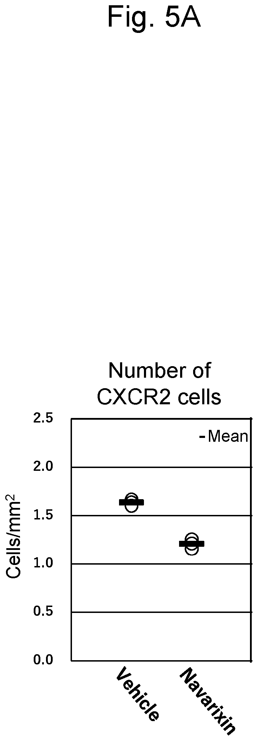

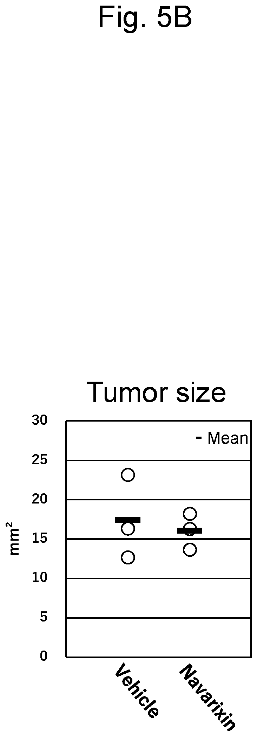

Navarixin is among small molecular compounds that act on CXCR2 and inhibit CXCL1-CXCR2 signaling. Navarixin has been known to inhibit the migration of CXCR2-expressing cells *in vitro.* Then, using a model with OCUM-12 orthotopic transplantation, the migration inhibition of CXCR2-expressing cells and antitumor activity were evaluated *in vivo* for Navarixin. As a vehicle, ultrapure water containing 20% 2-hydroxypropyl-β-cyclodextrin (FUIIFILM Wako Pure Chemical Corporation, Japan) was used. Navarixin (MedChemExpress) was dissolved in ultrapure water containing 20% 2-hydroxypropyl-β-cyclodextrin, and orally administered at a dose of 100 mg/kg/day in a schedule of once a day for 5 consecutive days, followed by a 2-day withdrawal. Navarixin administration was able to significantly inhibit the migration of CXCR2-expressing cells in a xenograft model with OCUM-12 orthotopic transplantation (Figure 5A), but no antitumor activity was observed (Figure 5B).

This indicates that among substances which inhibit CXCL1-CXCR2 signaling and inhibit the migration of CXCR2-expressing cells, some have no antitumor effect.

### [Example 5]

### [5] Sequence analysis of antibodies

Based on the following procedures, 4 types of candidate antibodies (1A-7H10, 4A-2F7, 5A-5E11 and 028-8-12) were analyzed for the sequences of their immunoglobulin gene variable regions.

Hybridoma cells were expanded, and cDNA synthesis was conducted with a TaqMan Gene Expression Cells-to-CT Kit (Thermo Fisher Scientific).

Using the synthesized cDNA as a template, antibody variable region sequences were amplified by PCR techniques. It should be noted that the heavy and light chains were each amplified with primers recognizing upstream of the variable region and downstream of the constant region.

The resulting DNA fragments were each cloned into a vector attached to a Zeroblunt TOPO TA Cloning and Sequencing kit (Thermo Fisher Scientific), followed by DNA sequence analysis.

For the resulting antibody sequences, CDR regions were determined according to the method of Kabat et al. (Sequences of Proteins of Immunological Interest, Fifth edition, NIH Publication No. 91-3242, US, Department of Health and Human Services, 1991).

The analyzed antibody sequences and their CDR region sequences are summarized in Table 1 below.

It should be noted that abbreviations in the table have the following meanings.
VH: heavy chain variable region
CDRH: heavy chain CDR
VL: light chain variable region
CDRL: light chain CDR

**[Table 1]**

| | VH | CDRH1 | CDRH2 | CDRH3 | VL | CDRL1 | CDRL2 | CDRL3 |
|---|---|---|---|---|---|---|---|---|
| m1A-7H10 | SEQ ID NO: 1 | SEQ ID NO: 2 | SEQ ID NO: 3 | SEQ ID NO: 4 | SEQ ID NO: 5 | SEQ ID NO: 6 | SEQ ID NO: 7 | SEQ ID NO: 8 |
| m4A-2F7 | SEQ ID NO: 9 | SEQ ID NO: 10 | SEQ ID NO: 11 | SEQ ID NO: 12 | SEQ ID NO: 13 | SEQ ID NO: 14 | SEQ ID NO: 15 | SEQ ID NO: 16 |
| m5A-5E11 | SEQ ID NO: 17 | SEQ ID NO: 18 | SEQ ID NO: 19 | SEQ ID NO: 20 | SEQ ID NO: 21 | SEQ ID NO: 22 | SEQ ID NO: 23 | SEQ ID NO: 24 |
| ADLib#028-08-12 | SEQ ID NO: 25 | SEQ ID NO: 26 | SEQ ID NO: 27 | SEQ ID NO: 28 | SEQ ID NO: 29 | SEQ ID NO: 30 | SEQ ID NO: 31 | SEQ ID NO: 32 |

### [Example 6]

### [6] Preparation of mouse-human chimeric antibodies and amino acid modifications

Based on the antibody sequences obtained in [5] above, a chimeric antibody composed of mouse heavy and light chain variable regions and human IgG1 heavy chain and κ chain constant regions (hereinafter referred to as a mouse-human chimeric antibody) was prepared for each of 3 types of antibodies (1A-7H10, 4A-2F7 and 5A-5E11) among the candidate antibodies.

For the individual antibody sequences, the same amino acid sequences as analyzed in [5] above were prepared, and amino acid modifications were also attempted to their CDR regions.

It should be noted that since the antibody 028-8-12 is a human antibody, no chimeric antibody was prepared therefor, and only amino acid modifications were attempted to its CDR regions and VL region.

Antibody sequences with modified CDR sequences, and their original sequences are shown in Table 2 below.

**[Table 2]**

| | | | SEQ ID NO | | | |
|---|---|---|---|---|---|---|
| | | | Variable region | CDR1 | CDR2 | CDR3 |
| mh1A-7H10 | VH | 1A-7H10_VH | SEQ ID NO: 1 | SEQ ID NO: 2 | SEQ ID NO: 3 | SEQ ID NO: 4 |
| | | 1A-7H10_VH1 | SEQ ID NO: 33 | SEQ ID NO: 2 | SEQ ID NO: 34 | SEQ ID NO: 4 |
| | VL | 1A-7H10_VL | SEQ ID NO: 5 | SEQ ID NO: 6 | SEQ ID NO: 7 | SEQ ID NO: 8 |
| mh4A-2F7 | VH | 4A-2F7_VH | SEQ ID NO: 9 | SEQ ID NO: 10 | SEQ ID NO: 11 | SEQ ID NO: 12 |
| | VL | 4A-2F7_VL | SEQ ID NO: 13 | SEQ ID NO: 14 | SEQ ID NO: 15 | SEQ ID NO: 16 |
| | | 4A-2F7_VL1 | SEQ ID NO: 35 | SEQ ID NO: 14 | SEQ ID NO: 36 | SEQ ID NO: 16 |
| | | 4A-2F7_VL2 | SEQ ID NO: 37 | SEQ ID NO: 14 | SEQ ID NO: 38 | SEQ ID NO: 16 |
| | | 4A-2F7_VL3 | SEQ ID NO: 39 | SEQ ID NO: 14 | SEQ ID NO: 40 | SEQ ID NO: 16 |
| | | 5A-5E11_VH | SEQ ID NO: 17 | SEQ ID NO: 18 | SEQ ID NO: 19 | SEQ ID NO: 20 |
| | | 5A-5E11_VH1 | SEQ ID NO: 41 | SEQ ID NO: 18 | SEQ ID NO: 42 | SEQ ID NO: 20 |
| | | 5A-5E11_VH2 | SEQ ID NO: 43 | SEQ ID NO: 18 | SEQ ID NO: 44 | SEQ ID NO: 20 |
| | | 5A-5E11_VH3 | SEQ ID NO: 45 | SEQ ID NO: 18 | SEQ ID NO: 46 | SEQ ID NO: 20 |
| | | 5A-5E11_VH4 | SEQ ID NO: 47 | SEQ ID NO: 18 | SEQ ID NO: 48 | SEQ ID NO: 20 |
| mh5A-5E11 | VH | 5A-5E11_VH5 | SEQ ID NO: 49 | SEQ ID NO: 18 | SEQ ID NO: 50 | SEQ ID NO: 20 |
| | | 5A-5E11_VH6 | SEQ ID NO: 51 | SEQ ID NO: 18 | SEQ ID NO: 52 | SEQ ID NO: 20 |
| | | 5A-5E11_VH7 | SEQ ID NO: 53 | SEQ ID NO: 18 | SEQ ID NO: 54 | SEQ ID NO: 20 |
| | | 5A-5E11_VH8 | SEQ ID NO: 55 | SEQ ID NO: 18 | SEQ ID NO: 56 | SEQ ID NO: 20 |
| | | 5A-5E11_VH9 | SEQ ID NO: 57 | SEQ ID NO: 18 | SEQ ID NO: 58 | SEQ ID NO: 20 |
| | | 5A-5E11_VH10 | SEQ ID NO: 59 | SEQ ID NO: 18 | SEQ ID NO: 60 | SEQ ID NO: 20 |
| | | 5A-5E11_VH11 | SEQ ID NO: 61 | SEQ ID NO: 18 | SEQ ID NO: 62 | SEQ ID NO: 20 |
| | VL | 5A-5E11_VL | SEQ ID NO: 21 | SEQ ID NO: 22 | SEQ ID NO: 23 | SEQ ID NO: 24 |
| ADLib#028-08-12 | VH | ADLib#028-08-12_VH | SEQ ID NO: 25 | SEQ ID NO: 26 | SEQ ID NO: 27 | SEQ ID NO: 28 |
| | | ADLib#028-8-12_VH1 | SEQ ID NO: 63 | SEQ ID NO: 26 | SEQ ID NO: 64 | SEQ ID NO: 28 |
| | | ADLib#028-8-12_VH2 | SEQ ID NO: 65 | SEQ ID NO: 26 | SEQ ID NO: 66 | SEQ ID NO: 28 |
| | VL | ADLib#028-8-12_VL | SEQ ID NO: 29 | SEQ ID NO: 30 | SEQ ID NO: 31 | SEQ ID NO: 32 |
| | | ADLib#028-8-12_VL1 | SEQ ID NO: 67 | SEQ ID NO: 30 | SEQ ID NO: 31 | SEQ ID NO: 32 |

The combinations of the heavy and light chains of each antibody based on the sequences shown in Table 2 above are shown in Tables 3 to 6 below.

**[Table 3]**

| **mh1A-7H10** | VH SEQ ID NO | VL SEQ ID NO |
|---|---|---|
| VH/VL | SEQ ID NO: 1 | SEQ ID NO: 5 |
| VH1/VL | SEQ ID NO: 33 | SEQ ID NO: 5 |

**[Table 4]**

| **mh4A-2F7** | VH SEQ ID NO | VL SEQ ID NO |
|---|---|---|
| VH/VL | SEQ ID NO: 9 | SEQ ID NO: 13 |
| VH/VL1 | SEQ ID NO: 9 | SEQ ID NO: 35 |
| VH/VL2 | SEQ ID NO: 9 | SEQ ID NO: 37 |
| VH/VL3 | SEQ ID NO: 9 | SEQ ID NO: 39 |

**[Table 5]**

| **mh5A-5E11** | VH SEQ ID NO | VL SEQ ID NO |
|---|---|---|
| VH/VL | SEQ ID NO: 17 | SEQ ID NO: 21 |
| VH1/VL | SEQ ID NO: 41 | SEQ ID NO: 21 |
| VH2/VL | SEQ ID NO: 43 | SEQ ID NO: 21 |
| VH3/VL | SEQ ID NO: 45 | SEQ ID NO: 21 |
| VH4/VL | SEQ ID NO: 47 | SEQ ID NO: 21 |
| VH5/VL | SEQ ID NO: 49 | SEQ ID NO: 21 |
| VH6/VL | SEQ ID NO: 51 | SEQ ID NO: 21 |
| VH7/VL | SEQ ID NO: 53 | SEQ ID NO: 21 |
| VH8/VL | SEQ ID NO: 55 | SEQ ID NO: 21 |
| VH9/VL | SEQ ID NO: 57 | SEQ ID NO: 21 |
| VH10/VL | SEQ ID NO: 59 | SEQ ID NO: 21 |
| VH11/VL | SEQ ID NO: 61 | SEQ ID NO: 21 |

**[Table 6]**

| **028-8-12** | VH SEQ ID NO | VL SEQ ID NO |
|---|---|---|
| VH/VL | SEQ ID NO: 25 | SEQ ID NO: 29 |
| VH1/VL | SEQ ID NO: 63 | SEQ ID NO: 29 |
| VH2/VL | SEQ ID NO: 65 | SEQ ID NO: 29 |
| VH/VL1 | SEQ ID NO: 25 | SEQ ID NO: 67 |
| VH1/VL1 | SEQ ID NO: 63 | SEQ ID NO: 67 |
| VH2/VL1 | SEQ ID NO: 65 | SEQ ID NO: 67 |

Amino acid-modified antibodies obtained upon combination of heavy and light chains were each evaluated for their antigen-binding activity and neutralizing activity by antigen-immobilized ELISA and Ca²⁺ Flux Assay, and all the amino acid-modified antibodies were confirmed to have antigen-binding activity and neutralizing activity comparable to those of their unmodified chimeric parental antibody (VH/VL). From among these amino acid-modified antibodies, those shown in Table 7 below were selected in terms of the properties of their modified amino acids.

**[Table 7]**

| | Heavy chain | SEQ ID NO | | | | Light chain | SEQ ID NO | | | |
|---|---|---|---|---|---|---|---|---|---|---|
| | | Variable region | CDR1 | CDR2 | CDR3 | | Variable region | CDR1 | CDR2 | CDR3 |
| 1A-7H10_VH1/VL | VH1 | SEQ ID NO: 33 | SEQ ID NO: 2 | SEQ ID NO: 34 | SEQ ID NO: 4 | VL | SEQ ID NO: 5 | SEQ ID NO: 6 | SEQ ID NO: 7 | SEQ ID NO: 8 |
| 4A-2F7_VH/VL1 | VH | SEQ ID NO: 9 | SEQ ID NO: 10 | SEQ ID NO: 11 | SEQ ID NO: 12 | VL1 | SEQ ID NO: 35 | SEQ ID NO: 14 | SEQ ID NO: 36 | SEQ ID NO: 16 |
| 5A-5E11_VH7/VL | VH7 | SEQ ID NO: 53 | SEQ ID NO: 18 | SEQ ID NO: 54 | SEQ ID NO: 20 | VL | SEQ ID NO: 21 | SEQ ID NO: 22 | SEQ ID NO: 23 | SEQ ID NO: 24 |
| ADLib#028-8-12_VH1/VL1 | VH1 | SEQ ID NO: 63 | SEQ ID NO: 26 | SEQ ID NO: 64 | SEQ ID NO: 28 | VL1 | SEQ ID NO: 67 | SEQ ID NO: 30 | SEQ ID NO: 31 | SEQ ID NO: 32 |

### [Example 7]

### [7] Preparation of humanized antibodies

Then, the above modified antibody (human chimeric antibody) sequences selected for 1A-7H10, 4A-2F7 and 5A-5E11 were humanized.

The sequence of each antibody variable region was humanized by CDR grafting. The design of humanized sequences was accomplished based on procedures described in a known article (Tsurushita et al., Design of humanized antibodies: From anti-Tac to Zenapax. Methods, 36:69-83, 2005).

First, a three-dimensional molecular model was prepared for mouse antibody in a standard manner. Then, this molecular model was used to estimate residues considered to be important for CDR structure formation in the amino acid sequences of framework regions, and residues considered to be essential for reaction with the antigen. In parallel, the cDNA sequence databases of human antibody heavy and light chain variable regions were searched for sequences highly homologous to the heavy and light chain variable regions of each anti-HuCXCL1 antibody. Then, the sequences of framework portions in the searched human antibody sequences were linked to the CDR sequences of each anti-HuCXCL1 antibody, and the thus designed sequences were further grafted with a sequence of residues considered to be essential for CDR structure formation or for reaction with the antigen to thereby design humanized antibody sequences. The designed sequences are as shown in Table 8 below. CDR sequences in the humanized antibody sequences are the same as those in their original mouse-human chimeric antibody, and are the same sequences as shown in the SEQ ID NOs in the tables shown above. In Table 8, the underlined sections represent CDRs.

**[Table 8]**

| Humanized antibody | | | SEQ ID NO | | | |
|---|---|---|---|---|---|---|
| Antibody name | Variable region | Amino acid sequence | **Variable region** | CDR1 | CDR2 | CDR3 |
| Hu1A-7H10 | VH1 | QVTLKESGP VLVKPTETLT LTCTVSGFSS TTYGVHWVRQ PPGKALEWLG VIWAGGSTNY NAALMSRLTI SKDTSKSQVV LTMTNMDPVDTAT YYCATQYHYP FAYWGQGTLV TVSS | **SEQ ID NO: 68** | SEQ ID NO: 2 | SEQ ID NO: 34 | SEQ ID NO: 4 |
| | VH2 | QVTLKESGP VLVKPTETLT LTCTVSGFSS TTYGVHWVRQ PPGKALEWLG VIWAGGSTNY NAALMSRLTI SKDNSKSQVV LTMTNMDPVDTAT YYCATQYHYP FAYWGQGTLV TVSS | **SEQ ID NO: 69** | SEQ ID NO: 2 | SEQ ID NO: 34 | SEQ ID NO: 4 |
| | VL1 | DIQMTQSPS SLSASVGDRV TITCKASENV GIYVSWYQQK PGKAPKLMIY GASNRNTGVP SRFSGSGSGT DFTLTISSLQ PEDFATYYCG QSYSYPLTFG GGTKVEIK | **SEQ ID NO: 70** | SEQ ID NO: 6 | SEQ ID NO: 7 | SEQ ID NO: 8 |
| | VL2 | DIQMTQSPS SLSASVGDRV TITCKASENV GIYVSWYQQK PGKAPKLMIY GASNRNTGVP SRFSGSGSAT DFTLTISSLQ PEDFATYYCG QSYSYPLTFG GGTKVEIK | **SEQ ID NO: 71** | SEQ ID NO: 6 | SEQ ID NO: 7 | SEQ ID NO: 8 |
| Hu4A-2F7 | VH1 | QVTLKESGP ALVKPTQTLT LTCTFSGFSL STSGMGVDWIRQ PPGKALEWLA HIWWDDDKYY NPSLKSQLTI SKDTSKNQVV LTMTNMDPVDTAT YYCARSYGYD MDYYAMDYWGQGTTV TVSS | **SEQ ID NO: 72** | SEQ ID NO: 10 | SEQ ID NO: 11 | SEQ ID NO: 12 |
| | VH2 | QVTLKESGP ALVKPTQTLT LTCTFSGFSL STSGMGVDWIRQ PPGKGLEWLA HIWWDDDKYY NPSLKSQLTI SKDTSKNQVV LTMTNMDPVDTAT YYCARSYGYD MDYYAMDYWGQGTTV TVSS | **SEQ ID NO: 73** | SEQ ID NO: 10 | SEQ ID NO: 11 | SEQ ID NO: 12 |
| | VL1 | DIQMTQSPS SLSASVGDRV TITCRASQEI SGYLSWLQQK PGKAPKRLIY AASTLSSGVP SRFSGSGSGT DFTLTISSLQ PEDFATYYCL QYASYPPWTFG GGTKVEIK | **SEQ ID NO: 74** | SEQ ID NO: 14 | SEQ ID NO: 36 | SEQ ID NO: 16 |
| | VL2 | DIQMTQSPS SLSASVGDRV TITCRASQEI SGYLSWLQQK PGKAPKRLIY AASTLSSGVP SRFSGSGSGT DYTLTISSLQ PEDFATYYCL QYASYPPWTFG GGTKVEIK | **SEQ ID NO: 75** | SEQ ID NO: 14 | SEQ ID NO: 36 | SEQ ID NO: 16 |
| Hu5A-5E11 | VH1 | QVQLVQSGA EVKKPGSSVK VSCKASGYAF SDYWMNWVRQ APGQGLEWIG QIYPGSGDTNY NAKFKGKGTI TADKSTSTAY MELSSLRSEDTAV YYCARERDYD VAMDYWGQGTTV TVSS | **SEQ ID NO: 76** | SEQ ID NO: 18 | SEQ ID NO: 54 | SEQ ID NO: 20 |
| | VH2 | QVQLVQSGA EVKKPGSSVK VSCKASGYAF SDYWMNWVRQ APGQGLEWIG QIYPGSGDTNY NAKFKGKGTL TADKSTSTAY MELSSLRSEDTAV YYCARERDYD VAMDYWGQGTTV TVSS | **SEQ ID NO: 77** | SEQ ID NO: 18 | SEQ ID NO: 54 | SEQ ID NO: 20 |
| | VL1 | DIQMTQSPS SLSASVGDRV TITCKASQDI NKYIAWFQQK PGKAPKLVIH YTSILQPGVP SRFSGSGSGT DYTFTISSLQ PEDIATYYCL QYDNLPWTFG GGTKVEIK | **SEQ ID NO: 78** | SEQ ID NO: 22 | SEQ ID NO: 23 | SEQ ID NO: 24 |
| | VL2 | DIQMTQSPS SLSASVGDRV TITCKASQDI NKYIAWFQQK PGKAPKLVIH YTSILQPGVP SRFSGVGSGT DYTFTISSLQ PEDIATYYCL QYDNLPWTFG GGTKVEIK | **SEQ ID NO: 79** | SEQ ID NO: 22 | SEQ ID NO: 23 | SEQ ID NO: 24 |

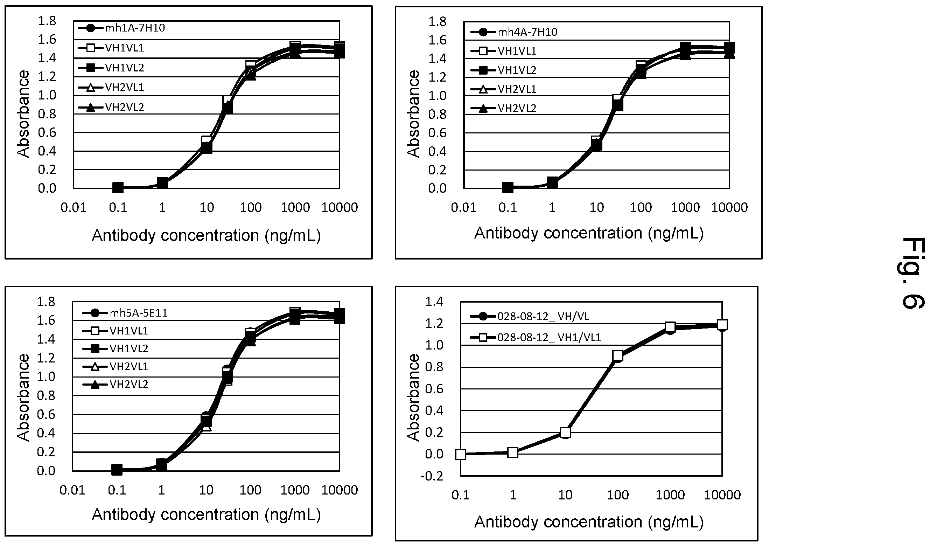

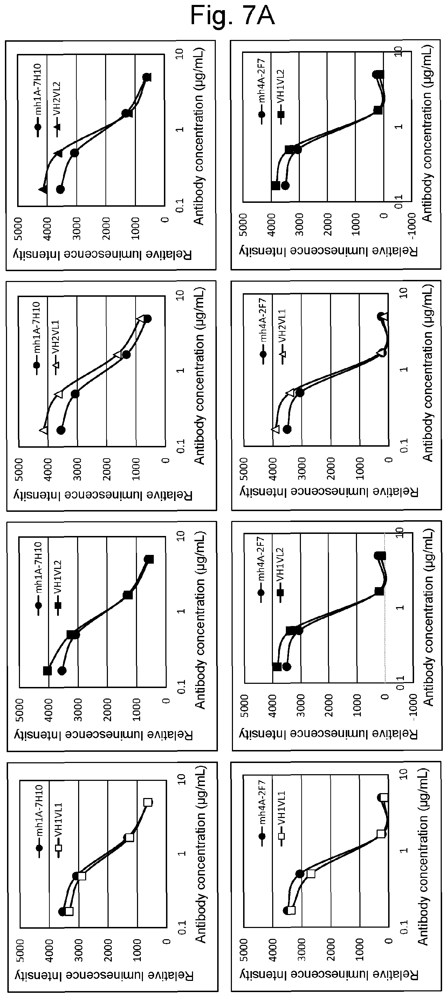

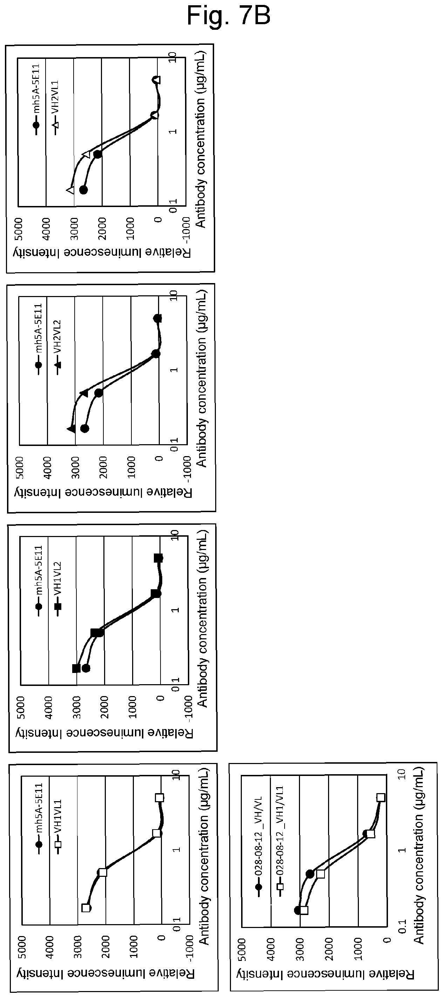

Depending on the combination of humanized sequences, 4 types of antibodies VH1/VL1, VH2/VL1, VH2/VL1 and VH2/VL2 are obtained for each clone. These humanized antibodies were evaluated for their antigen-binding activity and neutralizing activity by antigen-immobilized ELISA and Ca²⁺ Flux Assay, and all the combinations were confirmed to have antigen-binding activity and neutralizing activity comparable to those of their original chimeric antibody (mh1A-7H10_VH1/VL, mh4A-2F7_VH/VL1 or mh5A-5E11_VH7/VL) (Figures 6, 7A and 7B). This indicates that these humanized antibodies have *in vitro* HuCXCL1 binding activity and *in vitro* inhibitory activity against HuCXCL1 stimulatory signal transduction (inhibitory activity against the migration of CXCR2-expressing cells) comparable to those of their original chimeric antibody. From among these humanized antibodies, those whose modified amino acids were more highly homologous to framework portions in human antibody sequences were selected. The combinations and SEQ ID NOs of each antibody thus selected are shown in Table 9 below.

**[Table 9]**

| | Human heavy chain variable region | | CDR1 | CDR2 | CDR3 | Human light chain variable region | | CDR1 | CDR2 | CDR3 |
|---|---|---|---|---|---|---|---|---|---|---|
| Hu1A-7H10 | VH1 | SEQ ID NO: 68 | SEQ ID NO: 2 | SEQ ID NO: 34 | SEQ ID NO: 4 | VL1 | SEQ ID NO: 70 | SEQ ID NO: 6 | SEQ ID NO: 7 | SEQ ID NO: 8 |
| Hu4A-2F7 | VH1 | SEQ ID NO: 72 | SEQ ID NO: 10 | SEQ ID NO: 11 | SEQ ID NO: 12 | VL1 | SEQ ID NO: 74 | SEQ ID NO: 14 | SEQ ID NO: 36 | SEQ ID NO: 16 |
| Hu5A-5E11 | VH1 | SEQ ID NO: 76 | SEQ ID NO: 18 | SEQ ID NO: 54 | SEQ ID NO: 20 | VL1 | SEQ ID NO: 78 | SEQ ID NO: 22 | SEQ ID NO: 23 | SEQ ID NO: 24 |
| 028-8-12 | VH1 | SEQ ID NO: 63 | SEQ ID NO: 26 | SEQ ID NO: 64 | SEQ ID NO: 28 | VL1 | SEQ ID NO: 67 | SEQ ID NO: 30 | SEQ ID NO: 31 | SEQ ID NO: 32 |

### [Example 8]

### [8] In vivo evaluation tests on humanized antibodies and modified human antibody

As described above, humanized antibodies were prepared for three mouse antibody clones having CXCR2 migration inhibitory activity and antitumor activity *in vivo* (hereinafter designated as Hu1A-7H10, Hu4A-2F7 and Hu5A-5E11, respectively, or collectively referred to as humanized antibodies). Moreover, amino acid modifications were made to the CDR regions and VL region of the human antibody 028-8-12 obtained by the ADLib technique to thereby prepare one modified human antibody clone (hereinafter referred to as 028-8-12 or modified human antibody).

The humanized antibodies were evaluated for their *in vivo* antitumor activity and CXCR2 migration inhibitory activity in a xenograft model with OCUM-12 orthotopic transplantation. Procedures for the evaluation are shown below.

For use as mice, female nude mice (BALB/cAJcl-nu/nu) at 5 to 6 weeks of age were purchased from CLEA Japan, Inc., Japan. For use as human cancer cells, gastric cancer cell line, OCUM-12, was provided by Osaka Metropolitan University, Japan.

OCUM-12 cells were cultured in D-MED medium (FUJIFILM Wako Pure Chemical Corporation, Japan) supplemented with 10% fetal bovine serum (hereinafter abbreviated as FBS; Sigma-Aldrich Corporation) and 1/100 volumes of a penicillin-streptomycin mixture (Nacalai Tesque, Inc., Japan). The cells to be transplanted were detached with 0.25% trypsin/0.02% EDTA (Thermo Fisher Scientific) and washed with PBS, and 4 × 10⁵ cells per mouse were transplanted into the gastric wall of each mouse. On the day following the transplantation, the mice were randomized into groups (8 mice per group) and intraperitoneally administered with test antibodies at a dose of 400 µg/head. PBS was intraperitoneally administered in a volume of 200 µL with a frequency of twice a week for 3 weeks, starting from the day following the transplantation. The administration of the test drugs was then continued with a frequency of twice a week until the completion of the test. After 3 weeks had passed from the transplantation, the mice were sacrificed, and the short- and long-axis diameters of tumors formed in their gastric walls were each measured with a caliper. The tumor size was calculated according to the following: 1/2 short-axis diameter (mm) × 1/2 long-axis diameter (mm) × 3.14.

The stomachs after measurement were excised and fixed overnight at room temperature with a 10% neutral buffered formalin solution (Wako Pure Chemical Industries, Ltd., Japan), followed by replacement with PBS. Then, paraffin embedding and section preparation were outsourced to Applied Medical Research Laboratory, Japan. The prepared gastric tumor sections were immunohistologically stained with an anti-CXCR2 antibody to evaluate *in vivo* CXCR2 cell migration inhibitory activity. The sections were deparaffinized and rehydrated, and then treated to inactivate endogenous peroxidase. After antigen activation treatment, the sections were blocked with PBS containing 2% skimmed milk and 0.1% Tween-20 (hereinafter referred to as the blocking solution), and reacted overnight at 4°C with an anti-CXCR2 antibody (Biorbyt) as a primary antibody, which had been diluted 1667-fold with the blocking solution. After washing with PBS containing 0.1% Tween-20, the sections were reacted at room temperature for 1 hour with an HRP-labeled goat anti-rabbit IgG antibody (Abcam) as a secondary antibody. After washing with a washing solution, a simple stain DAB solution (5052, Nichirei Corporation, Japan) was used to cause color development. After images of the stained tissues were captured with an Explore (Olympus Corporation, Japan), imaging software CellSens (Olympus Corporation, Japan) was used to calculate positive cells per unit area.

### [8-1] In vivo CXCR2-expressing cell migration inhibitory activity of humanized antibodies and modified human antibody

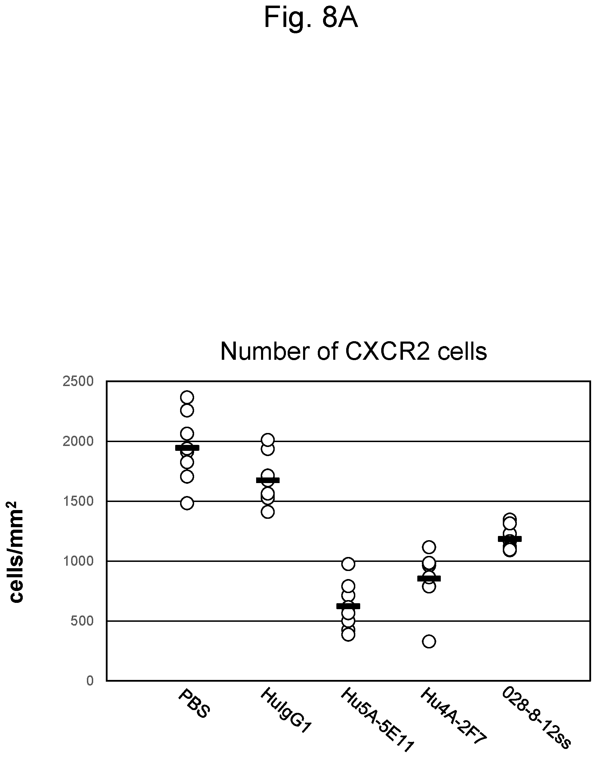

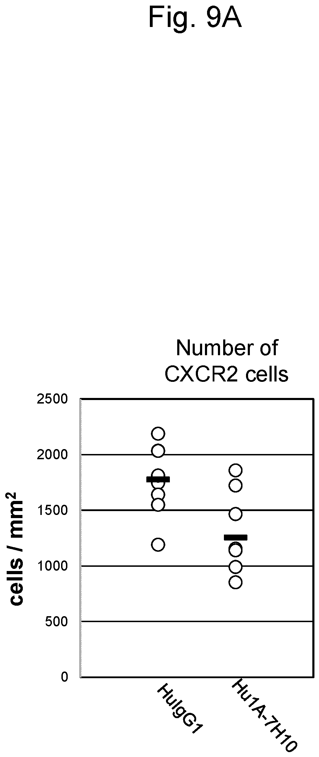

According to the above procedures, the humanized antibodies Hu1A-7H10, Hu4A-2F7 and Hu5A-5E11 and the modified human antibody 028-8-12 were measured for their inhibitory activity against the migration of CXCR2-expressing cells. The results obtained are shown in Figure 8A and Figure 9A. As indicated in these results, the groups receiving the humanized antibodies Hu1A-7H10, Hu4A-2F7 and Hu5A-5E11 and the modified human antibody 028-8-12 were found to significantly suppress the migration of CXCR2-expressing cells when compared to the group receiving hIgG1 antibody.

### [8-2] In vivo antitumor activity of humanized antibodies and modified human antibody

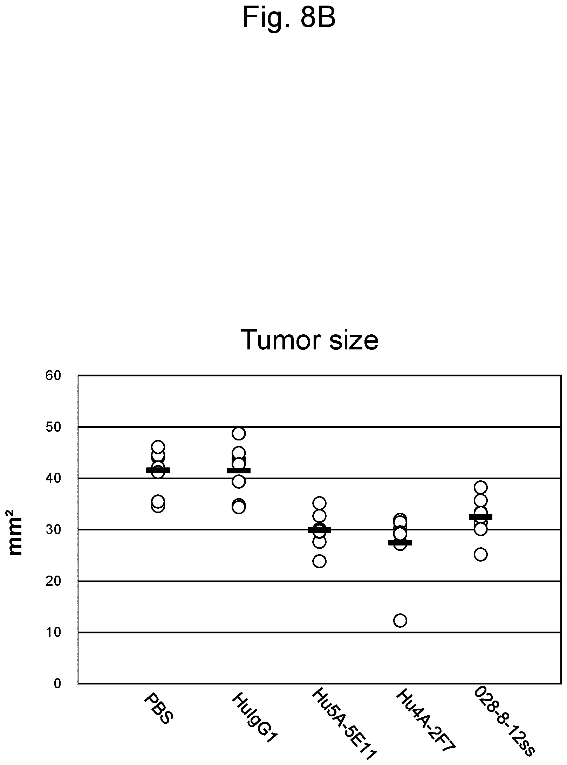

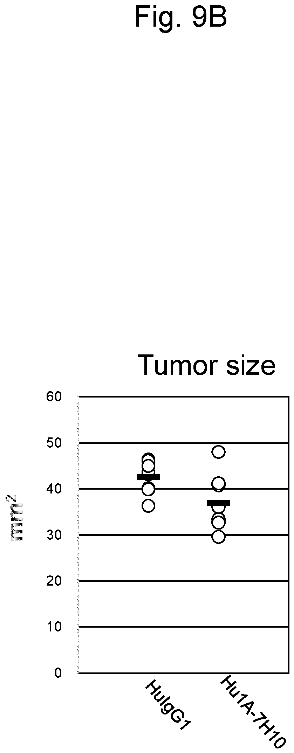

According to the above procedures, the mice administered with the humanized antibodies Hu1A-7H10, Hu4A-2F7 and Hu5A-5E11 and the modified human antibody 028-8-12 were measured for their tumor area. The results obtained are shown in Figure 8B and Figure 9B. As indicated in these results, the groups receiving Hu1A-7H10, Hu4A-2F7, Hu5A-5E11 and 028-8-12 were found to significantly suppress an increase in the tumor area when compared to the group receiving hIgG1 antibody.

In view of the foregoing, in the *in vivo* tests, the tree humanized antibodies (Hu1A-7H10, Hu4A-2F7 and Hu5A-5B11) and the modified human antibody (028-8-12) were found to have sufficient migration inhibitory activity when compared to the hIgG1 antibody, and have an antitumor effect.

### [Example 9]

### [9] Dose-response test on humanized antibody Hu5A-5E11 in a prevention model with OCUM-12 orthotopic transplantation

The dose-response relationship between antibody dose and *in vivo* antitumor activity or CXCR2 migration inhibitory activity was evaluated in a xenograft prevention model with OCUM-12 orthotopic transplantation. A prevention model is a model designed to start antibody administration from the day following tumor transplantation. The mice, cells, transplantation procedures, and evaluation procedures for antitumor activity and CXCR2 migration inhibitory activity as shown in [8] above were used. As a test antibody, Hu5A-5E11 was used and intraperitoneally administered at a dose of 20, 60, 100, 200 or 400 µg/head with a frequency of twice a week for 3 weeks, starting from the day following the transplantation. PBS was intraperitoneally administered in a volume of 200 µL with a frequency of twice a week for 3 weeks, starting from the day following the transplantation.

### [9-1] In vivo CXCR2-expressing cell migration inhibitory activity of Hu5A-5E11

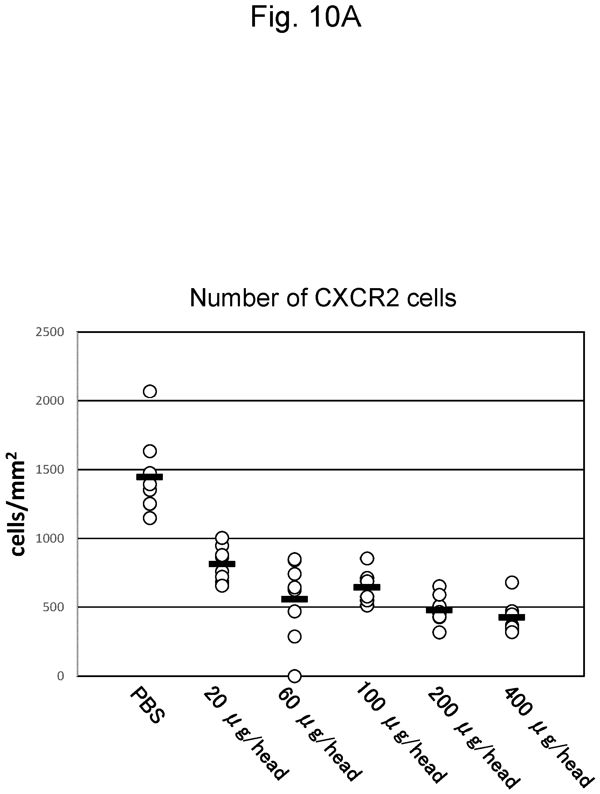

The results obtained are shown in Figure 10A. As indicated in these results, Hu5A-5E11 was found to significantly suppress the migration of CXCR2-expressing cells at a dose of 20 µg/head or more when compared to PBS administration, and the minimum efficacy dose was shown to be 20 µg/head or less. The migration suppressive effect tended to reach a plateau at a dose of 60 µg/head or more.

### [9-2] In vivo antitumor activity of Hu5A-5E11

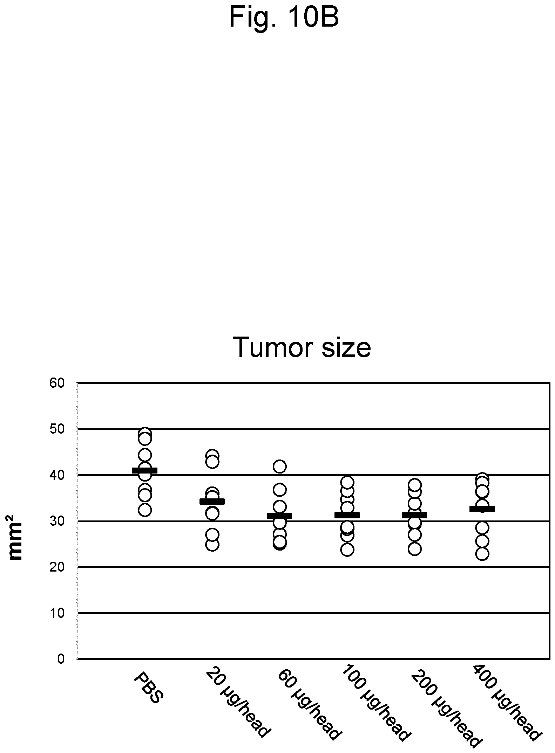

The evaluation results obtained are shown in Figure 10B. As indicated in these results, Hu5A-5E11 was found to significantly suppress the tumor area at a dose of 60 µg/head or more when compared to PBS administration.

### [Example 10]

### [10] Dose-response test on Hu5A-5E11 in a treatment model with OCUM-12 orthotopic transplantation

When OCUM-12 cells are transplanted into the mouse gastric wall, the established tumor is formed at 7 days after the transplantation, whose histological image and CXCR2 cell distribution are equal to those on day 21 of the transplantation. Based on this finding, a model designed to start test drug administration from 7 days after the transplantation was used as a treatment model.

The dose-response relationship between antibody dose and *in vivo* antitumor activity or CXCR2 migration inhibitory activity was evaluated in a xenograft treatment model with OCUM-12 orthotopic transplantation. The mice, transplantation procedures, and evaluation procedures for antitumor activity and CXCR2 migration inhibitory activity as shown in [8] above were used. As a test drug, Hu5A-5E11 was used. The administration of the test drug was started from 7 days after the transplantation, and the test drug was intraperitoneally administered at a dose of 20, 60, 100, 200 or 400 µg/head with a frequency of twice a week for 3 weeks. PBS was intraperitoneally administered in a volume of 200 µL with a frequency of twice a week for 3 weeks, starting from 7 days after the transplantation. After 4 weeks had passed from the transplantation, the mice were sacrificed and evaluated.

### [10-1] In vivo CXCR2-expressing cell migration inhibitory activity of Hu5A-5E11

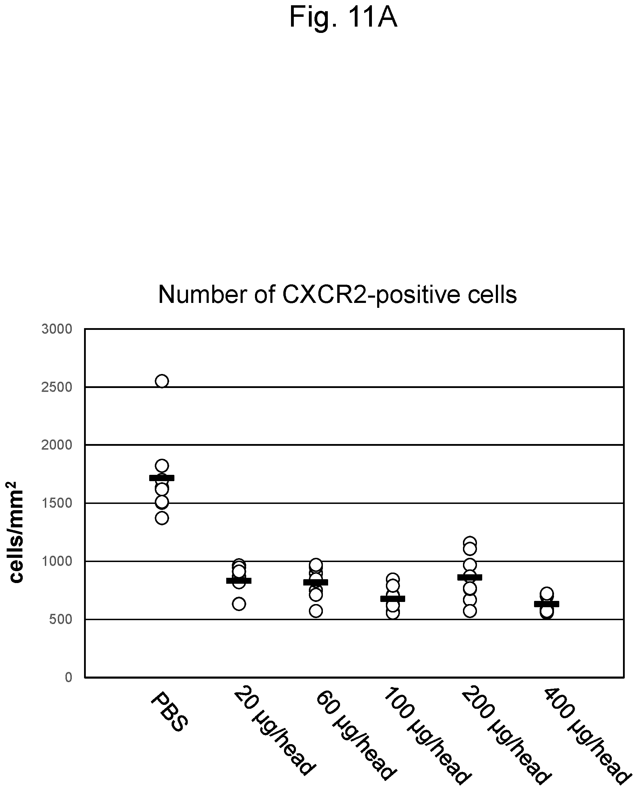

The evaluation results obtained are shown in Figure 11A. As indicated in these results, Hu5A-5E11 was found to significantly suppress the migration of CXCR2-expressing cells at a dose of 20 µg/head or more when compared to PBS administration, and the minimum efficacy dose was shown to be 20 µg/head or less. The migration suppressive effect tended to reach a plateau at a dose of 60 µg/head or more.

### [10-2] In vivo antitumor activity of Hu5A-5E11

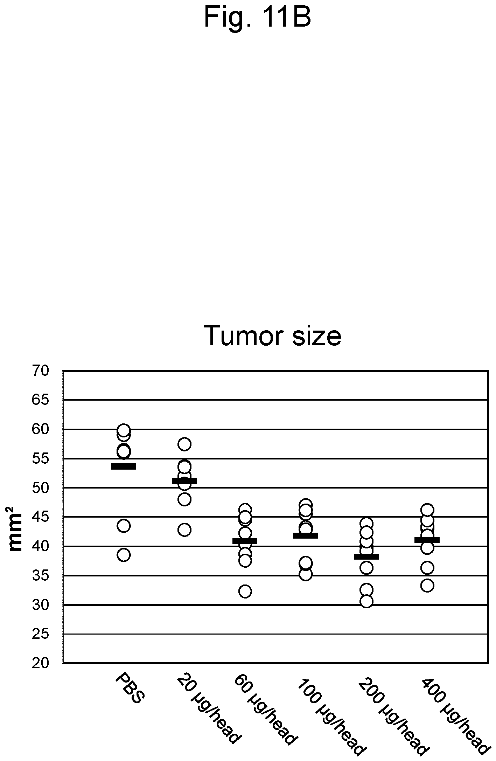

The evaluation results obtained are shown in Figure 11B. As indicated in these results, Hu5A-5E11 was found to significantly suppress the tumor area at a dose of 60 µg/head or more when compared to PBS administration.

*In vivo* dose response was evaluated in two models designed to start the administration of humanized Hu5A-5E11 from the day following the transplantation or from 7 days after the transplantation. In either model, humanized Hu5A-5E11 was found to significantly inhibit the migration of CXCR2-expressing cells at a dose of 20 µg/head or more when compared to PBS administration. Moreover, humanized Hu5A-5E11 was also found to significantly suppress the tumor area at a dose of 60 µg/head or more.

### [Example 11]

### [11] ELISA-based evaluation of the inhibitory activity of Hu5A-5E11 against the binding of HuCXCL1 to 3 and 5 or MsCXCL1 and 2 to glucosaminoglycan (GAG)

Glucosaminoglycan (hereinafter abbreviated as GAG) including heparin and heparan sulfate is present in the form of proteoglycan *in vivo* and is expressed on the cell surface of various cells. GAG has a strong negative charge, and directly binds to other proteins such as chemokines, cytokines and growth factors (hereinafter referred to as ligands) to thereby contribute to ligand enrichment near the target receptor, stabilization of the receptor-ligand complex, protection of protease-induced ligand degradation, etc., so that GAG plays an important role in biological functions. Moreover, animal models and cellular studies have demonstrated that interaction between GAG and chemokine determines the concentration gradient of the chemokine, and this concentration gradient induces or regulates the trafficking of CXCR2-expressing cells to tissue (or cell migration to tissue) (see Non-patent Document 4 listed above).

Thus, the effect of Hu5A-5E11 on the binding of CXCLs to GAG was evaluated by ELISA assay using plates on which the antigen (HuCXCL1 protein) used for immunization or HuCXCL2, 3 and 5 and MsCXCL1 and 2 proteins had been directly immobilized. CXCLs other than HuCXCL1 were purchased from PROTEINTECH and used. Biotin-labeled heparan sulfate and heparin were purchased from PG Research and used.

To 96-well plates, phosphate-buffered saline (PBS) containing 3 µg/mL of HuCXCL1, 2, 3 or 5 or MsCXCL1 or 10 µg/mL of MsCXCL2 was added in a volume of 50 µL per well, and the plates were allowed to stand overnight at 4°C. After removal of the antigen solutions, PBS containing 0.05% Tween-20 (hereinafter referred to as the blocking solution) was added to each well for blocking reaction, and the plates were allowed to stand at room temperature for 30 minutes. After removal of the blocking solution, solutions containing Hu5A-5E11 or HuIgG1 antibody diluted to concentrations of 0 to 10 µg/mL with the blocking solution were each added in a volume of 50 µL per well and reacted at room temperature for 30 minutes. After removal of the antibody solutions, biotin-labeled heparin or heparan sulfate diluted to a concentration of 10 µg/mL with the blocking solution was added and reacted at room temperature for 2 hours. After washing, an HRP-labeled secondary antibody diluted with the blocking solution was added and reacted at room temperature for 40 minutes. TMB (Dako) was added as a chromogenic substrate solution to cause color development, and 1 N sulfuric acid was added to stop the reaction, followed by measurement of absorbance at 450 nm. The binding rate of CXCLs to GAG was calculated as a ratio relative to the absorbance measured in the absence of the antibody, which was set to 100%.

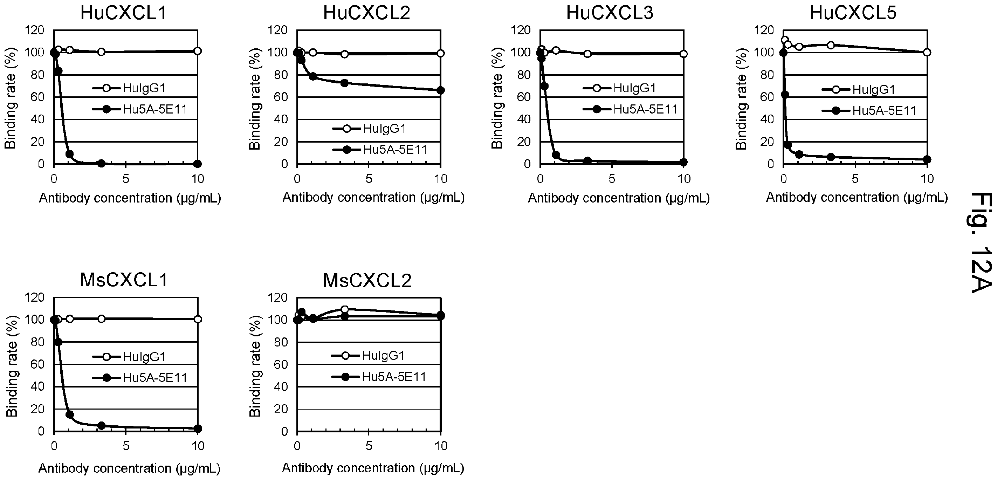

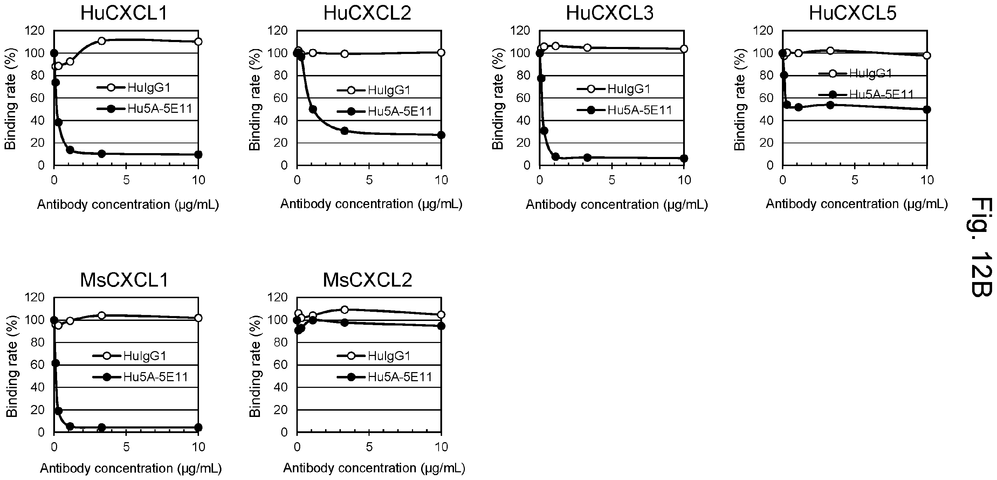

The evaluation results obtained are shown in Figure 12A and Figure 12B. Hu5A-5E11 was found to inhibit the binding of HuCXCL1, 2, 3 and 5 and MsCXCL1 to GAG in an antibody concentration-dependent manner.

### [Example 12]

### [12] FACS-based evaluation of the inhibitory activity of Hu5A-5E11 against the binding of HuCXCL1 to 3 and 5 or MsCXCL1 to cells

Chemokines are stabilized by directly binding to GAG on the cell surface and thereby allow efficient signal transduction to their receptors. Hu5A-5E11 was shown to inhibit the binding of CXCLs to GAG in [11] above. Then, to evaluate whether Hu5A-5E11 inhibits the binding of CXCLs to cells, the binding of fluorescently labeled CXCLs to cells was measured as fluorescence intensity by flow cytometry in FACS. For this purpose, HEK293 cells forced to express HuCXCR2 were used. For use as HuCXCL1, 2, 3 and 5 and MsCXCL1, the proteins in [11] above were labeled with APC.

The cultured cells were detached and washed with PBS, and then suspended at 5.0 × 10⁵ cells/mL in PBS containing 1% BSA (hereinafter referred to as the FACS buffer). Each of the APC-labeled ligands and Hu5A-5E11 were mixed at final concentrations of 10 µg/mL and 16.7 µg/mL, respectively, and reacted at room temperature for 30 minutes, and then added to the cell suspension and reacted on ice for 30 minutes. After washing with the FACS buffer, the cells were suspended again in PBS containing 1% 7-AAD, 0.5% BSA and 2 mM EDTA, followed by the above flow cytometry to measure fluorescence intensity.

As a result, Hu5A-5E1 1 was found to reduce the fluorescence intensity of the cells in an antibody concentration-dependent manner. This indicated that Hu5A-5E11 inhibited the binding of HuCXCL1, 2, 3 and 5 and MsCXCL 1 to CXCR2 or GAG and inhibited their biding to the cells per se.

Based on the results of Example 2, Hu5A-5E11 was confirmed to neutralize HuCXCL1 and inhibit signal transduction to CXCR2. Moreover, based on the results of Examples 11 and 12, Hu5A-5E11 was confirmed to inhibit the binding of GAG to HuCXCL1, 2, 3 and 5 and MsCXCL1 and confirmed to inhibit the binding of HuCXCL1, 2, 3 and 5 and MsCXCL1 to the cell surface. These results indicate that Hu5A-5E11 is expected to inhibit the migration of CXCR2 cells by not only neutralizing HuCXCL1 but also inhibiting the binding of HuCXCL1, 2, 3 and 5 and MsCXCL1 to the cell surface. Moreover, Hu5A-5E11 may be expected to inhibit the *in vivo* formation of chemokine concentration gradients mediated by HuCXCL1, 2, 3 and 5 and MsCXCL1 and GAG, thereby affecting the migration of CXCR2 cells toward tissues including tumor.

### Industrial Applicability

The present invention enables the provision of an anti-human CXCL1 antibody and a fragment thereof, which are capable of inducting alterations in the tumor microenvironment (TME), including the inhibition or suppression of TME formation. The anti-human CXCL1 antibody and fragment thereof according to the present invention are useful, for example, in terms of having antitumor activity and being available for use in the treatment and/or prevention of a tumor, etc. Moreover, the present invention also enables the provision of a pharmaceutical composition and a kit, etc., each comprising the anti-human CXCL1 antibody or a fragment thereof.

### Sequence Listing Free Text

SEQ ID NOs: 33 to 79: recombinant peptides

## Claims

1. An antibody against human CXCL1, wherein
(a) the amino acid sequences of heavy chain variable region (VH) complementarity determining region (CDR) 1, CDR2 and CDR3 consist of:
the amino acid sequences shown in SEQ ID NOs: 2, 3 and 4, respectively; or
the amino acid sequences shown in SEQ ID NOs: 2, 34 and 4, respectively, and
the amino acid sequences of light chain variable region (VL) CDR1, CDR2 and CDR3 consist of:
the amino acid sequences shown in SEQ ID NOs: 6, 7 and 8, respectively,
(b) the amino acid sequences of VH CDR1, CDR2 and CDR3 consist of:
the amino acid sequences shown in SEQ ID NOs: 10, 11 and 12, respectively, and
the amino acid sequences of VL CDR1, CDR2 and CDR3 consist of:
the amino acid sequences shown in SEQ ID NOs: 14, 15 and 16, respectively;
the amino acid sequences shown in SEQ ID NOs: 14, 36 and 16, respectively;
the amino acid sequences shown in SEQ ID NOs: 14, 38 and 16, respectively; or
the amino acid sequences shown in SEQ ID NOs: 14, 40 and 16, respectively,
(c) the amino acid sequences of VH CDR1, CDR2 and CDR3 consist of:
the amino acid sequences shown in SEQ ID NOs: 18, 19 and 20, respectively;
the amino acid sequences shown in SEQ ID NOs: 18, 42 and 20, respectively;
the amino acid sequences shown in SEQ ID NOs: 18, 44 and 20, respectively;
the amino acid sequences shown in SEQ ID NOs: 18, 46 and 20, respectively;
the amino acid sequences shown in SEQ ID NOs: 18, 48 and 20, respectively;
the amino acid sequences shown in SEQ ID NOs: 18, 50 and 20, respectively;
the amino acid sequences shown in SEQ ID NOs: 18, 52 and 20, respectively;
the amino acid sequences shown in SEQ ID NOs: 18, 54 and 20, respectively;
the amino acid sequences shown in SEQ ID NOs: 18, 56 and 20, respectively;
the amino acid sequences shown in SEQ ID NOs: 18, 58 and 20, respectively;
the amino acid sequences shown in SEQ ID NOs: 18, 60 and 20, respectively; or
the amino acid sequences shown in SEQ ID NOs: 18, 62 and 20, respectively, and
the amino acid sequences of VL CDR1, CDR2 and CDR3 consist of:
the amino acid sequences shown in SEQ ID NOs: 22, 23 and 24, respectively, or
(d) the amino acid sequences of VH CDR1, CDR2 and CDR3 consist of:
the amino acid sequences shown in SEQ ID NOs: 26, 27 and 28, respectively;
the amino acid sequences shown in SEQ ID NOs: 26, 64 and 28, respectively; or
the amino acid sequences shown in SEQ ID NOs: 26, 66 and 28, respectively, and
the amino acid sequences of VL CDR1, CDR2 and CDR3 consist of:
the amino acid sequences shown in SEQ ID NOs: 30, 31 and 32, respectively.

2. An antibody, wherein
(a) the amino acid sequences of VH CDR1, CDR2 and CDR3 consist of the amino acid sequences shown in SEQ ID NOs: 2, 34 and 4, respectively, and
the amino acid sequences of VL CDR1, CDR2 and CDR3 consist of the amino acid sequences shown in SEQ ID NOs: 6, 7 and 8, respectively,
(b) the amino acid sequences of VH CDR1, CDR2 and CDR3 consist of the amino acid sequences shown in SEQ ID NOs: 10, 11 and 12, respectively, and
the amino acid sequences of VL CDR1, CDR2 and CDR3 consist of the amino acid sequences shown in SEQ ID NOs: 14, 36 and 16, respectively,
(c) the amino acid sequences of VH CDR1, CDR2 and CDR3 consist of the amino acid sequences shown in SEQ ID NOs: 18, 54 and 20, respectively, and
the amino acid sequences of VL CDR1, CDR2 and CDR3 consist of the amino acid sequences shown in SEQ ID NOs: 22, 23 and 24, respectively, or
(d) the amino acid sequences of VH CDR1, CDR2 and CDR3 consist of the amino acid sequences shown in SEQ ID NOs: 26, 64 and 28, respectively, and
the amino acid sequences of VL CDR1, CDR2 and CDR3 consist of the amino acid sequences shown in SEQ ID NOs: 30, 31 and 32, respectively.

3. An antibody against human CXCL1, wherein
(a) the amino acid sequence of the heavy chain variable region (VH) consists of the amino acid sequence shown in SEQ ID NO: 1 or 33, and the amino acid sequence of the light chain variable region (VL) consists of the amino acid sequence shown in SEQ ID NO: 5,
(b) the amino acid sequence of VH consists of the amino acid sequence shown in SEQ ID NO: 9, and the amino acid sequence of VL consists of the amino acid sequence shown in SEQ ID NO: 13, 35, 37 or 39, or
(c) the amino acid sequence of VH consists of the amino acid sequence shown in SEQ ID NO: 17, 41, 43, 45, 47, 49, 51, 53, 55, 57, 59 or 61, and the amino acid sequence of VL consists of the amino acid sequence shown in SEQ ID NO: 21.

4. An antibody against human CXCL1, wherein
(a) the amino acid sequence of the heavy chain variable region (VH) consists of the amino acid sequence shown in SEQ ID NO: 68 or 69, and the amino acid sequence of the light chain variable region (VL) consists of the amino acid sequence shown in SEQ ID NO: 70 or 71,
(b) the amino acid sequence of VH consists of the amino acid sequence shown in SEQ ID NO: 72 or 73, and the amino acid sequence of VL consists of the amino acid sequence shown in SEQ ID NO: 74 or 75,
(c) the amino acid sequence of VH consists of the amino acid sequence shown in SEQ ID NO: 76 or 77, and the amino acid sequence of VL consists of the amino acid sequence shown in SEQ ID NO: 78 or 79, or
(d) the amino acid sequence of VH consists of the amino acid sequence shown in SEQ ID NO: 25, 63 or 65, and the amino acid sequence of VL consists of the amino acid sequence shown in SEQ ID NO: 29 or 67.

5. An antibody against human CXCL1, wherein
(a) the amino acid sequence of VH consists of the amino acid sequence shown in SEQ ID NO: 68, and the amino acid sequence of VL consists of the amino acid sequence shown in SEQ ID NO: 70,
(b) the amino acid sequence of VH consists of the amino acid sequence shown in SEQ ID NO: 72, and the amino acid sequence of VL consists of the amino acid sequence shown in SEQ ID NO: 74,
(c) the amino acid sequence of VH consists of the amino acid sequence shown in SEQ ID NO: 76, and the amino acid sequence of VL consists of the amino acid sequence shown in SEQ ID NO: 78, or
(d) the amino acid sequence of VH consists of the amino acid sequence shown in SEQ ID NO: 63, and the amino acid sequence of VL consists of the amino acid sequence shown in SEQ ID NO: 67.

6. The antibody according to any one of claims 1, 2, 4 and 5, wherein the antibody is a human antibody.

7. The antibody according to any one of claims 1 to 6, wherein the antibody has antitumor activity.

8. The antibody according to any one of claims 1 to 7, which is used for the treatment or prevention of a tumor.

9. The antibody according to claim 7 or 8, wherein the tumor is associated with stromal hyperplasia.

10. The antibody according to any one of claims 7 to 9, wherein the tumor is at least one selected from the group consisting of human gastric cancer, human pancreatic cancer, human breast cancer, human lung cancer, human skin cancer, human ovarian cancer, human colorectal cancer, human bladder cancer, human liver cancer, human esophageal cancer, prostate cancer and human biliary tract cancer.

11. The antibody according to claim 10, wherein the human gastric cancer is scirrhous gastric cancer.

12. The antibody according to any one of claims 1 to 6, which has inhibitory or suppressive activity against the migration of CXCR2-expressing cells.

13. An antibody fragment derived from the antibody according to any one of claims 1 to 12.

14. A pharmaceutical composition comprising the antibody according to any one of claims 1 to 12 and/or the antibody fragment according to claim 13.

15. The pharmaceutical composition according to claim 14, which is used for the treatment or prevention of a tumor.

16. The pharmaceutical composition according to claim 15, wherein the tumor is associated with stromal hyperplasia.

17. The pharmaceutical composition according to claim 15 or 16, wherein the tumor is at least one selected from the group consisting of human gastric cancer, human pancreatic cancer, human breast cancer, human lung cancer, human skin cancer, human ovarian cancer, human colorectal cancer, human bladder cancer, human liver cancer, human esophageal cancer, prostate cancer and human biliary tract cancer.

18. The pharmaceutical composition according to claim 17, wherein the human gastric cancer is scirrhous gastric cancer.

19. The pharmaceutical composition according to any one of claims 14 to 18, which further comprises or is used in combination with at least one or more selected from the group consisting of a compound having antitumor activity, a compound having cell killing activity, and an immune checkpoint inhibitor.

20. The pharmaceutical composition according to claim 14, which is used for the inhibition or suppression of the migration of CXCR2-expressing cells.

21. A method for treating or preventing a tumor, which comprises administering the antibody according to any one of claims 1 to 12 and/or the antibody fragment according to claim 13, or the pharmaceutical composition according to any one of claims 14 to 19 to a subj ect.

22. The method according to claim 21, wherein the tumor is associated with stromal hyperplasia.

23. The method according to claim 21 or 22, wherein the tumor is at least one selected from the group consisting of human gastric cancer, human pancreatic cancer, human breast cancer, human lung cancer, human skin cancer, human ovarian cancer, human colorectal cancer, human bladder cancer, human liver cancer, human esophageal cancer, prostate cancer and human biliary tract cancer.

24. The method according to claim 23, wherein the human gastric cancer is scirrhous gastric cancer.

25. A method for inhibiting or suppressing the migration of CXCR2-expressing cells, which comprises administering the antibody according to any one of claims 1 to 12 and/or the antibody fragment according to claim 13, or the pharmaceutical composition according to claim 20 to a subject.

26. A kit for treating, preventing or diagnosing a tumor, which comprises the antibody according to any one of claims 1 to 12 and/or the antibody fragment according to claim 13.

27. The kit according to claim 26, wherein the tumor is associated with stromal hyperplasia.

28. The kit according to claim 26 or 27, wherein the tumor is at least one selected from the group consisting of human gastric cancer, human pancreatic cancer, human breast cancer, human lung cancer, human skin cancer, human ovarian cancer, human colorectal cancer, human bladder cancer, human liver cancer, human esophageal cancer, prostate cancer and human biliary tract cancer.

29. The kit according to claim 28, wherein the human gastric cancer is scirrhous gastric cancer.

30. An inhibitor or suppressor of the binding of human CXCL and/or mouse CXCL to glucosaminoglycan, which comprises the antibody according to any one of claims 1 to 12 and/or the antibody fragment according to claim 13.


SEQUENCE LISTING

<110>  Chiome Bioscience Inc
       University Public Corporation Osaka

<120>  Anti-hCXCL1 antibody

<130>  G3032WO

<150>  JP 2022-011107
<151>  2022-01-27

<160>  82

<170>  PatentIn version 3.5

<210>  1
<211>  116
<212>  PRT
<213>  Mus musculus

<400>  1

Gln Val Gln Leu Lys Glu Ser Gly Pro Val Leu Val Ala Pro Ser Gln
1               5                   10                  15

Ser Leu Ser Ile Thr Cys Ser Val Ser Gly Phe Ser Ser Thr Thr Tyr
            20                  25                  30

Gly Val His Trp Val Arg Gln Pro Pro Gly Gln Gly Leu Glu Trp Leu
        35                  40                  45

Gly Val Ile Trp Ala Gly Gly Ser Thr Asn Tyr Asn Ser Ala Leu Met
    50                  55                  60

Ser Arg Leu Ser Ile Ser Lys Asp Asn Ser Lys Ser Gln Val Phe Leu
65                  70                  75                  80

Lys Met Asn Ser Leu Gln Thr Asp Asp Thr Ala Met Tyr Tyr Cys Ala
                85                  90                  95

Thr Gln Tyr His Tyr Pro Phe Ala Tyr Trp Gly Gln Gly Thr Leu Val
            100                 105                 110

Thr Val Ser Ala
        115

<210>  2
<211>  5
<212>  PRT
<213>  Mus musculus

<400>  2

Thr Tyr Gly Val His
1               5

<210>  3
<211>  16
<212>  PRT
<213>  Mus musculus

<400>  3

Val Ile Trp Ala Gly Gly Ser Thr Asn Tyr Asn Ser Ala Leu Met Ser
1               5                   10                  15

<210>  4
<211>  8
<212>  PRT
<213>  Mus musculus

<400>  4

Gln Tyr His Tyr Pro Phe Ala Tyr
1               5

<210>  5
<211>  107
<212>  PRT
<213>  Mus musculus

<400>  5

Asn Ile Val Met Thr Gln Ser Pro Lys Ser Met Ser Met Ser Val Gly
1               5                   10                  15

Glu Arg Val Thr Leu Ser Cys Lys Ala Ser Glu Asn Val Gly Ile Tyr
            20                  25                  30

Val Ser Trp Tyr Gln Gln Lys Pro Glu Gln Ser Pro Lys Leu Met Ile
        35                  40                  45

Tyr Gly Ala Ser Asn Arg Asn Thr Gly Val Pro Asp Arg Phe Thr Gly
    50                  55                  60

Ser Gly Ser Ala Thr Asp Phe Thr Leu Thr Ile Ser Ser Val Gln Ala
65                  70                  75                  80

Glu Asp Leu Ala Asp Tyr Tyr Cys Gly Gln Ser Tyr Ser Tyr Pro Leu
                85                  90                  95

Thr Phe Gly Ala Gly Thr Lys Leu Glu Leu Lys
            100                 105

<210>  6
<211>  11
<212>  PRT
<213>  Mus musculus

<400>  6

Lys Ala Ser Glu Asn Val Gly Ile Tyr Val Ser
1               5                   10

<210>  7
<211>  7
<212>  PRT
<213>  Mus musculus

<400>  7

Gly Ala Ser Asn Arg Asn Thr
1               5



<210>  8
<211>  9
<212>  PRT
<213>  Mus musculus

<400>  8

Gly Gln Ser Tyr Ser Tyr Pro Leu Thr
1               5



<210>  9
<211>  123
<212>  PRT
<213>  Mus musculus

<400>  9

Gln Val Thr Leu Lys Glu Ser Gly Pro Gly Ile Leu Lys Pro Ser Gln
1               5                   10                  15


Thr Leu Ser Leu Thr Cys Ser Phe Ser Gly Phe Ser Leu Ser Thr Ser
            20                  25                  30


Gly Met Gly Val Asp Trp Ile Arg Gln Pro Ser Gly Lys Gly Leu Glu
        35                  40                  45


Trp Leu Ala His Ile Trp Trp Asp Asp Asp Lys Tyr Tyr Asn Pro Ser
    50                  55                  60


Leu Lys Ser Gln Leu Thr Ile Ser Lys Asp Thr Ser Arg Asn Gln Val
65                  70                  75                  80


Phe Leu Lys Ile Thr Ser Val Asp Thr Ala Asp Thr Ala Thr Tyr Tyr

                85                  90                  95

Cys Ala Arg Ser Tyr Gly Tyr Asp Met Asp Tyr Tyr Ala Met Asp Tyr
            100                 105                 110

Trp Gly Gln Gly Thr Ser Val Thr Val Ser Ser
        115                 120

<210> 10
<211> 7
<212> PRT
<213> Mus musculus

<400> 10

Thr Ser Gly Met Gly Val Asp
1               5

<210> 11
<211> 16
<212> PRT
<213> Mus musculus

<400> 11

His Ile Trp Trp Asp Asp Asp Lys Tyr Tyr Asn Pro Ser Leu Lys Ser
1               5                   10                  15

<210> 12
<211> 13
<212> PRT
<213> Mus musculus

<400> 12

Ser Tyr Gly Tyr Asp Met Asp Tyr Tyr Ala Met Asp Tyr
1               5                   10

<210> 13
<211> 108

<212>  PRT
<213>  Mus musculus

<400>  13

Asp Ile Gln Met Thr Gln Ser Pro Ser Ser Leu Ser Ala Ser Leu Gly
1               5                   10                  15

Glu Arg Val Ser Leu Thr Cys Arg Ala Ser Gln Glu Ile Ser Gly Tyr
            20                  25                  30

Leu Ser Trp Leu Gln Gln Lys Pro Asp Gly Thr Ile Lys Arg Leu Ile
        35                  40                  45

Tyr Ala Ala Ser Thr Leu Asp Ser Gly Val Pro Lys Arg Phe Ser Gly
    50                  55                  60

Ser Arg Ser Gly Ser Asp Tyr Ser Leu Thr Ile Ser Ser Leu Glu Ser
65                  70                  75                  80

Glu Asp Phe Ala Asp Tyr Tyr Cys Leu Gln Tyr Ala Ser Tyr Pro Pro
                85                  90                  95

Trp Thr Phe Gly Gly Gly Thr Lys Leu Glu Ile Lys
            100                 105

<210>  14
<211>  11
<212>  PRT
<213>  Mus musculus

<400>  14

Arg Ala Ser Gln Glu Ile Ser Gly Tyr Leu Ser
1               5                   10

<210>  15
<211>  7

<212>  PRT
<213>  Mus musculus

<400>  15

Ala Ala Ser Thr Leu Asp Ser
1               5

<210>  16
<211>  10
<212>  PRT
<213>  Mus musculus

<400>  16

Leu Gln Tyr Ala Ser Tyr Pro Pro Trp Thr
1               5                   10

<210>  17
<211>  119
<212>  PRT
<213>  Mus musculus

<400>  17

Gln Val Gln Leu Gln Gln Ser Gly Ala Glu Leu Val Arg Pro Gly Ser
1               5                   10                  15

Ser Val Lys Ile Ser Cys Lys Ala Ser Gly Tyr Ala Phe Ser Asp Tyr
            20                  25                  30

Trp Met Asn Trp Val Lys Gln Arg Pro Gly Gln Gly Leu Glu Trp Ile
        35                  40                  45

Gly Gln Ile Tyr Pro Gly Asp Gly Asp Thr Asn Tyr Asn Gly Lys Phe
    50                  55                  60

Lys Gly Lys Gly Thr Leu Thr Ala Asp Lys Ser Ser Ser Thr Ala Tyr
65                  70                  75                  80

Met Gln Leu Asn Ser Leu Thr Ser Glu Asp Ser Ala Val Tyr Phe Cys
                85                  90                  95

Ala Arg Glu Arg Asp Tyr Asp Val Ala Met Asp Tyr Trp Gly Gln Gly
            100                 105                 110

Thr Ser Val Thr Val Ser Ser
        115

<210>  18
<211>  5
<212>  PRT
<213>  Mus musculus

<400>  18

Asp Tyr Trp Met Asn
1               5

<210>  19
<211>  17
<212>  PRT
<213>  Mus musculus

<400>  19

Gln Ile Tyr Pro Gly Asp Gly Asp Thr Asn Tyr Asn Gly Lys Phe Lys
1               5                   10                  15

Gly

<210>  20
<211>  10
<212>  PRT
<213>  Mus musculus

<400>  20

Glu Arg Asp Tyr Asp Val Ala Met Asp Tyr
1               5                   10

<210>  21
<211>  107
<212>  PRT
<213>  Mus musculus

<400>  21

Asp Ile Gln Met Thr Gln Ser Pro Ser Ser Leu Ser Ala Ser Leu Gly
1               5                   10                  15

Gly Lys Val Thr Ile Thr Cys Lys Ala Ser Gln Asp Ile Asn Lys Tyr
            20                  25                  30

Ile Ala Trp Phe Gln His Lys Pro Gly Lys Gly Pro Arg Leu Val Ile
        35                  40                  45

His Tyr Thr Ser Ile Leu Gln Pro Gly Ile Pro Ser Arg Phe Ser Gly
    50                  55                  60

Val Gly Ser Gly Arg Asp Tyr Ser Phe Ser Ile Arg Asn Leu Glu Pro
65                  70                  75                  80

Glu Asp Ile Ala Thr Tyr Tyr Cys Leu Gln Tyr Asp Asn Leu Pro Trp
                85                  90                  95

Thr Phe Gly Gly Gly Thr Lys Leu Glu Ile Lys
            100                 105

<210>  22
<211>  11
<212>  PRT
<213>  Mus musculus

<400>  22

Lys Ala Ser Gln Asp Ile Asn Lys Tyr Ile Ala
1               5                   10

<210>  23
<211>  7
<212>  PRT
<213>  Mus musculus

<400>  23

Tyr Thr Ser Ile Leu Gln Pro
1               5

<210>  24
<211>  9
<212>  PRT
<213>  Mus musculus

<400>  24

Leu Gln Tyr Asp Asn Leu Pro Trp Thr
1               5

<210>  25
<211>  125
<212>  PRT
<213>  Gallus gallus

<400>  25

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Glu Leu
            20                  25                  30

Ser Met Ser Trp Val Arg Gln Ala Pro Gly Lys Gly Leu Glu Trp Val
        35                  40                  45

Ser Ala Ile Ser Gly Ser Gly Gly Ser Thr Tyr Tyr Ala Asp Ser Val

50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Glu Gly Gly Arg Leu Glu Pro Tyr Tyr Tyr Tyr Tyr Ala Met
            100                 105                 110

Asp Val Trp Gly Gln Gly Thr Pro Val Thr Val Ser Ser
        115                 120                 125

<210>  26
<211>  5
<212>  PRT
<213>  Gallus gallus

<400>  26

Glu Leu Ser Met Ser
1               5

<210>  27
<211>  17
<212>  PRT
<213>  Gallus gallus

<400>  27

Ala Ile Ser Gly Ser Gly Gly Ser Thr Tyr Tyr Ala Asp Ser Val Lys
1               5                   10                  15

Gly

<210>  28

<211>  16
<212>  PRT
<213>  Gallus gallus

<400>  28

Glu Gly Gly Arg Leu Glu Pro Tyr Tyr Tyr Tyr Tyr Ala Met Asp Val
1               5                   10                  15



<210>  29
<211>  107
<212>  PRT
<213>  Gallus gallus

<400>  29

Asp Ile Gln Met Thr Gln Ser Pro Ser Ser Leu Ser Ala Ser Val Gly
1               5                   10                  15


Asp Arg Val Thr Ile Thr Cys Arg Ala Ser Gln Ser Ile Ser Asp Asn
            20                  25                  30


Leu His Trp Tyr Gln Gln Lys Pro Gly Lys Ala Pro Lys Leu Leu Ile
        35                  40                  45


Tyr Asp Ala Ser Asn Leu Glu Ser Gly Val Pro Ser Arg Phe Ser Gly
    50                  55                  60


Ser Gly Ser Gly Thr Asp Phe Thr Leu Thr Ile Asn Ser Leu Gln Pro
65                  70                  75                  80


Glu Asp Phe Ala Thr Tyr Tyr Cys Gln Gln Tyr Tyr Ser Thr Pro Leu
                85                  90                  95


Thr Phe Gly Gln Gly Thr Lys Leu Glu Ile Lys
            100                 105


<210>  30

<211>  11
<212>  PRT
<213>  Gallus gallus

<400>  30

Arg Ala Ser Gln Ser Ile Ser Asp Asn Leu His
1               5                   10



<210>  31
<211>  7
<212>  PRT
<213>  Gallus gallus

<400>  31

Asp Ala Ser Asn Leu Glu Ser
1               5



<210>  32
<211>  9
<212>  PRT
<213>  Gallus gallus

<400>  32

Gln Gln Tyr Tyr Ser Thr Pro Leu Thr
1               5



<210>  33
<211>  116
<212>  PRT
<213>  Artificial

<220>
<223>  Recombinant peptide

<400>  33

Gln Val Gln Leu Lys Glu Ser Gly Pro Val Leu Val Ala Pro Ser Gln
1               5                   10                  15

Ser Leu Ser Ile Thr Cys Ser Val Ser Gly Phe Ser Ser Thr Thr Tyr
            20                  25                  30

Gly Val His Trp Val Arg Gln Pro Pro Gly Gln Gly Leu Glu Trp Leu
        35                  40                  45

Gly Val Ile Trp Ala Gly Gly Ser Thr Asn Tyr Asn Ala Ala Leu Met
    50                  55                  60

Ser Arg Leu Ser Ile Ser Lys Asp Asn Ser Lys Ser Gln Val Phe Leu
65                  70                  75                  80

Lys Met Asn Ser Leu Gln Thr Asp Asp Thr Ala Met Tyr Tyr Cys Ala
                85                  90                  95

Thr Gln Tyr His Tyr Pro Phe Ala Tyr Trp Gly Gln Gly Thr Leu Val
            100                 105                 110

Thr Val Ser Ala
        115

<210>  34
<211>  16
<212>  PRT
<213>  Artificial

<220>
<223>  Recombinant peptide

<400>  34

Val Ile Trp Ala Gly Gly Ser Thr Asn Tyr Asn Ala Ala Leu Met Ser
1               5                   10                  15

<210>  35
<211>  108
<212>  PRT
<213>  Artificial

<220>
<223>  Recombinant peptide

<400>  35

Asp Ile Gln Met Thr Gln Ser Pro Ser Ser Leu Ser Ala Ser Leu Gly
1               5                   10                  15

Glu Arg Val Ser Leu Thr Cys Arg Ala Ser Gln Glu Ile Ser Gly Tyr
            20                  25                  30

Leu Ser Trp Leu Gln Gln Lys Pro Asp Gly Thr Ile Lys Arg Leu Ile
        35                  40                  45

Tyr Ala Ala Ser Thr Leu Ser Ser Gly Val Pro Lys Arg Phe Ser Gly
    50                  55                  60

Ser Arg Ser Gly Ser Asp Tyr Ser Leu Thr Ile Ser Ser Leu Glu Ser
65                  70                  75                  80

Glu Asp Phe Ala Asp Tyr Tyr Cys Leu Gln Tyr Ala Ser Tyr Pro Pro
                85                  90                  95

Trp Thr Phe Gly Gly Gly Thr Lys Leu Glu Ile Lys
            100                 105


<210>  36
<211>  7
<212>  PRT
<213>  Artificial

<220>
<223>  Recombinant peptide

<400>  36

Ala Ala Ser Thr Leu Ser Ser
1               5

<210>  37
<211>  108
<212>  PRT
<213>  Artificial

<220>
<223>  Recombinant peptide

<400>  37

Asp Ile Gln Met Thr Gln Ser Pro Ser Ser Leu Ser Ala Ser Leu Gly
1               5                   10                  15

Glu Arg Val Ser Leu Thr Cys Arg Ala Ser Gln Glu Ile Ser Gly Tyr
            20                  25                  30

Leu Ser Trp Leu Gln Gln Lys Pro Asp Gly Thr Ile Lys Arg Leu Ile
        35                  40                  45

Tyr Ala Ala Ser Thr Leu Glu Ser Gly Val Pro Lys Arg Phe Ser Gly
    50                  55                  60

Ser Arg Ser Gly Ser Asp Tyr Ser Leu Thr Ile Ser Ser Leu Glu Ser
65                  70                  75                  80

Glu Asp Phe Ala Asp Tyr Tyr Cys Leu Gln Tyr Ala Ser Tyr Pro Pro
                85                  90                  95

Trp Thr Phe Gly Gly Gly Thr Lys Leu Glu Ile Lys
            100                 105


<210>  38
<211>  7
<212>  PRT
<213>  Artificial

<220>

<223>  Recombinant peptide

<400>  38

Ala Ala Ser Thr Leu Glu Ser
1               5

<210>  39
<211>  108
<212>  PRT
<213>  Artificial

<220>
<223>  Recombinant peptide

<400>  39

Asp Ile Gln Met Thr Gln Ser Pro Ser Ser Leu Ser Ala Ser Leu Gly
1               5                   10                  15

Glu Arg Val Ser Leu Thr Cys Arg Ala Ser Gln Glu Ile Ser Gly Tyr
            20                  25                  30

Leu Ser Trp Leu Gln Gln Lys Pro Asp Gly Thr Ile Lys Arg Leu Ile
        35                  40                  45

Tyr Ala Ala Ser Thr Leu Asp Ala Gly Val Pro Lys Arg Phe Ser Gly
    50                  55                  60

Ser Arg Ser Gly Ser Asp Tyr Ser Leu Thr Ile Ser Ser Leu Glu Ser
65                  70                  75                  80

Glu Asp Phe Ala Asp Tyr Tyr Cys Leu Gln Tyr Ala Ser Tyr Pro Pro
                85                  90                  95

Trp Thr Phe Gly Gly Gly Thr Lys Leu Glu Ile Lys
            100                 105

<210>  40
<211>  7
<212>  PRT
<213>  Artificial

<220>
<223>  Recombinant peptide

<400>  40

Ala Ala Ser Thr Leu Asp Ala
1               5

<210>  41
<211>  119
<212>  PRT
<213>  Artificial

<220>
<223>  Recombinant peptide

<400>  41

Gln Val Gln Leu Gln Gln Ser Gly Ala Glu Leu Val Arg Pro Gly Ser
1               5                   10                  15

Ser Val Lys Ile Ser Cys Lys Ala Ser Gly Tyr Ala Phe Ser Asp Tyr
            20                  25                  30

Trp Met Asn Trp Val Lys Gln Arg Pro Gly Gln Gly Leu Glu Trp Ile
        35                  40                  45

Gly Gln Ile Tyr Pro Gly Ser Gly Asp Thr Asn Tyr Asn Gly Lys Phe
    50                  55                  60

Lys Gly Lys Gly Thr Leu Thr Ala Asp Lys Ser Ser Ser Thr Ala Tyr
65                  70                  75                  80

Met Gln Leu Asn Ser Leu Thr Ser Glu Asp Ser Ala Val Tyr Phe Cys
                85                  90                  95

Ala Arg Glu Arg Asp Tyr Asp Val Ala Met Asp Tyr Trp Gly Gln Gly
            100                 105                 110

Thr Ser Val Thr Val Ser Ser
        115

<210>  42
<211>  17
<212>  PRT
<213>  Artificial

<220>
<223>  Recombinant peptide

<400>  42

Gln Ile Tyr Pro Gly Ser Gly Asp Thr Asn Tyr Asn Gly Lys Phe Lys
1               5                   10                  15

Gly

<210>  43
<211>  119
<212>  PRT
<213>  Artificial

<220>
<223>  Recombinant peptide

<400>  43

Gln Val Gln Leu Gln Gln Ser Gly Ala Glu Leu Val Arg Pro Gly Ser
1               5                   10                  15

Ser Val Lys Ile Ser Cys Lys Ala Ser Gly Tyr Ala Phe Ser Asp Tyr
            20                  25                  30

Trp Met Asn Trp Val Lys Gln Arg Pro Gly Gln Gly Leu Glu Trp Ile
        35                  40                  45

Gly Gln Ile Tyr Pro Gly Glu Gly Asp Thr Asn Tyr Asn Gly Lys Phe
    50                  55                  60

Lys Gly Lys Gly Thr Leu Thr Ala Asp Lys Ser Ser Ser Thr Ala Tyr
65                  70                  75                  80

Met Gln Leu Asn Ser Leu Thr Ser Glu Asp Ser Ala Val Tyr Phe Cys
                85                  90                  95

Ala Arg Glu Arg Asp Tyr Asp Val Ala Met Asp Tyr Trp Gly Gln Gly
            100                 105                 110

Thr Ser Val Thr Val Ser Ser
        115

<210>  44
<211>  17
<212>  PRT
<213>  Artificial

<220>
<223>  Recombinant peptide

<400>  44

Gln Ile Tyr Pro Gly Glu Gly Asp Thr Asn Tyr Asn Gly Lys Phe Lys
1               5                   10                  15

Gly

<210>  45
<211>  119
<212>  PRT
<213>  Artificial

<220>
<223>  Recombinant peptide

<400>  45

Gln Val Gln Leu Gln Gln Ser Gly Ala Glu Leu Val Arg Pro Gly Ser
1               5                   10                  15

Ser Val Lys Ile Ser Cys Lys Ala Ser Gly Tyr Ala Phe Ser Asp Tyr
            20                  25                  30

Trp Met Asn Trp Val Lys Gln Arg Pro Gly Gln Gly Leu Glu Trp Ile
        35                  40                  45

Gly Gln Ile Tyr Pro Gly Asp Ala Asp Thr Asn Tyr Asn Gly Lys Phe
    50                  55                  60

Lys Gly Lys Gly Thr Leu Thr Ala Asp Lys Ser Ser Ser Thr Ala Tyr
65                  70                  75                  80

Met Gln Leu Asn Ser Leu Thr Ser Glu Asp Ser Ala Val Tyr Phe Cys
                85                  90                  95

Ala Arg Glu Arg Asp Tyr Asp Val Ala Met Asp Tyr Trp Gly Gln Gly
            100                 105                 110

Thr Ser Val Thr Val Ser Ser
        115

<210>  46
<211>  17
<212>  PRT
<213>  Artificial

<220>
<223>  Recombinant peptide

<400>  46

Gln Ile Tyr Pro Gly Asp Ala Asp Thr Asn Tyr Asn Gly Lys Phe Lys
1               5                   10                  15

Gly

<210>  47
<211>  119
<212>  PRT
<213>  Artificial

<220>
<223>  Recombinant peptide

<400>  47

Gln Val Gln Leu Gln Gln Ser Gly Ala Glu Leu Val Arg Pro Gly Ser
1               5                   10                  15

Ser Val Lys Ile Ser Cys Lys Ala Ser Gly Tyr Ala Phe Ser Asp Tyr
            20                  25                  30

Trp Met Asn Trp Val Lys Gln Arg Pro Gly Gln Gly Leu Glu Trp Ile
        35                  40                  45

Gly Gln Ile Tyr Pro Gly Asp Gly Asp Thr Asn Tyr Ser Gly Lys Phe
    50                  55                  60

Lys Gly Lys Gly Thr Leu Thr Ala Asp Lys Ser Ser Ser Thr Ala Tyr
65                  70                  75                  80

Met Gln Leu Asn Ser Leu Thr Ser Glu Asp Ser Ala Val Tyr Phe Cys
                85                  90                  95

Ala Arg Glu Arg Asp Tyr Asp Val Ala Met Asp Tyr Trp Gly Gln Gly
            100                 105                 110

Thr Ser Val Thr Val Ser Ser
        115

<210>  48
<211>  17
<212>  PRT
<213>  Artificial

<220>
<223>  Recombinant peptide

<400>  48

Gln Ile Tyr Pro Gly Asp Gly Asp Thr Asn Tyr Ser Gly Lys Phe Lys
1               5                   10                  15

Gly

<210>  49
<211>  119
<212>  PRT
<213>  Artificial

<220>
<223>  Recombinant peptide

<400>  49

Gln Val Gln Leu Gln Gln Ser Gly Ala Glu Leu Val Arg Pro Gly Ser
1               5                   10                  15

Ser Val Lys Ile Ser Cys Lys Ala Ser Gly Tyr Ala Phe Ser Asp Tyr
            20                  25                  30

Trp Met Asn Trp Val Lys Gln Arg Pro Gly Gln Gly Leu Glu Trp Ile
        35                  40                  45

Gly Gln Ile Tyr Pro Gly Asp Gly Asp Thr Asn Tyr Asn Ala Lys Phe
    50                  55                  60

Lys Gly Lys Gly Thr Leu Thr Ala Asp Lys Ser Ser Ser Thr Ala Tyr
65                  70                  75                  80

Met Gln Leu Asn Ser Leu Thr Ser Glu Asp Ser Ala Val Tyr Phe Cys
                85                  90                  95

Ala Arg Glu Arg Asp Tyr Asp Val Ala Met Asp Tyr Trp Gly Gln Gly
            100                 105                 110

Thr Ser Val Thr Val Ser Ser
        115

<210>  50
<211>  17
<212>  PRT
<213>  Artificial

<220>
<223>  Recombinant peptide

<400>  50

Gln Ile Tyr Pro Gly Asp Gly Asp Thr Asn Tyr Asn Ala Lys Phe Lys
1               5                   10                  15

Gly

<210>  51
<211>  119
<212>  PRT
<213>  Artificial

<220>
<223>  Recombinant peptide

<400>  51

Gln Val Gln Leu Gln Gln Ser Gly Ala Glu Leu Val Arg Pro Gly Ser
1               5                   10                  15

Ser Val Lys Ile Ser Cys Lys Ala Ser Gly Tyr Ala Phe Ser Asp Tyr
            20                  25                  30

Trp Met Asn Trp Val Lys Gln Arg Pro Gly Gln Gly Leu Glu Trp Ile
        35                  40                  45

Gly Gln Ile Tyr Pro Gly Ser Gly Asp Thr Asn Tyr Ser Gly Lys Phe
    50                  55                  60

Lys Gly Lys Gly Thr Leu Thr Ala Asp Lys Ser Ser Ser Thr Ala Tyr
65                  70                  75                  80

Met Gln Leu Asn Ser Leu Thr Ser Glu Asp Ser Ala Val Tyr Phe Cys
                85                  90                  95

Ala Arg Glu Arg Asp Tyr Asp Val Ala Met Asp Tyr Trp Gly Gln Gly
            100                 105                 110

Thr Ser Val Thr Val Ser Ser
        115

<210>  52
<211>  17
<212>  PRT
<213>  Artificial

<220>
<223>  Recombinant peptide

<400>  52

Gln Ile Tyr Pro Gly Ser Gly Asp Thr Asn Tyr Ser Gly Lys Phe Lys
1               5                   10                  15

Gly

<210> 53
<211> 119
<212> PRT
<213> Artificial

<220>
<223> Recombinant peptide

<400> 53

Gln Val Gln Leu Gln Gln Ser Gly Ala Glu Leu Val Arg Pro Gly Ser
1               5                   10                  15

Ser Val Lys Ile Ser Cys Lys Ala Ser Gly Tyr Ala Phe Ser Asp Tyr
            20                  25                  30

Trp Met Asn Trp Val Lys Gln Arg Pro Gly Gln Gly Leu Glu Trp Ile
        35                  40                  45

Gly Gln Ile Tyr Pro Gly Ser Gly Asp Thr Asn Tyr Asn Ala Lys Phe
    50                  55                  60

Lys Gly Lys Gly Thr Leu Thr Ala Asp Lys Ser Ser Ser Thr Ala Tyr
65                  70                  75                  80

Met Gln Leu Asn Ser Leu Thr Ser Glu Asp Ser Ala Val Tyr Phe Cys
                85                  90                  95

Ala Arg Glu Arg Asp Tyr Asp Val Ala Met Asp Tyr Trp Gly Gln Gly
            100                 105                 110

Thr Ser Val Thr Val Ser Ser
        115

<210>  54
<211>  17
<212>  PRT
<213>  Artificial

<220>
<223>  Recombinant peptide

<400>  54

Gln Ile Tyr Pro Gly Ser Gly Asp Thr Asn Tyr Asn Ala Lys Phe Lys
1               5                   10                  15

Gly

<210>  55
<211>  119
<212>  PRT
<213>  Artificial

<220>
<223>  Recombinant peptide

<400>  55

Gln Val Gln Leu Gln Gln Ser Gly Ala Glu Leu Val Arg Pro Gly Ser
1               5                   10                  15

Ser Val Lys Ile Ser Cys Lys Ala Ser Gly Tyr Ala Phe Ser Asp Tyr
            20                  25                  30

Trp Met Asn Trp Val Lys Gln Arg Pro Gly Gln Gly Leu Glu Trp Ile
        35                  40                  45

Gly Gln Ile Tyr Pro Gly Glu Gly Asp Thr Asn Tyr Ser Gly Lys Phe
    50                  55                  60

Lys Gly Lys Gly Thr Leu Thr Ala Asp Lys Ser Ser Ser Thr Ala Tyr
65                  70                  75                  80

Met Gln Leu Asn Ser Leu Thr Ser Glu Asp Ser Ala Val Tyr Phe Cys
                85                  90                  95

Ala Arg Glu Arg Asp Tyr Asp Val Ala Met Asp Tyr Trp Gly Gln Gly
            100                 105                 110

Thr Ser Val Thr Val Ser Ser
        115

<210>  56
<211>  17
<212>  PRT
<213>  Artificial

<220>
<223>  Recombinant peptide

<400>  56

Gln Ile Tyr Pro Gly Glu Gly Asp Thr Asn Tyr Ser Gly Lys Phe Lys
1               5                   10                  15

Gly

<210>  57
<211>  119
<212>  PRT
<213>  Artificial

<220>
<223>  Recombinant peptide

<400>  57

Gln Val Gln Leu Gln Gln Ser Gly Ala Glu Leu Val Arg Pro Gly Ser
1               5                   10                  15

Ser Val Lys Ile Ser Cys Lys Ala Ser Gly Tyr Ala Phe Ser Asp Tyr
            20                  25                  30

Trp Met Asn Trp Val Lys Gln Arg Pro Gly Gln Gly Leu Glu Trp Ile
        35                  40                  45

Gly Gln Ile Tyr Pro Gly Glu Gly Asp Thr Asn Tyr Asn Ala Lys Phe
    50                  55                  60

Lys Gly Lys Gly Thr Leu Thr Ala Asp Lys Ser Ser Ser Thr Ala Tyr
65                  70                  75                  80

Met Gln Leu Asn Ser Leu Thr Ser Glu Asp Ser Ala Val Tyr Phe Cys
                85                  90                  95

Ala Arg Glu Arg Asp Tyr Asp Val Ala Met Asp Tyr Trp Gly Gln Gly
            100                 105                 110

Thr Ser Val Thr Val Ser Ser
        115

<210>  58
<211>  17
<212>  PRT
<213>  Artificial

<220>
<223>  Recombinant peptide

<400>  58

Gln Ile Tyr Pro Gly Glu Gly Asp Thr Asn Tyr Asn Ala Lys Phe Lys
1               5                   10                  15

Gly

<210>  59
<211>  119
<212>  PRT
<213>  Artificial

<220>
<223>  Recombinant peptide

<400>  59

Gln Val Gln Leu Gln Gln Ser Gly Ala Glu Leu Val Arg Pro Gly Ser
1               5                   10                  15

Ser Val Lys Ile Ser Cys Lys Ala Ser Gly Tyr Ala Phe Ser Asp Tyr
            20                  25                  30

Trp Met Asn Trp Val Lys Gln Arg Pro Gly Gln Gly Leu Glu Trp Ile
        35                  40                  45

Gly Gln Ile Tyr Pro Gly Asp Ala Asp Thr Asn Tyr Ser Gly Lys Phe
    50                  55                  60

Lys Gly Lys Gly Thr Leu Thr Ala Asp Lys Ser Ser Ser Thr Ala Tyr
65                  70                  75                  80

Met Gln Leu Asn Ser Leu Thr Ser Glu Asp Ser Ala Val Tyr Phe Cys
                85                  90                  95

Ala Arg Glu Arg Asp Tyr Asp Val Ala Met Asp Tyr Trp Gly Gln Gly
            100                 105                 110

Thr Ser Val Thr Val Ser Ser
        115

<210>  60
<211>  17

<212>  PRT
<213>  Artificial

<220>
<223>  Recombinant peptide

<400>  60

Gln Ile Tyr Pro Gly Asp Ala Asp Thr Asn Tyr Ser Gly Lys Phe Lys
1               5                   10                  15

Gly

<210>  61
<211>  119
<212>  PRT
<213>  Artificial

<220>
<223>  Recombinant peptide

<400>  61

Gln Val Gln Leu Gln Gln Ser Gly Ala Glu Leu Val Arg Pro Gly Ser
1               5                   10                  15

Ser Val Lys Ile Ser Cys Lys Ala Ser Gly Tyr Ala Phe Ser Asp Tyr
            20                  25                  30

Trp Met Asn Trp Val Lys Gln Arg Pro Gly Gln Gly Leu Glu Trp Ile
        35                  40                  45

Gly Gln Ile Tyr Pro Gly Asp Ala Asp Thr Asn Tyr Asn Ala Lys Phe
    50                  55                  60

Lys Gly Lys Gly Thr Leu Thr Ala Asp Lys Ser Ser Ser Thr Ala Tyr
65                  70                  75                  80

Met Gln Leu Asn Ser Leu Thr Ser Glu Asp Ser Ala Val Tyr Phe Cys
                85                  90                  95

Ala Arg Glu Arg Asp Tyr Asp Val Ala Met Asp Tyr Trp Gly Gln Gly
            100                 105                 110

Thr Ser Val Thr Val Ser Ser
        115

<210>  62
<211>  17
<212>  PRT
<213>  Artificial

<220>
<223>  Recombinant peptide

<400>  62

Gln Ile Tyr Pro Gly Asp Ala Asp Thr Asn Tyr Asn Ala Lys Phe Lys
1               5                   10                  15

Gly

<210>  63
<211>  125
<212>  PRT
<213>  Artificial

<220>
<223>  Recombinant peptide

<400>  63

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Glu Leu
            20                  25                  30

Ser Met Ser Trp Val Arg Gln Ala Pro Gly Lys Gly Leu Glu Trp Val
        35                  40                  45

Ser Ala Ile Ser Gly Ser Gly Gly Ser Thr Tyr Tyr Ala Ser Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Glu Gly Gly Arg Leu Glu Pro Tyr Tyr Tyr Tyr Tyr Ala Met
            100                 105                 110

Asp Val Trp Gly Gln Gly Thr Pro Val Thr Val Ser Ser
        115                 120                 125

<210>  64
<211>  17
<212>  PRT
<213>  Artificial

<220>
<223>  Recombinant peptide

<400>  64

Ala Ile Ser Gly Ser Gly Gly Ser Thr Tyr Tyr Ala Ser Ser Val Lys
1               5                   10                  15

Gly

<210>  65
<211>  125

<212>  PRT
<213>  Artificial

<220>
<223>  Recombinant peptide

<400>  65

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Glu Leu
            20                  25                  30

Ser Met Ser Trp Val Arg Gln Ala Pro Gly Lys Gly Leu Glu Trp Val
        35                  40                  45

Ser Ala Ile Ser Gly Ser Gly Gly Ser Thr Tyr Tyr Ala Asp Ala Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Glu Gly Gly Arg Leu Glu Pro Tyr Tyr Tyr Tyr Tyr Ala Met
            100                 105                 110

Asp Val Trp Gly Gln Gly Thr Pro Val Thr Val Ser Ser
        115                 120                 125


<210>  66
<211>  17
<212>  PRT
<213>  Artificial

<220>

<223>  Recombinant peptide

<400>  66

Ala Ile Ser Gly Ser Gly Gly Ser Thr Tyr Tyr Ala Asp Ala Val Lys
1               5                   10                  15

Gly

<210>  67
<211>  107
<212>  PRT
<213>  Artificial

<220>
<223>  Recombinant peptide

<400>  67

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Ser Ile Ser Asp Asn
            20                  25                  30

Leu His Trp Tyr Gln Gln Lys Pro Gly Lys Ala Pro Lys Leu Leu Ile
        35                  40                  45

Tyr Asp Ala Ser Asn Leu Glu Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Tyr Tyr Ser Thr Pro Leu
                85                  90                  95

Thr Phe Gly Gln Gly Thr Lys Leu Glu Ile Lys
            100                 105

<210>  68
<211>  116
<212>  PRT
<213>  Artificial

<220>
<223>  Recombinant peptide

<400>  68

Gln Val Thr Leu Lys Glu Ser Gly Pro Val Leu Val Lys Pro Thr Glu
1               5                   10                  15

Thr Leu Thr Leu Thr Cys Thr Val Ser Gly Phe Ser Ser Thr Thr Tyr
            20                  25                  30

Gly Val His Trp Val Arg Gln Pro Pro Gly Lys Ala Leu Glu Trp Leu
        35                  40                  45

Gly Val Ile Trp Ala Gly Gly Ser Thr Asn Tyr Asn Ala Ala Leu Met
    50                  55                  60

Ser Arg Leu Thr Ile Ser Lys Asp Thr Ser Lys Ser Gln Val Val Leu
65                  70                  75                  80

Thr Met Thr Asn Met Asp Pro Val Asp Thr Ala Thr Tyr Tyr Cys Ala
                85                  90                  95

Thr Gln Tyr His Tyr Pro Phe Ala Tyr Trp Gly Gln Gly Thr Leu Val
            100                 105                 110

Thr Val Ser Ser
        115

<210> 69
<211> 116
<212> PRT
<213> Artificial

<220>
<223> Recombinant peptide

<400> 69

Gln Val Thr Leu Lys Glu Ser Gly Pro Val Leu Val Lys Pro Thr Glu
1               5                   10                  15

Thr Leu Thr Leu Thr Cys Thr Val Ser Gly Phe Ser Ser Thr Thr Tyr
            20                  25                  30

Gly Val His Trp Val Arg Gln Pro Pro Gly Lys Ala Leu Glu Trp Leu
        35                  40                  45

Gly Val Ile Trp Ala Gly Gly Ser Thr Asn Tyr Asn Ala Ala Leu Met
    50                  55                  60

Ser Arg Leu Thr Ile Ser Lys Asp Asn Ser Lys Ser Gln Val Val Leu
65                  70                  75                  80

Thr Met Thr Asn Met Asp Pro Val Asp Thr Ala Thr Tyr Tyr Cys Ala
                85                  90                  95

Thr Gln Tyr His Tyr Pro Phe Ala Tyr Trp Gly Gln Gly Thr Leu Val
            100                 105                 110

Thr Val Ser Ser
        115

<210> 70
<211> 107
<212> PRT
<213> Artificial

<220>
<223>  Recombinant peptide

<400>  70

Asp Ile Gln Met Thr Gln Ser Pro Ser Ser Leu Ser Ala Ser Val Gly
1               5                   10                  15

Asp Arg Val Thr Ile Thr Cys Lys Ala Ser Glu Asn Val Gly Ile Tyr
            20                  25                  30

Val Ser Trp Tyr Gln Gln Lys Pro Gly Lys Ala Pro Lys Leu Met Ile
        35                  40                  45

Tyr Gly Ala Ser Asn Arg Asn Thr Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gly Gln Ser Tyr Ser Tyr Pro Leu
                85                  90                  95

Thr Phe Gly Gly Gly Thr Lys Val Glu Ile Lys
            100                 105

<210>  71
<211>  107
<212>  PRT
<213>  Artificial

<220>
<223>  Recombinant peptide

<400>  71

Asp Ile Gln Met Thr Gln Ser Pro Ser Ser Leu Ser Ala Ser Val Gly
1               5                   10                  15

Asp Arg Val Thr Ile Thr Cys Lys Ala Ser Glu Asn Val Gly Ile Tyr
            20                  25                  30

Val Ser Trp Tyr Gln Gln Lys Pro Gly Lys Ala Pro Lys Leu Met Ile
        35                  40                  45

Tyr Gly Ala Ser Asn Arg Asn Thr Gly Val Pro Ser Arg Phe Ser Gly
    50                  55                  60

Ser Gly Ser Ala Thr Asp Phe Thr Leu Thr Ile Ser Ser Leu Gln Pro
65                  70                  75                  80

Glu Asp Phe Ala Thr Tyr Tyr Cys Gly Gln Ser Tyr Ser Tyr Pro Leu
                85                  90                  95

Thr Phe Gly Gly Gly Thr Lys Val Glu Ile Lys
            100                 105

<210> 72
<211> 123
<212> PRT
<213> Artificial

<220>
<223> Recombinant peptide

<400> 72

Gln Val Thr Leu Lys Glu Ser Gly Pro Ala Leu Val Lys Pro Thr Gln
1               5                   10                  15

Thr Leu Thr Leu Thr Cys Thr Phe Ser Gly Phe Ser Leu Ser Thr Ser
            20                  25                  30

Gly Met Gly Val Asp Trp Ile Arg Gln Pro Pro Gly Lys Ala Leu Glu
        35                  40                  45

Trp Leu Ala His Ile Trp Trp Asp Asp Asp Lys Tyr Tyr Asn Pro Ser
    50                  55                  60

Leu Lys Ser Gln Leu Thr Ile Ser Lys Asp Thr Ser Lys Asn Gln Val
65                  70                  75                  80

Val Leu Thr Met Thr Asn Met Asp Pro Val Asp Thr Ala Thr Tyr Tyr
                85                  90                  95

Cys Ala Arg Ser Tyr Gly Tyr Asp Met Asp Tyr Tyr Ala Met Asp Tyr
            100                 105                 110

Trp Gly Gln Gly Thr Thr Val Thr Val Ser Ser
        115                 120

<210>  73
<211>  123
<212>  PRT
<213>  Artificial

<220>
<223>  Recombinant peptide

<400>  73

Gln Val Thr Leu Lys Glu Ser Gly Pro Ala Leu Val Lys Pro Thr Gln
1               5                   10                  15

Thr Leu Thr Leu Thr Cys Thr Phe Ser Gly Phe Ser Leu Ser Thr Ser
            20                  25                  30

Gly Met Gly Val Asp Trp Ile Arg Gln Pro Pro Gly Lys Gly Leu Glu
        35                  40                  45

Trp Leu Ala His Ile Trp Trp Asp Asp Asp Lys Tyr Tyr Asn Pro Ser
    50                  55                  60

Leu Lys Ser Gln Leu Thr Ile Ser Lys Asp Thr Ser Lys Asn Gln Val
65                  70                  75                  80

Val Leu Thr Met Thr Asn Met Asp Pro Val Asp Thr Ala Thr Tyr Tyr
                85                  90                  95

Cys Ala Arg Ser Tyr Gly Tyr Asp Met Asp Tyr Tyr Ala Met Asp Tyr
            100                 105                 110

Trp Gly Gln Gly Thr Thr Val Thr Val Ser Ser
        115                 120

<210>  74
<211>  108
<212>  PRT
<213>  Artificial

<220>
<223>  Recombinant peptide

<400>  74

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Glu Ile Ser Gly Tyr
            20                  25                  30

Leu Ser Trp Leu Gln Gln Lys Pro Gly Lys Ala Pro Lys Arg Leu Ile
        35                  40                  45

Tyr Ala Ala Ser Thr Leu Ser Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Leu Gln Tyr Ala Ser Tyr Pro Pro
                85                  90                  95

Trp Thr Phe Gly Gly Gly Thr Lys Val Glu Ile Lys
            100                 105

<210> 75
<211> 108
<212> PRT
<213> Artificial

<220>
<223> Recombinant peptide

<400> 75

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Glu Ile Ser Gly Tyr
            20                  25                  30

Leu Ser Trp Leu Gln Gln Lys Pro Gly Lys Ala Pro Lys Arg Leu Ile
        35                  40                  45

Tyr Ala Ala Ser Thr Leu Ser Ser Gly Val Pro Ser Arg Phe Ser Gly
    50                  55                  60

Ser Gly Ser Gly Thr Asp Tyr Thr Leu Thr Ile Ser Ser Leu Gln Pro
65                  70                  75                  80

Glu Asp Phe Ala Thr Tyr Tyr Cys Leu Gln Tyr Ala Ser Tyr Pro Pro
                85                  90                  95

Trp Thr Phe Gly Gly Gly Thr Lys Val Glu Ile Lys
            100                 105

<210>  76
<211>  119
<212>  PRT
<213>  Artificial

<220>
<223>  Recombinant peptide

<400>  76

Gln Val Gln Leu Val Gln Ser Gly Ala Glu Val Lys Lys Pro Gly Ser
1               5                   10                  15

Ser Val Lys Val Ser Cys Lys Ala Ser Gly Tyr Ala Phe Ser Asp Tyr
            20                  25                  30

Trp Met Asn Trp Val Arg Gln Ala Pro Gly Gln Gly Leu Glu Trp Ile
        35                  40                  45

Gly Gln Ile Tyr Pro Gly Ser Gly Asp Thr Asn Tyr Asn Ala Lys Phe
    50                  55                  60

Lys Gly Lys Gly Thr Ile Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Glu Arg Asp Tyr Asp Val Ala Met Asp Tyr Trp Gly Gln Gly
            100                 105                 110

Thr Thr Val Thr Val Ser Ser
        115

<210>  77
<211>  119

<212>  PRT
<213>  Artificial

<220>
<223>  Recombinant peptide

<400>  77

Gln Val Gln Leu Val Gln Ser Gly Ala Glu Val Lys Lys Pro Gly Ser
1               5                   10                  15

Ser Val Lys Val Ser Cys Lys Ala Ser Gly Tyr Ala Phe Ser Asp Tyr
            20                  25                  30

Trp Met Asn Trp Val Arg Gln Ala Pro Gly Gln Gly Leu Glu Trp Ile
        35                  40                  45

Gly Gln Ile Tyr Pro Gly Ser Gly Asp Thr Asn Tyr Asn Ala Lys Phe
    50                  55                  60

Lys Gly Lys Gly Thr Leu Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Glu Arg Asp Tyr Asp Val Ala Met Asp Tyr Trp Gly Gln Gly
            100                 105                 110

Thr Thr Val Thr Val Ser Ser
        115


<210>  78
<211>  107
<212>  PRT
<213>  Artificial

<220>

<223>  Recombinant peptide

<400>  78

Asp Ile Gln Met Thr Gln Ser Pro Ser Ser Leu Ser Ala Ser Val Gly
1               5                   10                  15

Asp Arg Val Thr Ile Thr Cys Lys Ala Ser Gln Asp Ile Asn Lys Tyr
            20                  25                  30

Ile Ala Trp Phe Gln Gln Lys Pro Gly Lys Ala Pro Lys Leu Val Ile
        35                  40                  45

His Tyr Thr Ser Ile Leu Gln Pro Gly Val Pro Ser Arg Phe Ser Gly
    50                  55                  60

Ser Gly Ser Gly Thr Asp Tyr Thr Phe Thr Ile Ser Ser Leu Gln Pro
65                  70                  75                  80

Glu Asp Ile Ala Thr Tyr Tyr Cys Leu Gln Tyr Asp Asn Leu Pro Trp
                85                  90                  95

Thr Phe Gly Gly Gly Thr Lys Val Glu Ile Lys
            100                 105

<210>  79
<211>  107
<212>  PRT
<213>  Artificial

<220>
<223>  Recombinant peptide

<400>  79

Asp Ile Gln Met Thr Gln Ser Pro Ser Ser Leu Ser Ala Ser Val Gly
1               5                   10                  15

Asp Arg Val Thr Ile Thr Cys Lys Ala Ser Gln Asp Ile Asn Lys Tyr
            20                  25                  30

Ile Ala Trp Phe Gln Gln Lys Pro Gly Lys Ala Pro Lys Leu Val Ile
        35                  40                  45

His Tyr Thr Ser Ile Leu Gln Pro Gly Val Pro Ser Arg Phe Ser Gly
    50                  55                  60

Val Gly Ser Gly Thr Asp Tyr Thr Phe Thr Ile Ser Ser Leu Gln Pro
65                  70                  75                  80

Glu Asp Ile Ala Thr Tyr Tyr Cys Leu Gln Tyr Asp Asn Leu Pro Trp
                85                  90                  95

Thr Phe Gly Gly Gly Thr Lys Val Glu Ile Lys
            100                 105

<210>  80
<211>  324
<212>  DNA
<213>  Homo sapiens

<220>
<221>  CDS
<222>  (1)..(324)

<400>  80
atg gcc cgc gct gct ctc tcc gcc gcc ccc agc aat ccc cgg ctc ctg        48
Met Ala Arg Ala Ala Leu Ser Ala Ala Pro Ser Asn Pro Arg Leu Leu
1               5                   10                  15

cga gtg gca ctg ctg ctc ctg ctc ctg gta gcc gct ggc cgg cgc gca        96
Arg Val Ala Leu Leu Leu Leu Leu Leu Val Ala Ala Gly Arg Arg Ala
            20                  25                  30

gca gga gcg tcc gtg gcc act gaa ctg cgc tgc cag tgc ttg cag acc       144
Ala Gly Ala Ser Val Ala Thr Glu Leu Arg Cys Gln Cys Leu Gln Thr
        35                  40                  45

ctg cag gga att cac ccc aag aac atc caa agt gtg aac gtg aag tcc     192
Leu Gln Gly Ile His Pro Lys Asn Ile Gln Ser Val Asn Val Lys Ser
    50                  55                  60

ccc gga ccc cac tgc gcc caa acc gaa gtc ata gcc aca ctc aag aat     240
Pro Gly Pro His Cys Ala Gln Thr Glu Val Ile Ala Thr Leu Lys Asn
65                  70                  75                  80

ggg cgg aaa gct tgc ctc aat cct gca tcc ccc ata gtt aag aaa atc     288
Gly Arg Lys Ala Cys Leu Asn Pro Ala Ser Pro Ile Val Lys Lys Ile
                85                  90                  95

atc gaa aag atg ctg aac agt gac aaa tcc aac tag                     324
Ile Glu Lys Met Leu Asn Ser Asp Lys Ser Asn
            100                 105

<210>  81
<211>  107
<212>  PRT
<213>  Homo sapiens

<400>  81

Met Ala Arg Ala Ala Leu Ser Ala Ala Pro Ser Asn Pro Arg Leu Leu
1               5                   10                  15

Arg Val Ala Leu Leu Leu Leu Leu Leu Val Ala Ala Gly Arg Arg Ala
            20                  25                  30

Ala Gly Ala Ser Val Ala Thr Glu Leu Arg Cys Gln Cys Leu Gln Thr
        35                  40                  45

Leu Gln Gly Ile His Pro Lys Asn Ile Gln Ser Val Asn Val Lys Ser
    50                  55                  60

Pro Gly Pro His Cys Ala Gln Thr Glu Val Ile Ala Thr Leu Lys Asn
65                  70                  75                  80

Gly Arg Lys Ala Cys Leu Asn Pro Ala Ser Pro Ile Val Lys Lys Ile

                85                  90                  95

Ile Glu Lys Met Leu Asn Ser Asp Lys Ser Asn
            100                 105

<210> 82
<211> 107
<212> PRT
<213> Macaca fascicularis

<400> 82

Met Ala Arg Ala Ala Leu Ser Ala Ala Pro Ser Asn Pro Arg Phe Leu
1               5                   10                  15

Gln Val Ala Leu Leu Leu Leu Leu Leu Val Ala Thr Gly Arg Arg Ala
            20                  25                  30

Ala Gly Ala Ser Val Val Thr Glu Leu Arg Cys Gln Cys Leu Gln Thr
        35                  40                  45

Leu Gln Gly Ile His Pro Lys Asn Ile Gln Ser Val Asn Val Lys Ala
    50                  55                  60

Pro Gly Pro His Cys Ala Glu Thr Glu Val Ile Ala Thr Leu Lys Asn
65                  70                  75                  80

Gly Gln Lys Ala Cys Leu Asn Pro Ala Ser Pro Met Val Gln Lys Ile
                85                  90                  95

Ile Lys Lys Met Leu Asn Cys Asp Lys Ser Asn
            100                 105